# EUROPEAN PATENT APPLICATION

(11) **EP 3 385 378 A1**
(43) Date of publication of application: **10.10.2018**
(21) Application number: 15909683.3
(22) Date of filing: 30.11.2015
(51) Int. Cl.: C12N 15/00, C12N 1/21, C12P 5/00, C12P 5/02

(54) **RECOMBINANT CELL, METHOD FOR PRODUCING RECOMBINANT CELL, AND METHOD FOR PRODUCING ORGANIC COMPOUND**

(71) Applicant: Sekisui Chemical Co., Ltd., Osaka-shi, Osaka 530-8565 (JP)
(72) Inventor: FURUTANI, Masahiro, Tsukuba-shi Ibaraki 300-4292 (JP); NISHIYAMA, Norihide, Tsukuba-shi Ibaraki 300-4292 (JP); KAWABATA, Kazufumi, Tsukuba-shi Ibaraki 300-4292 (JP)
(74) Representative: Ter Meer Steinmeister & Partner
(86) International application number: PCT/JP2015/083545
(87) International publication number: WO 2017/094053

(57) **Abstract**

A recombinant cell having a function of synthesizing acetyl-CoA from methyltetrahydrofolate, carbon monoxide, and CoA, including: a gene that expresses an exogenous NAD(P)H consumption pathway, the gene being expressed in the recombinant cell, wherein expression in at least one of endogenous NAD(P)H consumption pathways of the recombinant cell is down-regulated, and the endogenous NAD(P)H consumption pathway is different from the exogenous NAD(P)H consumption pathway, and the recombinant cell produces an organic compound having 4 or more carbon atoms from at least one selected from the group consisting of carbon monoxide and carbon dioxide via the exogenous NAD(P)H consumption pathway.

SEQUENCE LISTING

<110>  Sekisui Chemical Co., Ltd.

<120>  RECOMBINANT CELL, METHOD FOR PRODUCING RECOMBINANT CELL, AND METHOD
FOR PRODUCING ORGANIC COMPOUND

<130>  15P01503

<160>  15

<170>  PatentIn version 3.1

<210>  1
<211>  1788
<212>  DNA
<213>  Populus nigra

<220>
<221>  CDS
<222>  (1)..(1788)
<223>

<400>  1
atg gca act gaa tta ttg tgc ttg cac cgt cca atc tca ctg aca cac        48
Met Ala Thr Glu Leu Leu Cys Leu His Arg Pro Ile Ser Leu Thr His
1               5                   10                  15

aaa ttg ttc aga aat ccc ttg cct aaa gtc atc cag gcc act ccc tta        96
Lys Leu Phe Arg Asn Pro Leu Pro Lys Val Ile Gln Ala Thr Pro Leu
            20                  25                  30

act ttg aaa ctc aga tgt tct gta agc aca gaa aac gtc agc ttc aca       144
Thr Leu Lys Leu Arg Cys Ser Val Ser Thr Glu Asn Val Ser Phe Thr
        35                  40                  45

gaa aca gaa aca gaa acc aga agg tct gcc aat tat gaa cca aat agc       192
Glu Thr Glu Thr Glu Thr Arg Arg Ser Ala Asn Tyr Glu Pro Asn Ser
    50                  55                  60

tgg gat tat gat tat ttg ctg tct tcg gac act gac gaa tcg att gaa       240
Trp Asp Tyr Asp Tyr Leu Leu Ser Ser Asp Thr Asp Glu Ser Ile Glu
65                  70                  75                  80

gta tac aaa gac aag gcc aaa aag ctg gag gct gag gtg aga aga gag       288
Val Tyr Lys Asp Lys Ala Lys Lys Leu Glu Ala Glu Val Arg Arg Glu
                85                  90                  95

att aac aat gaa aag gca gag ttt ttg act ctg cct gaa ctg ata gat       336
Ile Asn Asn Glu Lys Ala Glu Phe Leu Thr Leu Pro Glu Leu Ile Asp
            100                 105                 110

aat gtc caa agg tta gga tta ggt tac cgg ttc gag agt gac ata agg       384
Asn Val Gln Arg Leu Gly Leu Gly Tyr Arg Phe Glu Ser Asp Ile Arg
        115                 120                 125

aga gcc ctt gat aga ttt gtt tct tca gga gga ttt gat gct gtt aca       432
Arg Ala Leu Asp Arg Phe Val Ser Ser Gly Gly Phe Asp Ala Val Thr
    130                 135                 140

aaa act agc ctt cat gct act gct ctt agc ttc agg ctt ctc aga cag       480
Lys Thr Ser Leu His Ala Thr Ala Leu Ser Phe Arg Leu Leu Arg Gln
145                 150                 155                 160

cat ggc ttt gag gtc tct caa gaa gcg ttc agc gga ttc aag gat caa 528
His Gly Phe Glu Val Ser Gln Glu Ala Phe Ser Gly Phe Lys Asp Gln
                165                 170                 175

aat ggc aat ttc ttg aaa aac ctt aag gag gac atc aag gca ata cta 576
Asn Gly Asn Phe Leu Lys Asn Leu Lys Glu Asp Ile Lys Ala Ile Leu
            180                 185                 190

agc cta tat gaa gct tca ttt ctt gcc tta gaa gga gaa aat atc ttg 624
Ser Leu Tyr Glu Ala Ser Phe Leu Ala Leu Glu Gly Glu Asn Ile Leu
        195                 200                 205

gat gag gcc aag gtg ttt gca ata tca cat cta aaa gag ctc agc gaa 672
Asp Glu Ala Lys Val Phe Ala Ile Ser His Leu Lys Glu Leu Ser Glu
    210                 215                 220

gaa aag att gga aaa gac ctg gcc gaa cag gtg aat cat gca ttg gag 720
Glu Lys Ile Gly Lys Asp Leu Ala Glu Gln Val Asn His Ala Leu Glu
225                 230                 235                 240

ctt cca ttg cat cga agg acg caa aga cta gaa gct gtt tgg agc att 768
Leu Pro Leu His Arg Arg Thr Gln Arg Leu Glu Ala Val Trp Ser Ile
                245                 250                 255

gaa gca tac cgt aaa aag gaa gat gca gat caa gta ctg cta gaa ctt 816
Glu Ala Tyr Arg Lys Lys Glu Asp Ala Asp Gln Val Leu Leu Glu Leu
            260                 265                 270

gct ata ttg gac tac aac atg att caa tca gta tac caa aga gat ctt 864
Ala Ile Leu Asp Tyr Asn Met Ile Gln Ser Val Tyr Gln Arg Asp Leu
        275                 280                 285

cgc gag aca tca agg tgg tgg agg cgt gtg ggt ctt gca aca aag ttg 912
Arg Glu Thr Ser Arg Trp Trp Arg Arg Val Gly Leu Ala Thr Lys Leu
    290                 295                 300

cat ttt gct aga gac agg tta att gaa agc ttt tac tgg gca gtt gga 960
His Phe Ala Arg Asp Arg Leu Ile Glu Ser Phe Tyr Trp Ala Val Gly
305                 310                 315                 320

gtt gcg ttt gaa cct caa tac agt gat tgc cgt aat tcc gta gca aaa 1008
Val Ala Phe Glu Pro Gln Tyr Ser Asp Cys Arg Asn Ser Val Ala Lys
                325                 330                 335

atg ttt tcg ttt gta aca atc att gat gat atc tat gat gtt tat ggt 1056
Met Phe Ser Phe Val Thr Ile Ile Asp Asp Ile Tyr Asp Val Tyr Gly
            340                 345                 350

act ctg gat gag ttg gag cta ttt aca gat gct gtt gag aga tgg gat 1104
Thr Leu Asp Glu Leu Glu Leu Phe Thr Asp Ala Val Glu Arg Trp Asp
        355                 360                 365

gtt aat gcc atc gat gat ctt ccg gat tat atg aag ctc tgc ttc cta 1152
Val Asn Ala Ile Asp Asp Leu Pro Asp Tyr Met Lys Leu Cys Phe Leu
    370                 375                 380

gct ctc tat aac act atc aat gag ata gct tat gat aat ctg aag gac 1200
Ala Leu Tyr Asn Thr Ile Asn Glu Ile Ala Tyr Asp Asn Leu Lys Asp
385                 390                 395                 400

aag ggg gaa aac att ctt cca tac cta aca aaa gcg tgg gca gat tta 1248
Lys Gly Glu Asn Ile Leu Pro Tyr Leu Thr Lys Ala Trp Ala Asp Leu

                    405                     410                     415

tgc aat gca ttc cta caa gaa gca aaa tgg ttg tac aat aag tcc aca      1296
Cys Asn Ala Phe Leu Gln Glu Ala Lys Trp Leu Tyr Asn Lys Ser Thr
            420                 425                 430

cca aca ttt gat gaa tat ttc gga aat gca tgg aaa tca tcc tca ggg      1344
Pro Thr Phe Asp Glu Tyr Phe Gly Asn Ala Trp Lys Ser Ser Ser Gly
        435                 440                 445

cct ctt caa cta gtt ttt gcc tac ttt gcc gtt gtt caa aac atc aag      1392
Pro Leu Gln Leu Val Phe Ala Tyr Phe Ala Val Val Gln Asn Ile Lys
    450                 455                 460

aaa gag gaa att gat aac tta caa aag tat cat gat atc atc agt agg      1440
Lys Glu Glu Ile Asp Asn Leu Gln Lys Tyr His Asp Ile Ile Ser Arg
465                 470                 475                 480

cct tcc cac atc ttt cgt ctt tgc aac gac ttg gct tca gca tcg gct      1488
Pro Ser His Ile Phe Arg Leu Cys Asn Asp Leu Ala Ser Ala Ser Ala
                485                 490                 495

gag ata gcg aga ggt gaa acc gcg aat tct gta tca tgc tac atg cgt      1536
Glu Ile Ala Arg Gly Glu Thr Ala Asn Ser Val Ser Cys Tyr Met Arg
            500                 505                 510

aca aaa ggc att tct gag gaa ctt gct act gaa tcc gta atg aat ttg      1584
Thr Lys Gly Ile Ser Glu Glu Leu Ala Thr Glu Ser Val Met Asn Leu
        515                 520                 525

atc gac gaa acc tgg aaa aag atg aac aaa gaa aag ctt ggt ggc tct      1632
Ile Asp Glu Thr Trp Lys Lys Met Asn Lys Glu Lys Leu Gly Gly Ser
    530                 535                 540

ctg ttt gca aaa cct ttt gtc gaa aca gct att aac ctt gca cga caa      1680
Leu Phe Ala Lys Pro Phe Val Glu Thr Ala Ile Asn Leu Ala Arg Gln
545                 550                 555                 560

tcc cat tgc act tat cac aac gga gat gcg cat act tca cca gat gag      1728
Ser His Cys Thr Tyr His Asn Gly Asp Ala His Thr Ser Pro Asp Glu
                565                 570                 575

ctc act agg aaa cgt gtc ctg tca gta atc aca gag cct att cta ccc      1776
Leu Thr Arg Lys Arg Val Leu Ser Val Ile Thr Glu Pro Ile Leu Pro
            580                 585                 590

ttt gag aga taa                                                      1788
Phe Glu Arg
        595


<210>  2
<211>  595
<212>  PRT
<213>  Populus nigra

<400>  2

Met Ala Thr Glu Leu Leu Cys Leu His Arg Pro Ile Ser Leu Thr His
1               5                   10                  15

Lys Leu Phe Arg Asn Pro Leu Pro Lys Val Ile Gln Ala Thr Pro Leu

20                  25                  30

Thr Leu Lys Leu Arg Cys Ser Val Ser Thr Glu Asn Val Ser Phe Thr
        35                  40                  45

Glu Thr Glu Thr Glu Thr Arg Arg Ser Ala Asn Tyr Glu Pro Asn Ser
    50                  55                  60

Trp Asp Tyr Asp Tyr Leu Leu Ser Ser Asp Thr Asp Glu Ser Ile Glu
65                  70                  75                  80

Val Tyr Lys Asp Lys Ala Lys Lys Leu Glu Ala Glu Val Arg Arg Glu
                85                  90                  95

Ile Asn Asn Glu Lys Ala Glu Phe Leu Thr Leu Pro Glu Leu Ile Asp
            100                 105                 110

Asn Val Gln Arg Leu Gly Leu Gly Tyr Arg Phe Glu Ser Asp Ile Arg
        115                 120                 125

Arg Ala Leu Asp Arg Phe Val Ser Ser Gly Gly Phe Asp Ala Val Thr
    130                 135                 140

Lys Thr Ser Leu His Ala Thr Ala Leu Ser Phe Arg Leu Leu Arg Gln
145                 150                 155                 160

His Gly Phe Glu Val Ser Gln Glu Ala Phe Ser Gly Phe Lys Asp Gln
                165                 170                 175

Asn Gly Asn Phe Leu Lys Asn Leu Lys Glu Asp Ile Lys Ala Ile Leu
            180                 185                 190

Ser Leu Tyr Glu Ala Ser Phe Leu Ala Leu Glu Gly Glu Asn Ile Leu
        195                 200                 205

Asp Glu Ala Lys Val Phe Ala Ile Ser His Leu Lys Glu Leu Ser Glu
    210                 215                 220

Glu Lys Ile Gly Lys Asp Leu Ala Glu Gln Val Asn His Ala Leu Glu
225                 230                 235                 240

Leu Pro Leu His Arg Arg Thr Gln Arg Leu Glu Ala Val Trp Ser Ile
                245                 250                 255

Glu Ala Tyr Arg Lys Lys Glu Asp Ala Asp Gln Val Leu Leu Glu Leu
            260                 265                 270

Ala Ile Leu Asp Tyr Asn Met Ile Gln Ser Val Tyr Gln Arg Asp Leu
        275                 280                 285

Arg Glu Thr Ser Arg Trp Trp Arg Arg Val Gly Leu Ala Thr Lys Leu
    290                 295                 300

His Phe Ala Arg Asp Arg Leu Ile Glu Ser Phe Tyr Trp Ala Val Gly
305                 310                 315                 320

Val Ala Phe Glu Pro Gln Tyr Ser Asp Cys Arg Asn Ser Val Ala Lys
                325                 330                 335

Met Phe Ser Phe Val Thr Ile Ile Asp Asp Ile Tyr Asp Val Tyr Gly
            340                 345                 350

Thr Leu Asp Glu Leu Glu Leu Phe Thr Asp Ala Val Glu Arg Trp Asp
        355                 360                 365

Val Asn Ala Ile Asp Asp Leu Pro Asp Tyr Met Lys Leu Cys Phe Leu
    370                 375                 380

Ala Leu Tyr Asn Thr Ile Asn Glu Ile Ala Tyr Asp Asn Leu Lys Asp
385                 390                 395                 400

Lys Gly Glu Asn Ile Leu Pro Tyr Leu Thr Lys Ala Trp Ala Asp Leu
                405                 410                 415

Cys Asn Ala Phe Leu Gln Glu Ala Lys Trp Leu Tyr Asn Lys Ser Thr
            420                 425                 430

Pro Thr Phe Asp Glu Tyr Phe Gly Asn Ala Trp Lys Ser Ser Ser Gly
        435                 440                 445

Pro Leu Gln Leu Val Phe Ala Tyr Phe Ala Val Val Gln Asn Ile Lys
    450                 455                 460

Lys Glu Glu Ile Asp Asn Leu Gln Lys Tyr His Asp Ile Ile Ser Arg
465                 470                 475                 480

Pro Ser His Ile Phe Arg Leu Cys Asn Asp Leu Ala Ser Ala Ser Ala
                485                 490                 495

Glu Ile Ala Arg Gly Glu Thr Ala Asn Ser Val Ser Cys Tyr Met Arg
            500                 505                 510

Thr Lys Gly Ile Ser Glu Glu Leu Ala Thr Glu Ser Val Met Asn Leu
        515                 520                 525

Ile Asp Glu Thr Trp Lys Lys Met Asn Lys Glu Lys Leu Gly Gly Ser
    530                 535                 540

Leu Phe Ala Lys Pro Phe Val Glu Thr Ala Ile Asn Leu Ala Arg Gln
545                 550                 555                 560

Ser His Cys Thr Tyr His Asn Gly Asp Ala His Thr Ser Pro Asp Glu
                565                 570                 575

Leu Thr Arg Lys Arg Val Leu Ser Val Ile Thr Glu Pro Ile Leu Pro
            580                 585                 590

Phe Glu Arg
        595

<210>  3
<211>  1269
<212>  DNA
<213>  Arabidopsis thaliana

<220>
<221>  CDS
<222>  (1)..(1269)
<223>

<400>  3
atg tta ttc acg agg agt gtt gct cgg att tct tct aag ttt ctg aga       48
Met Leu Phe Thr Arg Ser Val Ala Arg Ile Ser Ser Lys Phe Leu Arg
1               5                   10                  15

aac cgt agc ttc tat ggc tcc tct caa tct ctc gcc tct cat cgg ttc       96
Asn Arg Ser Phe Tyr Gly Ser Ser Gln Ser Leu Ala Ser His Arg Phe
            20                  25                  30

gca atc att ccc gat cag ggt cac tct tgt tct gac tct cca cac aag      144
Ala Ile Ile Pro Asp Gln Gly His Ser Cys Ser Asp Ser Pro His Lys
        35                  40                  45

ggt tac gtt tgc aga aca act tat tca ttg aaa tct ccg gtt ttt ggt      192
Gly Tyr Val Cys Arg Thr Thr Tyr Ser Leu Lys Ser Pro Val Phe Gly
    50                  55                  60

gga ttt agt cat caa ctc tat cac cag agt agc tcc ttg gtt gag gag      240
Gly Phe Ser His Gln Leu Tyr His Gln Ser Ser Ser Leu Val Glu Glu
65                  70                  75                  80

gag ctt gac cca ttt tcg ctt gtt gcc gat gag ctg tca ctt ctt agt      288
Glu Leu Asp Pro Phe Ser Leu Val Ala Asp Glu Leu Ser Leu Leu Ser
                85                  90                  95

aat aag ttg aga gag atg gta ctt gcc gag gtt cca aag ctt gcc tct      336
Asn Lys Leu Arg Glu Met Val Leu Ala Glu Val Pro Lys Leu Ala Ser
            100                 105                 110

gct gct gag tac ttc ttc aaa agg ggt gtg caa gga aaa cag ttt cgt      384
Ala Ala Glu Tyr Phe Phe Lys Arg Gly Val Gln Gly Lys Gln Phe Arg
        115                 120                 125

tca act att ttg ctg ctg atg gcg aca gct ctg gat gta cga gtt cca 432
Ser Thr Ile Leu Leu Leu Met Ala Thr Ala Leu Asp Val Arg Val Pro
    130                 135                 140

gaa gca ttg att ggg gaa tca aca gat ata gtc aca tca gaa tta cgc 480
Glu Ala Leu Ile Gly Glu Ser Thr Asp Ile Val Thr Ser Glu Leu Arg
145                 150                 155                 160

gta agg caa cgg ggt att gct gaa atc act gaa atg ata cac gtc gca 528
Val Arg Gln Arg Gly Ile Ala Glu Ile Thr Glu Met Ile His Val Ala
                165                 170                 175

agt cta ctg cac gat gat gtc ttg gat gat gcc gat aca agg cgt ggt 576
Ser Leu Leu His Asp Asp Val Leu Asp Asp Ala Asp Thr Arg Arg Gly
            180                 185                 190

gtt ggt tcc tta aat gtt gta atg ggt aac aag atg tcg gta tta gca 624
Val Gly Ser Leu Asn Val Val Met Gly Asn Lys Met Ser Val Leu Ala
        195                 200                 205

gga gac ttc ttg ctc tcc cgg gct tgt ggg gct ctc gct gct tta aag 672
Gly Asp Phe Leu Leu Ser Arg Ala Cys Gly Ala Leu Ala Ala Leu Lys
    210                 215                 220

aac aca gag gtt gta gca tta ctt gca act gct gta gaa cat ctt gtt 720
Asn Thr Glu Val Val Ala Leu Leu Ala Thr Ala Val Glu His Leu Val
225                 230                 235                 240

acc ggt gaa acc atg gag ata act agt tca acc gag cag cgt tat agt 768
Thr Gly Glu Thr Met Glu Ile Thr Ser Ser Thr Glu Gln Arg Tyr Ser
                245                 250                 255

atg gac tac tac atg cag aag aca tat tat aag aca gca tcg cta atc 816
Met Asp Tyr Tyr Met Gln Lys Thr Tyr Tyr Lys Thr Ala Ser Leu Ile
            260                 265                 270

tct aac agc tgc aaa gct gtt gcc gtt ctc act gga caa aca gca gaa 864
Ser Asn Ser Cys Lys Ala Val Ala Val Leu Thr Gly Gln Thr Ala Glu
        275                 280                 285

gtt gcc gtg tta gct ttt gag tat ggg agg aat ctg ggt tta gca ttc 912
Val Ala Val Leu Ala Phe Glu Tyr Gly Arg Asn Leu Gly Leu Ala Phe
    290                 295                 300

caa tta ata gac gac att ctt gat ttc acg ggc aca tct gcc tct ctc 960
Gln Leu Ile Asp Asp Ile Leu Asp Phe Thr Gly Thr Ser Ala Ser Leu
305                 310                 315                 320

gga aag gga tcg ttg tca gat att cgc cat gga gtc ata aca gcc cca 1008
Gly Lys Gly Ser Leu Ser Asp Ile Arg His Gly Val Ile Thr Ala Pro
                325                 330                 335

atc ctc ttt gcc atg gaa gag ttt cct caa cta cgc gaa gtt gtt gat 1056
Ile Leu Phe Ala Met Glu Glu Phe Pro Gln Leu Arg Glu Val Val Asp
            340                 345                 350

caa gtt gaa aaa gat cct agg aat gtt gac att gct tta gag tat ctt 1104
Gln Val Glu Lys Asp Pro Arg Asn Val Asp Ile Ala Leu Glu Tyr Leu
        355                 360                 365

ggg aag agc aag gga ata cag agg gca aga gaa tta gcc atg gaa cat 1152
Gly Lys Ser Lys Gly Ile Gln Arg Ala Arg Glu Leu Ala Met Glu His

370                 375                 380

gcg aat cta gca gca gct gca atc ggg tct cta cct gaa aca gac aat      1200
Ala Asn Leu Ala Ala Ala Ala Ile Gly Ser Leu Pro Glu Thr Asp Asn
385                 390                 395                 400

gaa gat gtc aaa aga tcg agg cgg gca ctt att gac ttg acc cat aga      1248
Glu Asp Val Lys Arg Ser Arg Arg Ala Leu Ile Asp Leu Thr His Arg
                405                 410                 415

gtc atc acc aga aac aag tga                                          1269
Val Ile Thr Arg Asn Lys
            420

<210>  4
<211>  422
<212>  PRT
<213>  Arabidopsis thaliana

<400>  4

Met Leu Phe Thr Arg Ser Val Ala Arg Ile Ser Ser Lys Phe Leu Arg
1               5                   10                  15

Asn Arg Ser Phe Tyr Gly Ser Ser Gln Ser Leu Ala Ser His Arg Phe
            20                  25                  30

Ala Ile Ile Pro Asp Gln Gly His Ser Cys Ser Asp Ser Pro His Lys
        35                  40                  45

Gly Tyr Val Cys Arg Thr Thr Tyr Ser Leu Lys Ser Pro Val Phe Gly
    50                  55                  60

Gly Phe Ser His Gln Leu Tyr His Gln Ser Ser Ser Leu Val Glu Glu
65                  70                  75                  80

Glu Leu Asp Pro Phe Ser Leu Val Ala Asp Glu Leu Ser Leu Leu Ser
                85                  90                  95

Asn Lys Leu Arg Glu Met Val Leu Ala Glu Val Pro Lys Leu Ala Ser
            100                 105                 110

Ala Ala Glu Tyr Phe Phe Lys Arg Gly Val Gln Gly Lys Gln Phe Arg
        115                 120                 125

Ser Thr Ile Leu Leu Leu Met Ala Thr Ala Leu Asp Val Arg Val Pro
    130                 135                 140

Glu Ala Leu Ile Gly Glu Ser Thr Asp Ile Val Thr Ser Glu Leu Arg
145                 150                 155                 160

Val Arg Gln Arg Gly Ile Ala Glu Ile Thr Glu Met Ile His Val Ala

165                 170                 175

Ser Leu Leu His Asp Asp Val Leu Asp Asp Ala Asp Thr Arg Arg Gly
            180                 185                 190

Val Gly Ser Leu Asn Val Val Met Gly Asn Lys Met Ser Val Leu Ala
        195                 200                 205

Gly Asp Phe Leu Leu Ser Arg Ala Cys Gly Ala Leu Ala Ala Leu Lys
    210                 215                 220

Asn Thr Glu Val Val Ala Leu Leu Ala Thr Ala Val Glu His Leu Val
225                 230                 235                 240

Thr Gly Glu Thr Met Glu Ile Thr Ser Ser Thr Glu Gln Arg Tyr Ser
                245                 250                 255

Met Asp Tyr Tyr Met Gln Lys Thr Tyr Tyr Lys Thr Ala Ser Leu Ile
            260                 265                 270

Ser Asn Ser Cys Lys Ala Val Ala Val Leu Thr Gly Gln Thr Ala Glu
        275                 280                 285

Val Ala Val Leu Ala Phe Glu Tyr Gly Arg Asn Leu Gly Leu Ala Phe
    290                 295                 300

Gln Leu Ile Asp Asp Ile Leu Asp Phe Thr Gly Thr Ser Ala Ser Leu
305                 310                 315                 320

Gly Lys Gly Ser Leu Ser Asp Ile Arg His Gly Val Ile Thr Ala Pro
                325                 330                 335

Ile Leu Phe Ala Met Glu Glu Phe Pro Gln Leu Arg Glu Val Val Asp
            340                 345                 350

Gln Val Glu Lys Asp Pro Arg Asn Val Asp Ile Ala Leu Glu Tyr Leu
        355                 360                 365

Gly Lys Ser Lys Gly Ile Gln Arg Ala Arg Glu Leu Ala Met Glu His
    370                 375                 380

Ala Asn Leu Ala Ala Ala Ala Ile Gly Ser Leu Pro Glu Thr Asp Asn
385                 390                 395                 400

Glu Asp Val Lys Arg Ser Arg Arg Ala Leu Ile Asp Leu Thr His Arg
                405                 410                 415

Val Ile Thr Arg Asn Lys
            420

<210>  5
<211>  912
<212>  DNA
<213>  Solanum lycopersicum

<220>
<221>  CDS
<222>  (1)..(912)
<223>

<400>  5
atg agt tct ttg gtt ctt caa tgt tgg aaa tta tca tct cca tct ctg        48
Met Ser Ser Leu Val Leu Gln Cys Trp Lys Leu Ser Ser Pro Ser Leu
1               5                   10                  15

att tta caa caa aat aca tca ata tcc atg ggt gca ttc aaa ggt att        96
Ile Leu Gln Gln Asn Thr Ser Ile Ser Met Gly Ala Phe Lys Gly Ile
            20                  25                  30

cat aaa ctt caa atc cca aat tcg cct ctg aca gtg tct gct cgt gga       144
His Lys Leu Gln Ile Pro Asn Ser Pro Leu Thr Val Ser Ala Arg Gly
        35                  40                  45

ctc aac aag att tca tgc tca ctc aac tta caa acc gaa aag ctt tgt       192
Leu Asn Lys Ile Ser Cys Ser Leu Asn Leu Gln Thr Glu Lys Leu Cys
    50                  55                  60

tat gag gat aat gat aat gat ctt gat gaa gaa ctt atg cct aaa cac       240
Tyr Glu Asp Asn Asp Asn Asp Leu Asp Glu Glu Leu Met Pro Lys His
65                  70                  75                  80

att gct ttg ata atg gat ggt aat agg aga tgg gca aag gat aag ggt       288
Ile Ala Leu Ile Met Asp Gly Asn Arg Arg Trp Ala Lys Asp Lys Gly
                85                  90                  95

tta gaa gta tat gaa ggt cac aaa cat att att cca aaa tta aaa gag       336
Leu Glu Val Tyr Glu Gly His Lys His Ile Ile Pro Lys Leu Lys Glu
            100                 105                 110

att tgt gac att tct tct aaa ttg gga ata caa att atc act gct ttt       384
Ile Cys Asp Ile Ser Ser Lys Leu Gly Ile Gln Ile Ile Thr Ala Phe
        115                 120                 125

gca ttc tct act gaa aat tgg aaa cga tcc aag gag gag gtt gat ttc       432
Ala Phe Ser Thr Glu Asn Trp Lys Arg Ser Lys Glu Glu Val Asp Phe
    130                 135                 140

ttg ttg caa atg ttc gaa gaa atc tat gat gag ttt tcg agg tct gga       480
Leu Leu Gln Met Phe Glu Glu Ile Tyr Asp Glu Phe Ser Arg Ser Gly
145                 150                 155                 160

gta aga gtg tct att ata ggt tgt aaa tcc gac ctc cca atg aca tta       528
Val Arg Val Ser Ile Ile Gly Cys Lys Ser Asp Leu Pro Met Thr Leu
                165                 170                 175

caa aaa tgc ata gca tta aca gaa gag act aca aag ggc aac aaa gga       576
Gln Lys Cys Ile Ala Leu Thr Glu Glu Thr Thr Lys Gly Asn Lys Gly
            180                 185                 190

ctt cac ctt gtg att gca cta aac tat ggt gga tat tat gac ata ttg 624
Leu His Leu Val Ile Ala Leu Asn Tyr Gly Gly Tyr Tyr Asp Ile Leu
        195                 200                 205

caa gca aca aaa agc att gtt aat aaa gca atg aat ggt tta tta gat 672
Gln Ala Thr Lys Ser Ile Val Asn Lys Ala Met Asn Gly Leu Leu Asp
    210                 215                 220

gta gaa gat atc aac aag aat tta ttt gat caa gaa ctt gaa agc aag 720
Val Glu Asp Ile Asn Lys Asn Leu Phe Asp Gln Glu Leu Glu Ser Lys
225                 230                 235                 240

tgt cca aat cct gat tta ctt ata agg aca gga ggt gaa caa aga gtt 768
Cys Pro Asn Pro Asp Leu Leu Ile Arg Thr Gly Gly Glu Gln Arg Val
                245                 250                 255

agt aac ttt ttg ttg tgg caa ttg gct tac act gaa ttt tac ttc acc 816
Ser Asn Phe Leu Leu Trp Gln Leu Ala Tyr Thr Glu Phe Tyr Phe Thr
            260                 265                 270

aac aca ttg ttt cct gat ttt gga gag gaa gat ctt aaa gag gca ata 864
Asn Thr Leu Phe Pro Asp Phe Gly Glu Glu Asp Leu Lys Glu Ala Ile
        275                 280                 285

atg aac ttt caa caa agg cat aga cgt ttt ggt gga cac aca tat tga 912
Met Asn Phe Gln Gln Arg His Arg Arg Phe Gly Gly His Thr Tyr
    290                 295                 300

<210> 6
<211> 303
<212> PRT
<213> Solanum lycopersicum

<400> 6

Met Ser Ser Leu Val Leu Gln Cys Trp Lys Leu Ser Ser Pro Ser Leu
1               5                   10                  15

Ile Leu Gln Gln Asn Thr Ser Ile Ser Met Gly Ala Phe Lys Gly Ile
            20                  25                  30

His Lys Leu Gln Ile Pro Asn Ser Pro Leu Thr Val Ser Ala Arg Gly
        35                  40                  45

Leu Asn Lys Ile Ser Cys Ser Leu Asn Leu Gln Thr Glu Lys Leu Cys
    50                  55                  60

Tyr Glu Asp Asn Asp Asn Asp Leu Asp Glu Glu Leu Met Pro Lys His
65                  70                  75                  80

Ile Ala Leu Ile Met Asp Gly Asn Arg Arg Trp Ala Lys Asp Lys Gly
                85                  90                  95

Leu Glu Val Tyr Glu Gly His Lys His Ile Ile Pro Lys Leu Lys Glu
            100                 105                 110

Ile Cys Asp Ile Ser Ser Lys Leu Gly Ile Gln Ile Ile Thr Ala Phe
        115                 120                 125

Ala Phe Ser Thr Glu Asn Trp Lys Arg Ser Lys Glu Glu Val Asp Phe
    130                 135                 140

Leu Leu Gln Met Phe Glu Glu Ile Tyr Asp Glu Phe Ser Arg Ser Gly
145                 150                 155                 160

Val Arg Val Ser Ile Ile Gly Cys Lys Ser Asp Leu Pro Met Thr Leu
                165                 170                 175

Gln Lys Cys Ile Ala Leu Thr Glu Glu Thr Thr Lys Gly Asn Lys Gly
            180                 185                 190

Leu His Leu Val Ile Ala Leu Asn Tyr Gly Gly Tyr Tyr Asp Ile Leu
        195                 200                 205

Gln Ala Thr Lys Ser Ile Val Asn Lys Ala Met Asn Gly Leu Leu Asp
    210                 215                 220

Val Glu Asp Ile Asn Lys Asn Leu Phe Asp Gln Glu Leu Glu Ser Lys
225                 230                 235                 240

Cys Pro Asn Pro Asp Leu Leu Ile Arg Thr Gly Gly Glu Gln Arg Val
                245                 250                 255

Ser Asn Phe Leu Leu Trp Gln Leu Ala Tyr Thr Glu Phe Tyr Phe Thr
            260                 265                 270

Asn Thr Leu Phe Pro Asp Phe Gly Glu Glu Asp Leu Lys Glu Ala Ile
        275                 280                 285

Met Asn Phe Gln Gln Arg His Arg Arg Phe Gly Gly His Thr Tyr
    290                 295                 300

<210>  7
<211>  2337
<212>  DNA
<213>  Solanum lycopersicum

<220>
<221>  CDS
<222>  (1)..(2337)
<223>

<400>  7
atg ata gtt ggc tat aga agc aca atc ata acc ctt tct cat cct aag        48
Met Ile Val Gly Tyr Arg Ser Thr Ile Ile Thr Leu Ser His Pro Lys
1               5                   10                  15

cta ggc aat ggg aaa aca att tca tcc aat gca att ttc cag aga tca     96
Leu Gly Asn Gly Lys Thr Ile Ser Ser Asn Ala Ile Phe Gln Arg Ser
            20                  25                  30

tgt aga gta aga tgc agc cac agt acc act tca tca atg aat ggt ttc     144
Cys Arg Val Arg Cys Ser His Ser Thr Thr Ser Ser Met Asn Gly Phe
        35                  40                  45

gaa gat gca agg gat aga ata agg gaa agt ttt ggg aaa tta gag tta     192
Glu Asp Ala Arg Asp Arg Ile Arg Glu Ser Phe Gly Lys Leu Glu Leu
    50                  55                  60

tct cct tct tcc tat gac aca gca tgg gta gct atg gtc cct tca aga     240
Ser Pro Ser Ser Tyr Asp Thr Ala Trp Val Ala Met Val Pro Ser Arg
65                  70                  75                  80

cat tca cta aat gag cca tgt ttt cca caa tgt ttg gat tgg att att     288
His Ser Leu Asn Glu Pro Cys Phe Pro Gln Cys Leu Asp Trp Ile Ile
                85                  90                  95

gaa aat caa aga gaa gat gga tct tgg gga cta aac cct acc cat cca     336
Glu Asn Gln Arg Glu Asp Gly Ser Trp Gly Leu Asn Pro Thr His Pro
            100                 105                 110

ttg ctt cta aag gac tca ctt tct tcc act ctt gca tgt ttg ctt gca     384
Leu Leu Leu Lys Asp Ser Leu Ser Ser Thr Leu Ala Cys Leu Leu Ala
        115                 120                 125

cta acc aaa tgg aga gtt gga gat gag caa atc aaa aga ggt ctt ggc     432
Leu Thr Lys Trp Arg Val Gly Asp Glu Gln Ile Lys Arg Gly Leu Gly
    130                 135                 140

ttc att gaa acg tat ggt tgg gca gta gat aac aag gat caa att tca     480
Phe Ile Glu Thr Tyr Gly Trp Ala Val Asp Asn Lys Asp Gln Ile Ser
145                 150                 155                 160

cct tta gga ttt gaa gtt ata ttt tct agt atg atc aaa tct gca gag     528
Pro Leu Gly Phe Glu Val Ile Phe Ser Ser Met Ile Lys Ser Ala Glu
                165                 170                 175

aaa tta gat tta aat ttg cct ttg aat ctt cat ctt gta aat ttg gtg     576
Lys Leu Asp Leu Asn Leu Pro Leu Asn Leu His Leu Val Asn Leu Val
            180                 185                 190

aaa tgc aaa aga gat tca aca att aaa agg aat gtt gaa tat atg ggt     624
Lys Cys Lys Arg Asp Ser Thr Ile Lys Arg Asn Val Glu Tyr Met Gly
        195                 200                 205

gaa gga gtt ggt gaa tta tgt gat tgg aag gaa atg ata aag tta cat     672
Glu Gly Val Gly Glu Leu Cys Asp Trp Lys Glu Met Ile Lys Leu His
    210                 215                 220

caa aga caa aat ggt tca tta ttt gat tca cca gcc act act gca gct     720
Gln Arg Gln Asn Gly Ser Leu Phe Asp Ser Pro Ala Thr Thr Ala Ala
225                 230                 235                 240

gcc ttg att tat cat caa cat gat caa aaa tgc tat caa tat ctt aat     768
Ala Leu Ile Tyr His Gln His Asp Gln Lys Cys Tyr Gln Tyr Leu Asn
                245                 250                 255

tca atc ttc caa caa cac aaa aat tgg gtt ccc act atg tat cca aca     816
Ser Ile Phe Gln Gln His Lys Asn Trp Val Pro Thr Met Tyr Pro Thr

260                 265                 270

aag gta cat tca ttg ctt tgc ttg gtt gat aca ctt caa aat ctt gga      864
Lys Val His Ser Leu Leu Cys Leu Val Asp Thr Leu Gln Asn Leu Gly
        275                 280                 285

gta cat cgg cat ttt aaa tca gaa ata aag aaa gct cta gat gaa ata      912
Val His Arg His Phe Lys Ser Glu Ile Lys Lys Ala Leu Asp Glu Ile
    290                 295                 300

tac agg cta tgg caa caa aag aat gaa caa att ttc tca aat gtc acc      960
Tyr Arg Leu Trp Gln Gln Lys Asn Glu Gln Ile Phe Ser Asn Val Thr
305                 310                 315                 320

cat tgt gct atg gct ttt cga ctt cta agg atg agc tac tat gat gtc     1008
His Cys Ala Met Ala Phe Arg Leu Leu Arg Met Ser Tyr Tyr Asp Val
                325                 330                 335

tcc tca gat gaa cta gca gaa ttt gtg gat gaa gaa cat ttc ttt gca     1056
Ser Ser Asp Glu Leu Ala Glu Phe Val Asp Glu Glu His Phe Phe Ala
            340                 345                 350

aca aat ggg aaa tat aaa agt cat gtt gaa att ctt gaa ctc cac aaa     1104
Thr Asn Gly Lys Tyr Lys Ser His Val Glu Ile Leu Glu Leu His Lys
        355                 360                 365

gca tca caa ttg gct att gat cat gag aaa gat gac att ttg gat aaa     1152
Ala Ser Gln Leu Ala Ile Asp His Glu Lys Asp Asp Ile Leu Asp Lys
    370                 375                 380

ata aac aat tgg aca aga gct ttt atg gag caa aaa ctc tta aac aat     1200
Ile Asn Asn Trp Thr Arg Ala Phe Met Glu Gln Lys Leu Leu Asn Asn
385                 390                 395                 400

ggc ttc ata gat agg atg tca aag aaa gag gtg gaa ctt gct ttg agg     1248
Gly Phe Ile Asp Arg Met Ser Lys Lys Glu Val Glu Leu Ala Leu Arg
                405                 410                 415

aag ttt tat acc aca tct cat cta gca gaa aat aga aga tat ata aag     1296
Lys Phe Tyr Thr Thr Ser His Leu Ala Glu Asn Arg Arg Tyr Ile Lys
            420                 425                 430

tca tac gaa gag aac aat ttt aaa atc tta aaa gca gct tat agg tca     1344
Ser Tyr Glu Glu Asn Asn Phe Lys Ile Leu Lys Ala Ala Tyr Arg Ser
        435                 440                 445

ccc aac att aac aat aag gac ttg tta gca ttt tca ata cac gac ttt     1392
Pro Asn Ile Asn Asn Lys Asp Leu Leu Ala Phe Ser Ile His Asp Phe
    450                 455                 460

gaa tta tgc caa gct caa cac cga gaa gaa ctt caa caa ctc aag agg     1440
Glu Leu Cys Gln Ala Gln His Arg Glu Glu Leu Gln Gln Leu Lys Arg
465                 470                 475                 480

tgg ttt gaa gat tat aga ttg gac caa ctc gga ctt gca gaa cga tat     1488
Trp Phe Glu Asp Tyr Arg Leu Asp Gln Leu Gly Leu Ala Glu Arg Tyr
                485                 490                 495

ata cat gct agt tac tta ttt ggt gtt act gtt atc ccc gag cct gaa     1536
Ile His Ala Ser Tyr Leu Phe Gly Val Thr Val Ile Pro Glu Pro Glu
            500                 505                 510

tta tcc gat gct cgc ctc atg tac gcg aaa tac gtc atg ctc ctg act     1584

Leu Ser Asp Ala Arg Leu Met Tyr Ala Lys Tyr Val Met Leu Leu Thr
        515                 520                 525

att gtc gat gat cat ttc gag agt ttt gca tct aaa gat gaa tgt ttc      1632
Ile Val Asp Asp His Phe Glu Ser Phe Ala Ser Lys Asp Glu Cys Phe
    530                 535                 540

aac atc att gaa tta gta gaa agg tgg gat gac tat gca agt gta ggt      1680
Asn Ile Ile Glu Leu Val Glu Arg Trp Asp Asp Tyr Ala Ser Val Gly
545                 550                 555                 560

tat aaa tct gag aag gtt aaa gtt ttt ttt tct gtt ttc tat aaa tca      1728
Tyr Lys Ser Glu Lys Val Lys Val Phe Phe Ser Val Phe Tyr Lys Ser
                565                 570                 575

ata gag gag ctt gca aca att gct gaa att aaa caa gga cga tcc gtc      1776
Ile Glu Glu Leu Ala Thr Ile Ala Glu Ile Lys Gln Gly Arg Ser Val
            580                 585                 590

aaa aat cac ctt att aat ttg tgg ctt gaa ttg atg aag ttg atg ttg      1824
Lys Asn His Leu Ile Asn Leu Trp Leu Glu Leu Met Lys Leu Met Leu
        595                 600                 605

atg gag cga gta gag tgg tgt tct ggc aag aca ata cca agc ata gaa      1872
Met Glu Arg Val Glu Trp Cys Ser Gly Lys Thr Ile Pro Ser Ile Glu
    610                 615                 620

gag tac ttg tat gtt aca tct ata aca ttt tgt gca aaa ttg att cct      1920
Glu Tyr Leu Tyr Val Thr Ser Ile Thr Phe Cys Ala Lys Leu Ile Pro
625                 630                 635                 640

ctc tca aca caa tat ttt ctt gga ata aaa ata tcc aaa gat cta cta      1968
Leu Ser Thr Gln Tyr Phe Leu Gly Ile Lys Ile Ser Lys Asp Leu Leu
                645                 650                 655

gaa agt gat gaa ata tgt ggc cta tgg aat tgt agc ggt aga gtg atg      2016
Glu Ser Asp Glu Ile Cys Gly Leu Trp Asn Cys Ser Gly Arg Val Met
            660                 665                 670

cga atc ctt aat gat tta caa gat tcc aag aga gaa caa aag gag gtc      2064
Arg Ile Leu Asn Asp Leu Gln Asp Ser Lys Arg Glu Gln Lys Glu Val
        675                 680                 685

tca ata aat tta gtc aca tta cta atg aaa agt atg tct gag gaa gaa      2112
Ser Ile Asn Leu Val Thr Leu Leu Met Lys Ser Met Ser Glu Glu Glu
    690                 695                 700

gct ata atg aag ata aag gaa atc ttg gaa atg aat aga aga gag tta      2160
Ala Ile Met Lys Ile Lys Glu Ile Leu Glu Met Asn Arg Arg Glu Leu
705                 710                 715                 720

ttg aaa atg gtt tta gtt caa aaa aag gga agc caa ttg cct caa tta      2208
Leu Lys Met Val Leu Val Gln Lys Lys Gly Ser Gln Leu Pro Gln Leu
                725                 730                 735

tgc aaa gat ata ttt tgg agg aca agc aaa tgg gct cat ttc act tat      2256
Cys Lys Asp Ile Phe Trp Arg Thr Ser Lys Trp Ala His Phe Thr Tyr
            740                 745                 750

tca caa act gat gga tat aga att gca gag gaa atg aag aat cac att      2304
Ser Gln Thr Asp Gly Tyr Arg Ile Ala Glu Glu Met Lys Asn His Ile
        755                 760                 765

gat gaa gtc ttt tac aaa cca ctc aat cat taa 2337
Asp Glu Val Phe Tyr Lys Pro Leu Asn His
    770                 775

<210> 8
<211> 778
<212> PRT
<213> Solanum lycopersicum

<400> 8

Met Ile Val Gly Tyr Arg Ser Thr Ile Ile Thr Leu Ser His Pro Lys
1               5                   10                  15

Leu Gly Asn Gly Lys Thr Ile Ser Ser Asn Ala Ile Phe Gln Arg Ser
            20                  25                  30

Cys Arg Val Arg Cys Ser His Ser Thr Thr Ser Ser Met Asn Gly Phe
        35                  40                  45

Glu Asp Ala Arg Asp Arg Ile Arg Glu Ser Phe Gly Lys Leu Glu Leu
    50                  55                  60

Ser Pro Ser Ser Tyr Asp Thr Ala Trp Val Ala Met Val Pro Ser Arg
65                  70                  75                  80

His Ser Leu Asn Glu Pro Cys Phe Pro Gln Cys Leu Asp Trp Ile Ile
                85                  90                  95

Glu Asn Gln Arg Glu Asp Gly Ser Trp Gly Leu Asn Pro Thr His Pro
            100                 105                 110

Leu Leu Leu Lys Asp Ser Leu Ser Ser Thr Leu Ala Cys Leu Leu Ala
        115                 120                 125

Leu Thr Lys Trp Arg Val Gly Asp Glu Gln Ile Lys Arg Gly Leu Gly
    130                 135                 140

Phe Ile Glu Thr Tyr Gly Trp Ala Val Asp Asn Lys Asp Gln Ile Ser
145                 150                 155                 160

Pro Leu Gly Phe Glu Val Ile Phe Ser Ser Met Ile Lys Ser Ala Glu
                165                 170                 175

Lys Leu Asp Leu Asn Leu Pro Leu Asn Leu His Leu Val Asn Leu Val
            180                 185                 190

Lys Cys Lys Arg Asp Ser Thr Ile Lys Arg Asn Val Glu Tyr Met Gly
        195                 200                 205

Glu Gly Val Gly Glu Leu Cys Asp Trp Lys Glu Met Ile Lys Leu His
    210                 215                 220

Gln Arg Gln Asn Gly Ser Leu Phe Asp Ser Pro Ala Thr Thr Ala Ala
225                 230                 235                 240

Ala Leu Ile Tyr His Gln His Asp Gln Lys Cys Tyr Gln Tyr Leu Asn
                245                 250                 255

Ser Ile Phe Gln Gln His Lys Asn Trp Val Pro Thr Met Tyr Pro Thr
            260                 265                 270

Lys Val His Ser Leu Leu Cys Leu Val Asp Thr Leu Gln Asn Leu Gly
        275                 280                 285

Val His Arg His Phe Lys Ser Glu Ile Lys Lys Ala Leu Asp Glu Ile
    290                 295                 300

Tyr Arg Leu Trp Gln Gln Lys Asn Glu Gln Ile Phe Ser Asn Val Thr
305                 310                 315                 320

His Cys Ala Met Ala Phe Arg Leu Leu Arg Met Ser Tyr Tyr Asp Val
                325                 330                 335

Ser Ser Asp Glu Leu Ala Glu Phe Val Asp Glu Glu His Phe Phe Ala
            340                 345                 350

Thr Asn Gly Lys Tyr Lys Ser His Val Glu Ile Leu Glu Leu His Lys
        355                 360                 365

Ala Ser Gln Leu Ala Ile Asp His Glu Lys Asp Asp Ile Leu Asp Lys
    370                 375                 380

Ile Asn Asn Trp Thr Arg Ala Phe Met Glu Gln Lys Leu Leu Asn Asn
385                 390                 395                 400

Gly Phe Ile Asp Arg Met Ser Lys Lys Glu Val Glu Leu Ala Leu Arg
                405                 410                 415

Lys Phe Tyr Thr Thr Ser His Leu Ala Glu Asn Arg Arg Tyr Ile Lys
            420                 425                 430

Ser Tyr Glu Glu Asn Asn Phe Lys Ile Leu Lys Ala Ala Tyr Arg Ser
        435                 440                 445

Pro Asn Ile Asn Asn Lys Asp Leu Leu Ala Phe Ser Ile His Asp Phe
    450                 455                 460

Glu Leu Cys Gln Ala Gln His Arg Glu Glu Leu Gln Gln Leu Lys Arg
465                 470                 475                 480

Trp Phe Glu Asp Tyr Arg Leu Asp Gln Leu Gly Leu Ala Glu Arg Tyr
                485                 490                 495

Ile His Ala Ser Tyr Leu Phe Gly Val Thr Val Ile Pro Glu Pro Glu
            500                 505                 510

Leu Ser Asp Ala Arg Leu Met Tyr Ala Lys Tyr Val Met Leu Leu Thr
        515                 520                 525

Ile Val Asp Asp His Phe Glu Ser Phe Ala Ser Lys Asp Glu Cys Phe
    530                 535                 540

Asn Ile Ile Glu Leu Val Glu Arg Trp Asp Asp Tyr Ala Ser Val Gly
545                 550                 555                 560

Tyr Lys Ser Glu Lys Val Lys Val Phe Phe Ser Val Phe Tyr Lys Ser
                565                 570                 575

Ile Glu Glu Leu Ala Thr Ile Ala Glu Ile Lys Gln Gly Arg Ser Val
            580                 585                 590

Lys Asn His Leu Ile Asn Leu Trp Leu Glu Leu Met Lys Leu Met Leu
        595                 600                 605

Met Glu Arg Val Glu Trp Cys Ser Gly Lys Thr Ile Pro Ser Ile Glu
    610                 615                 620

Glu Tyr Leu Tyr Val Thr Ser Ile Thr Phe Cys Ala Lys Leu Ile Pro
625                 630                 635                 640

Leu Ser Thr Gln Tyr Phe Leu Gly Ile Lys Ile Ser Lys Asp Leu Leu
                645                 650                 655

Glu Ser Asp Glu Ile Cys Gly Leu Trp Asn Cys Ser Gly Arg Val Met
            660                 665                 670

Arg Ile Leu Asn Asp Leu Gln Asp Ser Lys Arg Glu Gln Lys Glu Val
        675                 680                 685

Ser Ile Asn Leu Val Thr Leu Leu Met Lys Ser Met Ser Glu Glu Glu
    690                 695                 700

Ala Ile Met Lys Ile Lys Glu Ile Leu Glu Met Asn Arg Arg Glu Leu

705                 710                 715                 720

Leu Lys Met Val Leu Val Gln Lys Lys Gly Ser Gln Leu Pro Gln Leu
                725                 730                 735

Cys Lys Asp Ile Phe Trp Arg Thr Ser Lys Trp Ala His Phe Thr Tyr
            740                 745                 750

Ser Gln Thr Asp Gly Tyr Arg Ile Ala Glu Glu Met Lys Asn His Ile
        755                 760                 765

Asp Glu Val Phe Tyr Lys Pro Leu Asn His
    770                 775

<210>  9
<211>  1746
<212>  DNA
<213>  Lavandula angustifolia

<220>
<221>  CDS
<222>  (1)..(1746)
<223>

<400>  9
atg tct acc att att gcg ata caa gtg ttg ctt cct att cca act act      48
Met Ser Thr Ile Ile Ala Ile Gln Val Leu Leu Pro Ile Pro Thr Thr
1               5                   10                  15

aaa aca tac cct agt cat gac ttg gag aag tcc tct tcg cgg tgt cgc      96
Lys Thr Tyr Pro Ser His Asp Leu Glu Lys Ser Ser Ser Arg Cys Arg
            20                  25                  30

tcc tcc tcc act cct cgc cct aga ctg tgt tgc tcg ttg cag gtg agt      144
Ser Ser Ser Thr Pro Arg Pro Arg Leu Cys Cys Ser Leu Gln Val Ser
        35                  40                  45

gat ccg atc cca acg ggc cgg cga tcc gga ggc tac ccg ccc gcc cta      192
Asp Pro Ile Pro Thr Gly Arg Arg Ser Gly Gly Tyr Pro Pro Ala Leu
    50                  55                  60

tgg gat ttc gac act att caa tcg ctc aac acc gag tat aag gga gag      240
Trp Asp Phe Asp Thr Ile Gln Ser Leu Asn Thr Glu Tyr Lys Gly Glu
65                  70                  75                  80

agg cac atg aga agg gaa gaa gac cta att ggg caa gtt aga gag atg      288
Arg His Met Arg Arg Glu Glu Asp Leu Ile Gly Gln Val Arg Glu Met
                85                  90                  95

ctg gtg cat gaa gta gag gat ccc act cca cag ctg gag ttc att gat      336
Leu Val His Glu Val Glu Asp Pro Thr Pro Gln Leu Glu Phe Ile Asp
            100                 105                 110

gat ttg cat aag ctt ggc ata tct tgc cat ttt gag aat gaa atc ctc      384
Asp Leu His Lys Leu Gly Ile Ser Cys His Phe Glu Asn Glu Ile Leu
        115                 120                 125

caa atc ttg aaa tcc ata tat ctt aat caa aac tac aaa agg gat ttg      432

Gln Ile Leu Lys Ser Ile Tyr Leu Asn Gln Asn Tyr Lys Arg Asp Leu
    130                 135                 140

tac tca aca tct cta gca ttc aga ctc ctc aga caa tat ggc ttc atc      480
Tyr Ser Thr Ser Leu Ala Phe Arg Leu Leu Arg Gln Tyr Gly Phe Ile
145                 150                 155                 160

ctt cca caa gaa gta ttt gat tgt ttc aag aat gag gag ggt acg gat      528
Leu Pro Gln Glu Val Phe Asp Cys Phe Lys Asn Glu Glu Gly Thr Asp
                165                 170                 175

ttc aag cca agc ttc ggc cgt gat atc aaa ggc ttg tta caa ttg tat      576
Phe Lys Pro Ser Phe Gly Arg Asp Ile Lys Gly Leu Leu Gln Leu Tyr
            180                 185                 190

gaa gct tct ttc cta tca aga aaa gga gaa gaa act tta caa cta gca      624
Glu Ala Ser Phe Leu Ser Arg Lys Gly Glu Glu Thr Leu Gln Leu Ala
        195                 200                 205

aga gag ttt gca aca aag att ctg caa aaa gaa gtt gat gag aga gag      672
Arg Glu Phe Ala Thr Lys Ile Leu Gln Lys Glu Val Asp Glu Arg Glu
    210                 215                 220

ttt gca acc aag atg gag ttc cct tct cat tgg acg gtt caa atg ccg      720
Phe Ala Thr Lys Met Glu Phe Pro Ser His Trp Thr Val Gln Met Pro
225                 230                 235                 240

aat gca aga cct ttc atc gat gct tac cgt agg agg ccg gat atg aat      768
Asn Ala Arg Pro Phe Ile Asp Ala Tyr Arg Arg Arg Pro Asp Met Asn
                245                 250                 255

cca gtt gtg ctc gag cta gcc ata ctt gat aca aat ata gtt caa gca      816
Pro Val Val Leu Glu Leu Ala Ile Leu Asp Thr Asn Ile Val Gln Ala
            260                 265                 270

caa ttt caa gaa gaa ctc aaa gag acc tca agg tgg tgg gag agt aca      864
Gln Phe Gln Glu Glu Leu Lys Glu Thr Ser Arg Trp Trp Glu Ser Thr
        275                 280                 285

ggc att gtc caa gag ctt cca ttt gtg agg gat agg att gtg gaa ggc      912
Gly Ile Val Gln Glu Leu Pro Phe Val Arg Asp Arg Ile Val Glu Gly
    290                 295                 300

tac ttt tgg acg att gga gtg act cag aga cgc gag cat gga tac gaa      960
Tyr Phe Trp Thr Ile Gly Val Thr Gln Arg Arg Glu His Gly Tyr Glu
305                 310                 315                 320

aga atc atg acc gca aag gtt att gcc tta gta aca tgt tta gac gac     1008
Arg Ile Met Thr Ala Lys Val Ile Ala Leu Val Thr Cys Leu Asp Asp
                325                 330                 335

ata tac gat gtt tat ggc acg ata gaa gag ctt caa ctt ttc aca agc     1056
Ile Tyr Asp Val Tyr Gly Thr Ile Glu Glu Leu Gln Leu Phe Thr Ser
            340                 345                 350

aca atc caa aga tgg gat ttg gaa tca atg aag caa ctc cct acc tac     1104
Thr Ile Gln Arg Trp Asp Leu Glu Ser Met Lys Gln Leu Pro Thr Tyr
        355                 360                 365

atg caa gta agc ttt ctt gca cta cac aac ttt gta acc gag gtg gct     1152
Met Gln Val Ser Phe Leu Ala Leu His Asn Phe Val Thr Glu Val Ala
    370                 375                 380

tac gat act ctc aag aaa aag ggc tac aac tcc aca cca tat tta aga 1200
Tyr Asp Thr Leu Lys Lys Lys Gly Tyr Asn Ser Thr Pro Tyr Leu Arg
385                 390                 395                 400

aaa acg tgg gtg gat ctt gtt gaa tca tat atc aaa gag gca act tgg 1248
Lys Thr Trp Val Asp Leu Val Glu Ser Tyr Ile Lys Glu Ala Thr Trp
                405                 410                 415

tac tac aac ggt tat aaa cct agt atg caa gaa tac ctt aac aat gca 1296
Tyr Tyr Asn Gly Tyr Lys Pro Ser Met Gln Glu Tyr Leu Asn Asn Ala
            420                 425                 430

tgg ata tca gtc gga agt atg gct ata ctc aac cac ctc ttc ttc cgg 1344
Trp Ile Ser Val Gly Ser Met Ala Ile Leu Asn His Leu Phe Phe Arg
        435                 440                 445

ttc aca aac gag aga atg cat aaa tac cgc gat atg aac cgt gtc tcg 1392
Phe Thr Asn Glu Arg Met His Lys Tyr Arg Asp Met Asn Arg Val Ser
    450                 455                 460

tcc aac att gtg agg ctt gct gat gat atg gga aca tca ttg gct gag 1440
Ser Asn Ile Val Arg Leu Ala Asp Asp Met Gly Thr Ser Leu Ala Glu
465                 470                 475                 480

gtg gag aga ggg gac gtg ccg aaa gca att caa tgc tac atg aat gag 1488
Val Glu Arg Gly Asp Val Pro Lys Ala Ile Gln Cys Tyr Met Asn Glu
                485                 490                 495

acg aat gct tct gaa gaa gaa gca aga gaa tat gta aga aga gtc ata 1536
Thr Asn Ala Ser Glu Glu Glu Ala Arg Glu Tyr Val Arg Arg Val Ile
            500                 505                 510

cag gaa gaa tgg gaa aag ttg aac aca gaa ttg atg cgg gat gat gat 1584
Gln Glu Glu Trp Glu Lys Leu Asn Thr Glu Leu Met Arg Asp Asp Asp
        515                 520                 525

gat gat gat gat ttt aca cta tcc aaa tat tac tgt gag gtg gtt gct 1632
Asp Asp Asp Asp Phe Thr Leu Ser Lys Tyr Tyr Cys Glu Val Val Ala
    530                 535                 540

aat ctt aca aga atg gca cag ttt ata tac caa gat gga tcg gat ggc 1680
Asn Leu Thr Arg Met Ala Gln Phe Ile Tyr Gln Asp Gly Ser Asp Gly
545                 550                 555                 560

ttc ggc atg aaa gat tcc aag gtt aat aga ctg cta aaa gag acg ttg 1728
Phe Gly Met Lys Asp Ser Lys Val Asn Arg Leu Leu Lys Glu Thr Leu
                565                 570                 575

atc gag cgc tac gaa taa 1746
Ile Glu Arg Tyr Glu
            580

<210> 10
<211> 581
<212> PRT
<213> Lavandula angustifolia

<400> 10

Met Ser Thr Ile Ile Ala Ile Gln Val Leu Leu Pro Ile Pro Thr Thr
1               5                   10                  15

Lys Thr Tyr Pro Ser His Asp Leu Glu Lys Ser Ser Ser Arg Cys Arg
            20                  25                  30

Ser Ser Ser Thr Pro Arg Pro Arg Leu Cys Cys Ser Leu Gln Val Ser
        35                  40                  45

Asp Pro Ile Pro Thr Gly Arg Arg Ser Gly Gly Tyr Pro Pro Ala Leu
    50                  55                  60

Trp Asp Phe Asp Thr Ile Gln Ser Leu Asn Thr Glu Tyr Lys Gly Glu
65                  70                  75                  80

Arg His Met Arg Arg Glu Glu Asp Leu Ile Gly Gln Val Arg Glu Met
                85                  90                  95

Leu Val His Glu Val Glu Asp Pro Thr Pro Gln Leu Glu Phe Ile Asp
            100                 105                 110

Asp Leu His Lys Leu Gly Ile Ser Cys His Phe Glu Asn Glu Ile Leu
        115                 120                 125

Gln Ile Leu Lys Ser Ile Tyr Leu Asn Gln Asn Tyr Lys Arg Asp Leu
    130                 135                 140

Tyr Ser Thr Ser Leu Ala Phe Arg Leu Leu Arg Gln Tyr Gly Phe Ile
145                 150                 155                 160

Leu Pro Gln Glu Val Phe Asp Cys Phe Lys Asn Glu Glu Gly Thr Asp
                165                 170                 175

Phe Lys Pro Ser Phe Gly Arg Asp Ile Lys Gly Leu Leu Gln Leu Tyr
            180                 185                 190

Glu Ala Ser Phe Leu Ser Arg Lys Gly Glu Glu Thr Leu Gln Leu Ala
        195                 200                 205

Arg Glu Phe Ala Thr Lys Ile Leu Gln Lys Glu Val Asp Glu Arg Glu
    210                 215                 220

Phe Ala Thr Lys Met Glu Phe Pro Ser His Trp Thr Val Gln Met Pro
225                 230                 235                 240

Asn Ala Arg Pro Phe Ile Asp Ala Tyr Arg Arg Arg Pro Asp Met Asn
                245                 250                 255

Pro Val Val Leu Glu Leu Ala Ile Leu Asp Thr Asn Ile Val Gln Ala
            260                 265                 270

Gln Phe Gln Glu Glu Leu Lys Glu Thr Ser Arg Trp Trp Glu Ser Thr
        275                 280                 285

Gly Ile Val Gln Glu Leu Pro Phe Val Arg Asp Arg Ile Val Glu Gly
    290                 295                 300

Tyr Phe Trp Thr Ile Gly Val Thr Gln Arg Arg Glu His Gly Tyr Glu
305                 310                 315                 320

Arg Ile Met Thr Ala Lys Val Ile Ala Leu Val Thr Cys Leu Asp Asp
                325                 330                 335

Ile Tyr Asp Val Tyr Gly Thr Ile Glu Glu Leu Gln Leu Phe Thr Ser
            340                 345                 350

Thr Ile Gln Arg Trp Asp Leu Glu Ser Met Lys Gln Leu Pro Thr Tyr
        355                 360                 365

Met Gln Val Ser Phe Leu Ala Leu His Asn Phe Val Thr Glu Val Ala
    370                 375                 380

Tyr Asp Thr Leu Lys Lys Lys Gly Tyr Asn Ser Thr Pro Tyr Leu Arg
385                 390                 395                 400

Lys Thr Trp Val Asp Leu Val Glu Ser Tyr Ile Lys Glu Ala Thr Trp
                405                 410                 415

Tyr Tyr Asn Gly Tyr Lys Pro Ser Met Gln Glu Tyr Leu Asn Asn Ala
            420                 425                 430

Trp Ile Ser Val Gly Ser Met Ala Ile Leu Asn His Leu Phe Phe Arg
        435                 440                 445

Phe Thr Asn Glu Arg Met His Lys Tyr Arg Asp Met Asn Arg Val Ser
    450                 455                 460

Ser Asn Ile Val Arg Leu Ala Asp Asp Met Gly Thr Ser Leu Ala Glu
465                 470                 475                 480

Val Glu Arg Gly Asp Val Pro Lys Ala Ile Gln Cys Tyr Met Asn Glu
                485                 490                 495

Thr Asn Ala Ser Glu Glu Glu Ala Arg Glu Tyr Val Arg Arg Val Ile
            500                 505                 510

Gln Glu Glu Trp Glu Lys Leu Asn Thr Glu Leu Met Arg Asp Asp Asp

515                 520                 525

Asp Asp Asp Asp Phe Thr Leu Ser Lys Tyr Tyr Cys Glu Val Val Ala
    530                 535                 540

Asn Leu Thr Arg Met Ala Gln Phe Ile Tyr Gln Asp Gly Ser Asp Gly
545                 550                 555                 560

Phe Gly Met Lys Asp Ser Lys Val Asn Arg Leu Leu Lys Glu Thr Leu
                565                 570                 575

Ile Glu Arg Tyr Glu
            580

<210>  11
<211>  5803
<212>  DNA
<213>  Artificial

<220>
<223>  Gene cluster

<400>  11
tcgcgcgttt cggtgatgac ggtgaaaacc tctgacacat gcagctcccg gagacggtca      60

cagcttgtct gtaagcggat gccgggagca gacaagcccg tcagggcgcg tcagcgggtg     120

ttggcgggtg tcggggctgg cttaactatg cggcatcaga gcagattgta ctgagagtgc     180

accatatgcg gtgtgaaata ccgcacagat gcgtaaggag aaaataccgc atcaggcgcc     240

attcgccatt caggctgcgc aactgttggg aagggcgatc ggtgcgggcc tcttcgctat     300

tacgccagct ggcgaaaggg ggatgtgctg caaggcgatt aagttgggta acgccagggt     360

tttcccagtc acgacgttgt aaaacgacgg ccagtgaatt ggagatcggt acttcgcgaa     420

tgcgtcgaga tggcgcgcct tggtaaagga tatatggtag tatttgcagg ggatacatag     480

ggagatatag aagttctacc aataggttca gaatttagcc atagttctga tctaaaagct     540

gtactagacg tatttgatac tgtaaaggta tttatatata acttaactcc tgaatcctct     600

gggtttatca agcctcccca agcatttaaa ttacgttcta gaattagaaa gggggtatga     660

cccataaaat attttccttt aaaagattca tatacacgat agggtaggtt tactacttta     720

gttggtttat catgatgact tacggtatcg gaatttaaaa tgttttgatt ttccataaat     780

atgacctcct agtatttagt attattttat gtaaatatat atgtagaagt gtaccatttg     840

tgcaagattt caataaaggg tatattttac ctattttttt agtataaaaa atgcaaaaaa     900

tatgaacaaa agtagagttc ctatgtatta aattgtaaaa tatccactaa aaaaataaaa     960

ttataataaa aaatacaaaa aaataattga caatatataa ataattatgc ataattatat    1020

catgataaca attagttaag cataattaca tatatatgaa cataatatga catcttagaa    1080

gcatatcttt cgttagtaat aatataattt cctttagaag aaaatgattt atttaaaata 1140

aatagtgtaa tgttttttat aatttcaaaa agttccccaa tttagcatac taggcatgat 1200

aaaaatagct tgaataagtg cccgggatta tttattgata catagagaat ttcactcttt 1260

gcattttatc taacatcaag gggtttattt gtcacaaatt atgtaaaaat aaaacaaaga 1320

tgtaagaaag tcctatgata taaattttgt aaacataata aattagcttt cataagattg 1380

gaagaatgat aattactact tagaactgct aaaaattagg aaagaggtgt cgttaattat 1440

accgttcgta taatgtatgc tatacgaagt tatttcagat taaatttttg cttatttgat 1500

ttacattata taatattgag taaagtattg actagcaaaa ttttttgata ctttaatttg 1560

tgaaatttct tatcaaaagt tatatttttg aatgattttt attgaaaaat acaactaaaa 1620

aggattatag tataagtgtg tgtaattttg tgttaaattt aaagggagga aatgaacatg 1680

aactttaata aaattgattt agacaattgg aagagaaaag agatatttaa tcattatttg 1740

aaccaacaaa cgacttttag tataaccaca gaaattgata ttagtgtttt atacagaaac 1800

ataaaacaag aaggatataa attttaccct gcatttattt tcttagtgac aagggtgata 1860

aactcaaata cagcttttag aactggttac aatagcgacg gagagttagg ttattgggat 1920

aagttagagc cactttatac aatttttgat ggtgtatcta aaacattctc tggtatttgg 1980

actcctgtaa agaatgactt caaagagttt tatgatttat acctttctga tgtagagaaa 2040

tataatggtt cggggaaatt gtttcccaaa acacctatac ctgaaaatgc tttttctctt 2100

tctattattc catggacttc atttactggg tttaacttaa atatcaataa taatagtaat 2160

taccttctac ccattattac agcaggaaaa ttcattaata aaggtaattc aatatattta 2220

ccgctatctt tacaggtaca tcattctgtt tgtgatggtt atcatgcagg attgtttatg 2280

aactctattc aggaattgtc agataggcct aatgactggc ttttataatt taaaagcaaa 2340

tataaatgaa aaattgaacc ctagcattat gtaaatgcag ggtttaattt ttatattaag 2400

cagcataata gaaagttttt taaatgcatg tatatatggg gtatttaaag ggaaatctat 2460

aatataattt aggactatat aacttcgtat aatgtatgct atacgaacgg tacctaggat 2520

atataataaa ttgaatatag taaacaaaaa gggacatatt tataatatgt tctttttagt 2580

ttaatactca atttttgcac ataagaaatt aacttaatat aaaaaaattt gcgaagcttt 2640

gcttcgcagt ttaatattgt ttaggtggtt aaattatgaa tctggaagtg ttaaaaacag 2700

agtttaagta tttaagagat aaaataattg aaaagcaata tgaacatctt gatcctatgc 2760

aaagaaaagc agttttaaat ggtgaaaata actgtattgt tattgcttgt cctggagcag 2820

gaaagaccca gactattatt aatagagtgg actacttatg tagattcggt cctatataca 2880

atacagatta tgtacctaat tgtctaaaga ccgatgattt acagataatg aagaaatatt 2940

taaatgataa ttcttttaaa gatgtgactg cagtaaataa aattgagcat ttgttaaata 3000

gcaataaaat aaatccacag aacatagttg ttataacttt tactagagca gctgctctca 3060

atatgaaaaa cagatacata tctataggaa ataaagaaaa gtcacctttt tttggaacat 3120

tccactccct attttataat atattgaaaa agcataataa agaaataaat attatagatc 3180

cttataaggc acatgagata gttaaaaata cacttatgta ttatctggac tttataggag 3240

aagagagagt aaaggaagtt ctaaatgaca tatctctttt aaaaaatagt gaaactaaca 3300

tagatttatt taaaagtaaa attgacaaaa gtgtattttt aaaatgtttt aatgaatatg 3360

aaaattataa agctagaaat aagcttatgg attttgatga tttacaatta aaagttaaag 3420

atatgtttct aaatcagaaa tctattctag atagttatca gaatttgttc aagtatattt 3480

tagttgatga gtttcaggat tcagataacc tccaaatatt cgaaatcgga tgccgggacc 3540

gacgagtgca gaggcgtgca agcgagcttg gcgtaatcat ggtcatagct gtttcctgtg 3600

tgaaattgtt atccgctcac aattccacac aacatacgag ccggaagcat aaagtgtaaa 3660

gcctggggtg cctaatgagt gagctaactc acattaattg cgttgcgctc actgcccgct 3720

ttccagtcgg gaaacctgtc gtgccagctg cattaatgaa tcggccaacg cgcggggaga 3780

ggcggtttgc gtattgggcg ctcttccgct tcctcgctca ctgactcgct gcgctcggtc 3840

gttcggctgc ggcgagcggt atcagctcac tcaaaggcgg taatacggtt atccacagaa 3900

tcaggggata acgcaggaaa gaacatgtga gcaaaaggcc agcaaaaggc caggaaccgt 3960

aaaaaggccg cgttgctggc gtttttccat aggctccgcc cccctgacga gcatcacaaa 4020

aatcgacgct caagtcagag gtggcgaaac ccgacaggac tataaagata ccaggcgttt 4080

ccccctggaa gctccctcgt gcgctctcct gttccgaccc tgccgcttac cggatacctg 4140

tccgcctttc tcccttcggg aagcgtggcg ctttctcata gctcacgctg taggtatctc 4200

agttcggtgt aggtcgttcg ctccaagctg ggctgtgtgc acgaaccccc cgttcagccc 4260

gaccgctgcg ccttatccgg taactatcgt cttgagtcca acccggtaag acacgactta 4320

tcgccactgg cagcagccac tggtaacagg attagcagag cgaggtatgt aggcggtgct 4380

acagagttct tgaagtggtg gcctaactac ggctacacta gaagaacagt atttggtatc 4440

tgcgctctgc tgaagccagt taccttcgga aaaagagttg gtagctcttg atccggcaaa 4500

caaaccaccg ctggtagcgg tggttttttt gtttgcaagc agcagattac gcgcagaaaa 4560

aaaggatctc aagaagatcc tttgatcttt tctacggggt ctgacgctca gtggaacgaa 4620

aactcacgtt aagggatttt ggtcatgaga ttatcaaaaa ggatcttcac ctagatcctt 4680

ttaaattaaa aatgaagttt taaatcaatc taaagtatat atgagtaaac ttggtctgac 4740

agttaccaat gcttaatcag tgaggcacct atctcagcga tctgtctatt tcgttcatcc 4800

atagttgcct gactccccgt cgtgtagata actacgatac gggagggctt accatctggc 4860

cccagtgctg caatgatacc gcgagaccca cgctcaccgg ctccagattt atcagcaata 4920

aaccagccag ccggaagggc cgagcgcaga agtggtcctg caactttatc cgcctccatc 4980

cagtctatta attgttgccg ggaagctaga gtaagtagtt cgccagttaa tagtttgcgc 5040

aacgttgttg ccattgctac aggcatcgtg gtgtcacgct cgtcgtttgg tatggcttca 5100

ttcagctccg gttcccaacg atcaaggcga gttacatgat cccccatgtt gtgcaaaaaa 5160

gcggttagct ccttcggtcc tccgatcgtt gtcagaagta agttggccgc agtgttatca 5220

ctcatggtta tggcagcact gcataattct cttactgtca tgccatccgt aagatgcttt 5280

tctgtgactg gtgagtactc aaccaagtca ttctgagaat agtgtatgcg gcgaccgagt 5340

tgctcttgcc cggcgtcaat acgggataat accgcgccac atagcagaac tttaaaagtg 5400

ctcatcattg gaaaacgttc ttcggggcga aaactctcaa ggatcttacc gctgttgaga 5460

tccagttcga tgtaacccac tcgtgcaccc aactgatctt cagcatcttt tactttcacc 5520

agcgtttctg ggtgagcaaa aacaggaagg caaaatgccg caaaaaaggg aataagggcg 5580

acacggaaat gttgaatact catactcttc ctttttcaat attattgaag catttatcag 5640

ggttattgtc tcatgagcgg atacatattt gaatgtattt agaaaaataa acaaataggg 5700

gttccgcgca catttccccg aaaagtgcca cctgacgtct aagaaaccat tattatcatg 5760

acattaacct ataaaaatag gcgtatcacg aggccctttc gtc 5803

<210> 12
<211> 10704
<212> DNA
<213> Artificial

<220>
<223> Gene cluster

<400> 12
gaattcgagc tcggtacccg gggatcctct agagtcgacc tgcaggcatg cccgcggtcg 60

actttttaac aaaatatatt gataaaaata ataatagtgg gtataattaa gttgttagag 120

aaaacgtata aattagggat aaactatgga acttatgaaa tagattgaaa tggtttatct 180

gttaccccgt aggatccaga atttaaaagg agggattaaa catatgaatg gtttcgaaga 240

tgcaagggat agaataaggg aaagttttgg gaaattagag ttatctcctt cttcctatga 300

cacagcatgg gtagctatgg tcccttcaag acattcacta aatgagccat gttttccaca 360

atgtttggat tggattattg aaaatcaaag agaagatgga tcttggggac taaaccctac 420

ccatccattg cttctaaagg actcactttc ttccactctt gcatgtttgc ttgcactaac 480

caaatggaga gttggagatg agcaaatcaa aagaggtctt ggcttcattg aaacgtatgg 540

ttgggcagta gataacaagg atcaaatttc acctttagga tttgaagtta tattttctag 600

tatgatcaaa tctgcagaga aattagattt aaatttgcct ttgaatcttc atcttgtaaa 660

tttggtgaaa tgcaaaagag attcaacaat taaaaggaat gttgaatata tgggtgaagg 720

agttggtgaa ttatgtgatt ggaaggaaat gataaagtta catcaaagac aaaatggttc 780

attatttgat tcaccagcca ctactgcagc tgccttgatt tatcatcaac atgatcaaaa 840

atgctatcaa tatcttaatt caatcttcca acaacacaaa aattgggttc ccactatgta 900

tccaacaaag gtacattcat tgctttgctt ggttgataca cttcaaaatc ttggagtaca 960

taggcatttt aaatcagaaa taaagaaagc tctagatgaa atatacaggc tatggcaaca 1020

aaagaatgaa caaattttct caaatgtcac ccattgtgct atggctttta gacttctaag 1080

gatgagctac tatgatgtct cctcagatga actagcagaa tttgtggatg aagaacattt 1140

ctttgcaaca aatgggaaat ataaaagtca tgttgaaatt cttgaactcc acaaagcatc 1200

acaattggct attgatcatg agaaagatga cattttggat aaaataaaca attggacaag 1260

agcttttatg gagcaaaaac tcttaaacaa tggcttcata gataggatgt caaagaaaga 1320

ggtggaactt gctttgagga agttttatac cacatctcat ctagcagaaa atagaagata 1380

tataaagtca tacgaagaga acaattttaa aatcttaaaa gcagcttata ggtcacccaa 1440

cattaacaat aaggacttgt tagcattttc aatacacgac tttgaattat gccaagctca 1500

acacagagaa gaacttcaac aactcaagag gtggtttgaa gattatagat tggaccaact 1560

cggacttgca gaaagatata tacatgctag ttacttattt ggtgttactg ttatccccga 1620

gcctgaatta tccgatgcta gactcatgta cgcgaaatac gtcatgctcc tgactattgt 1680

cgatgatcat ttcgagagtt ttgcatctaa agatgaatgt ttcaacatca ttgaattagt 1740

agaaaggtgg gatgactatg caagtgtagg ttataaatct gagaaggtta aagttttttt 1800

ttctgttttc tataaatcaa tagaggagct tgcaacaatt gctgaaatta aacaaggaag 1860

atccgtcaaa aatcacctta ttaatttgtg gcttgaattg atgaagttga tgttgatgga 1920

gagagtagag tggtgttctg gcaagacaat accaagcata gaagagtact tgtatgttac 1980

atctataaca ttttgtgcaa aattgattcc tctctcaaca caatattttc ttggaataaa 2040

aatatccaaa gatctactag aaagtgatga aatatgtggc ctatggaatt gtagcggtag 2100

agtgatgaga atccttaatg atttacaaga ttccaagaga gaacaaaagg aggtctcaat 2160

aaatttagtc acattactaa tgaaaagtat gtctgaggaa gaagctataa tgaagataaa 2220

ggaaatcttg gaaatgaata gaagagagtt attgaaaatg gttttagttc aaaaaaaggg 2280

aagccaattg cctcaattat gcaaagatat attttggagg acaagcaaat gggctcattt 2340

cacttattca caaactgatg gatatagaat tgcagaggaa atgaagaatc acattgatga 2400

agtcttttac aaaccactca atcattaata atagcataac cccttggggc ctctaaacgg 2460

gtcttgaggg gttttttggg gccctcgact ttttaacaaa atatattgat aaaaataata 2520

atagtgggta taattaagtt gttagagaaa acgtataaat tagggataaa ctatggaact 2580

tatgaaatag attgaaatgg tttatctgtt accccgtagg atccagaatt taaaaggagg 2640

gattaaaatg tctgctcgtg gactcaacaa gatttcatgc tcactcaact tacaaaccga 2700

aaagctttgt tatgaggata atgataatga tcttgatgaa gaacttatgc ctaaacacat 2760

tgctttgata atggatggta ataggagatg ggcaaaggat aagggtttag aagtatatga 2820

aggtcacaaa catattattc caaaattaaa agagatttgt gacatttctt ctaaattggg 2880

aatacaaatt atcactgctt ttgcattctc tactgaaaat tggaaaagat ccaaggagga 2940

ggttgatttc ttgttgcaaa tgttcgaaga aatctatgat gagttttcga ggtctggagt 3000

aagagtgtct attataggtt gtaaatccga cctcccaatg acattacaaa aatgcatagc 3060

attaacagaa gagactacaa agggcaacaa aggacttcac cttgtgattg cactaaacta 3120

tggtggatat tatgacatat tgcaagcaac aaaaagcatt gttaataaag caatgaatgg 3180

tttattagat gtagaagata tcaacaagaa tttatttgat caagaacttg aaagcaagtg 3240

tccaaatcct gatttactta taaggacagg aggtgaacaa agagttagta actttttgtt 3300

gtggcaattg gcttacactg aattttactt caccaacaca ttgtttcctg attttggaga 3360

ggaagatctt aaagaggcaa taatgaactt tcaacaaagg catagacgtt ttggtggaca 3420

cacatattaa taataataat taattcgaac agaaaaaata agtatttata taacggttaa 3480

ttgtaaggag ggttttttat gcaaactgaa catgttattt tattgaatgc acagggagtt 3540

cctactggta ctctggaaaa gtatgccgca catacagcag acacccgctt acatctcgct 3600

ttctccagtt ggctgtttaa tgccaaagga caattattag ttaccagaag agcactgagc 3660

aaaaaagcat ggcctggcgt gtggactaac tctgtttgtg ggcatccaca actgggagaa 3720

agcaacgaag acgcagtgat cagaagatgt cgttatgagc ttggcgtgga aattactcct 3780

cctgaatcta tctatcctga ctttagatac agagccaccg atcctagtgg cattgtggaa 3840

aatgaagtgt gtcctgtatt tgccgcaaga accactagtg cattacagat caatgatgat 3900

gaagtgatgg attatcaatg gtgtgattta gcagatgtat tacatggtat tgatgccact 3960

ccttgggctt tcagtccttg gatggtgatg caggcaacaa atagagaagc cagaaaaaga 4020

ttatctgcat ttacccagct taaataaaaa taagagttac cttaaatggt aactcttatt 4080

tttttaatgt cctcgagcga tcgcccttcc caacagttgc gcagcctgaa tggcgaatgg 4140

cgcctgatgc ggtattttct ccttacgcat ctgtgcggta tttcacaccg catatggtgc 4200

actctcagta caatctgctc tgatgccgca tagttaagcc agccccgaca cccgccaaca 4260

cccgctgacg cgccctgacg ggcttgtctg ctcccggcat ccgcttacag acaagctgtg 4320

accgtctccg ggagctgcat gtgtcagagg ttttcaccgt catcaccgaa acgcgcgaga 4380

cgaaagggcc tcgtgatacg cctattttta taggttaatg tcatgataat aatggtttct 4440

tagacgtcag gtggcacttt tcggggaaat gtgcgcggaa cccctatttg tttatttttc 4500

taaatacatt caaatatgta tccgctcatg agacaataac cctgataaat gcttcaataa 4560

tattgaaaaa ggaagagtat gagtattcaa catttccgtg tcgcccttat tccctttttt 4620

gcggcatttt gccttcctgt ttttgctcac ccagaaacgc tggtgaaagt aaaagatgct 4680

gaagatcagt tgggtgcacg agtgggttac atcgaactgg atctcaacag cggtaagatc 4740

cttgagagtt ttcgccccga agaacgtttt ccaatgatga gcacttttaa attaaaaatg 4800

aagttttaaa acttcatttt taatttaaat taaaaatgaa gttttatcaa aaaaatttcc 4860

aataatccca ctctaagcca caaacacgcc ctataaaatc ccgctttaat cccactttga 4920

gacacatgta atattacttt acgccctagt atagtgataa ttttttacat tcaatgccac 4980

gcaaaaaaat aaaggggcac tataataaaa gttccttcgg aactaactaa agtaaaaaat 5040

tatctttaca acctccccaa aaaaaagaac aggtacaaag taccctataa tacaagcgta 5100

aaaaaatgag ggtaaaaata aaaaaataaa aaaataaaaa aataaaaaaa taaaaaaaat 5160

aaaaaaataa aaaaataaaa aaataaaaaa ataaaaaaat aaaaaaataa aaaaataaaa 5220

aaatataaaa ataaaaaaat ataaaaataa aaaaatataa aaataaaaaa atataaaaat 5280

aaaaaaataa aaaaatataa aaataaaaaa ataaaaaaat ataaaaatat tttttattta 5340

aagtttgaaa aaaatttttt tatattatat aatctttgaa gaaaagaata taaaaaatga 5400

gcctttataa aagcccattt tttttcatat acgtaatatg acgttctaat gtttttattg 5460

gtacttctaa cattagagta atttctttat ttttaaagcc tttttcttta agggctttta 5520

ttttttttct taatacattt aattcctctt tttttgttgc ttttccttta gcttttaatt 5580

gctcttgata atttttttta cctctaatat tttctcttct cttatattcc tttttagaaa 5640

ttattattgt catatatttt tgttcttctt ctgtaatttc taataactct ataagagttt 5700

cattcttata cttatattgc ttatttttat ctaaataaca tctttcagca cttctagttg 5760

ctcttataac ttctctttca cttaaatgtt gtctaaacat actattaagt tctaaaacat 5820

catttaatgc cttctcaatg tcttctgtaa agctacaaag ataatatcta tataaaaata 5880

atataagctc tctgtgtcct tttaaatcat attctcttag ttcacaaagt tttattatgt 5940

cttgtattct tccataatat aaacttcttt ctctataaat ataatttatt ttgcttggtc 6000

tacccttttt cctttcatat ggttttaatt caggtaaaaa tccattttgt atttctctta 6060

agtcataaat atattcgtac tcatctaata tattgactac tgtttttgat ttagagttta 6120

tacttcctgg aactcttaat attctggttg catctaaggc ttgtctatct gctccaaagt 6180

attttaattg attatataaa tattcttgaa ccgctttcca taatggtaat gctttactag 6240

gtactgcatt tattatccat attaaataca ttcctcttcc actatctatt acatagtttg 6300

gtataggaat actttgatta aaataattct tttctaagtc cattaatacc tggtctttag 6360
ttttgccagt tttataataa tccaagtcta taaacagtgt atttaactct tttatatttt 6420
ctaatcgcct acacggctta taaaaggtat ttagagttat atagatattt tcatcactca 6480
tatctaaatc ttttaattca gcgtatttat agtgccattg gctatatcct tttttatcta 6540
taacgctcct ggttatccac cctttacttc tactatgaat attatctata tagttctttt 6600
tattcagctt taatgcgttt ctcacttatt cacctcccct tctgtaaaac taagaaaatt 6660
atatcatatt ttcaataatt attaactatt cttaaactct taataaaaaa tagagtaagt 6720
ccccaattga aacttaatct attttttatg ttttaattta ttatttttat taaaatattt 6780
taaactaaat taaatgattc tttttaattt tttactattt cattccataa tatattacta 6840
taattattta caaataatat ttcttcattt gtaatattta gatgatttac taattttagt 6900
ttttatatat taaataatta atgtataatt tatataaaaa atcaaaggag cttataaatt 6960
atgattattt ccaaagatac taaagattta attttttcaa ttttaacaat actttttgta 7020
atattatgtt taaatttaat tgtatttttt tcatataata aagccgttga agtaaaccaa 7080
tccattttcc ttatgatgtt attattaaat ttaagtttta taataatatc tttattatat 7140
ttattgtttt taaaaaaact agtgaaattt ccggctttat taaacttatt tttaggaatt 7200
ttattttcat tttcatcttt acaggatttg attatatctt taaatatgtt ttatcaaata 7260
ttatcttttt ctaaatttat atatattttt attatattta ttattatata tattttattt 7320
ttaagtttct ttctaacagc tattaaaaag aaacttaaaa ataaaaacac gtactctaaa 7380
ccaataaata aaactatttt tattattgct gccttgattg gaatagtttt tagtaaaatt 7440
aatttcaata ttccacaata ttatattata agctagcttt gcattgtact tttcaatcgc 7500
ttcacgaatg cggttatctc cgaaagataa agtcttttca tcttccttga tgaagataag 7560
attttctccg tctccgccgg cagaattgaa gcggggtact acggtatcgt ctgcgtcatc 7620
ttccgttgtc tgatagatga tagtcatagg ctcattttct tccgtttcgg taaaggggat 7680
aggttcgccc tttgagagca gggcggcgat ggaaagcatt aacttgcttt tcccatcgcc 7740
cggatctccc tgcaatagcg taactttgcc aaacggaata tacggatacc acagccactt 7800
tacttctttc ggctcgattt cacttgcctt gatgatttca agaggtacgc tgaaattcat 7860
ttcgttttca tttagtttca ttttttcttg ttctcctttt ctctgaaaat ataaaaacca 7920
cagattgata ctaaaacctt ggttgtgttg cttttcgggg cttaaatcaa ggaaaaatcc 7980
ttgttttaag cctttcaaaa agaaacacaa ggtctttgta ctaacctgtg gttatgtata 8040
aaattgtaga ttttagggta acaaaaaaca ccgtatttct acgatgtttt tgcttaaata 8100
cttgttttta gttacagaca aacctgaagt tatcatagtc ctaaattata ttatagattt 8160

ccctttaaat accccatata tacatgcatt taaaaaactt tctattatgc tgcttaatat 8220
aaaaattaaa ccctgcattt acataatgct agggttcaat ttttcattta tatttgcttt 8280
taaattataa aagccagtca ttaggcctat ctgacaattc ctgaatagag ttcataaaca 8340
atcctgcatg ataaccatca caaacagaat gatgtacctg taaagatagc ggtaaatata 8400
ttgaattacc tttattaatg aattttcctg ctgtaataat gggtagaagg taattactat 8460
tattattgat atttaagtta aacccagtaa atgaagtcca tggaataata gaaagagaaa 8520
aagcattttc aggtataggt gttttgggaa acaatttccc cgaaccatta tatttctcta 8580
catcagaaag gtataaatca taaaactctt tgaagtcatt ctttacagga gtccaaatac 8640
cagagaatgt tttagataca ccatcaaaaa ttgtataaag tggctctaac ttatcccaat 8700
aacctaactc tccgtcgcta ttgtaaccag ttctaaaagc tgtatttgag tttatcaccc 8760
ttgtcactaa gaaaataaat gcagggtaaa atttatatcc ttcttgtttt atgtttctgt 8820
ataaaacact aatatcaatt tctgtggtta tactaaaagt cgtttgttgg ttcaaataat 8880
gattaaatat ctcttttctc ttccaattgt ctaaatcaat tttattaaag ttcatgttca 8940
tttcctccct ttaaatttaa cacaaaatta cacacactta tactataatc ctttttagtt 9000
gtatttttca ataaaaatca ttcaaaaata taacttttga taagaaattt cacaaattaa 9060
agtatcaaaa aattttgcta gtcaatactt tactcaatat tatataatgt aaatcaaata 9120
agcaaaaatt taatctgaag atgcttagtg ggaatttgta ccccttatcg atacaaattc 9180
cccgtaggcg ctagggacac tttttcactc gttaaaaagt tttgagaata ttttatattt 9240
ttgttcatgt aatcactcct tcttaattac aaatttttag catctaattt aacttcaatt 9300
cctattatac aaaattttaa gatactgcac tatcaacaca ctcttaagtt tgcttctaag 9360
tcttatttcc ataacttctt ttacgtttcc gggtacaatt cgtaatcatg tcatagctgt 9420
ttcctgtgtg aaattcttat ccgctcacaa ttccacacaa catacgagcc ggaagcataa 9480
agtgtaaagc ctggggtgcc taatgagtga gctaactcac attaattgcg ttgcgctcac 9540
tgcccgcttt ccagtcggga aacctgtcgt gccagaaaac ttcattttta atttaaaagg 9600
atctaggtga agatcctttt tgataatctc atgaccaaaa tcccttaacg tgagttttcg 9660
ttccactgag cgtcagaccc cgtagaaaag atcaaaggat cttcttgaga tccttttttt 9720
ctgcgcgtaa tctgctgctt gcaaacaaaa aaaccaccgc taccagcggt ggtttgtttg 9780
ccggatcaag agctaccaac tctttttccg aaggtaactg gcttcagcag agcgcagata 9840
ccaaatactg tccttctagt gtagccgtag ttaggccacc acttcaagaa ctctgtagca 9900
ccgcctacat acctcgctct gctaatcctg ttaccagtgg ctgctgccag tggcgataag 9960
tcgtgtctta ccgggttgga ctcaagacga tagttaccgg ataaggcgca gcggtcgggc 10020
tgaacggggg gttcgtgcac acagcccagc ttggagcgaa cgacctacac cgaactgaga 10080

tacctacagc gtgagctatg agaaagcgcc acgcttcccg aagggagaaa ggcggacagg 10140

tatccggtaa gcggcagggt cggaacagga gagcgcacga gggagcttcc agggggaaac 10200

gcctggtatc tttatagtcc tgtcgggttt cgccacctct gacttgagcg tcgatttttg 10260

tgatgctcgt caggggggcg gagcctatgg aaaaacgcca gcaacgcggc ctttttacgg 10320

ttcctggcct tttgctggcc ttttgctcac atgttctttc ctgcgttatc ccctgattct 10380

gtggataacc gtattaccgc ctttgagtga gctgataccg ctcgccgcag ccgaacgacc 10440

gagcgcagcg agtcagtgag cgaggaagcg gaagagcgcc caatacgcaa accgcctctc 10500

cccgcgcgtt ggccgattca ttaatgcagc tggcacgaca ggtttcccga ctggaaagcg 10560

ggcagtgagc gcaacgcaat taatgtgagt tagctcactc attaggcacc ccaggcttta 10620

cactttatgc ttccggctcg tatgttgtgt ggaattgtga gcggataaca atttcacaca 10680

ggaaacagct atgaccatga ttac 10704

<210> 13
<211> 18188
<212> DNA
<213> Artificial

<220>
<223> Gene cluster

<400> 13
gaattcgagc tcggtacccg gggatcctct agagtcgacc tgcaggcatg ccctaggtcg 60

actttttaac aaaatatatt gataaaaata ataatagtgg gtataattaa gttgttagag 120

aaaacgtata aattagggat aaactatgga acttatgaaa tagattgaaa tggtttatct 180

gttaccccgt aggatccaga atttaaaagg agggattaaa catatgaatg gtttcgaaga 240

tgcaagggat agaataaggg aaagttttgg gaaattagag ttatctcctt cttcctatga 300

cacagcatgg gtagctatgg tcccttcaag acattcacta aatgagccat gttttccaca 360

atgtttggat tggattattg aaaatcaaag agaagatgga tcttggggac taaaccctac 420

ccatccattg cttctaaagg actcactttc ttccactctt gcatgtttgc ttgcactaac 480

caaatggaga gttggagatg agcaaatcaa aagaggtctt ggcttcattg aaacgtatgg 540

ttgggcagta gataacaagg atcaaatttc acctttagga tttgaagtta tattttctag 600

tatgatcaaa tctgcagaga aattagattt aaatttgcct ttgaatcttc atcttgtaaa 660

tttggtgaaa tgcaaaagag attcaacaat taaaaggaat gttgaatata tgggtgaagg 720

agttggtgaa ttatgtgatt ggaaggaaat gataaagtta catcaaagac aaaatggttc 780

attatttgat tcaccagcca ctactgcagc tgccttgatt tatcatcaac atgatcaaaa 840

atgctatcaa tatcttaatt caatcttcca acaacacaaa aattgggttc ccactatgta 900

tccaacaaag gtacattcat tgctttgctt ggttgataca cttcaaaatc ttggagtaca 960
taggcatttt aaatcagaaa taaagaaagc tctagatgaa atatacaggc tatggcaaca 1020
aaagaatgaa caaattttct caaatgtcac ccattgtgct atggctttta gacttctaag 1080
gatgagctac tatgatgtct cctcagatga actagcagaa tttgtggatg aagaacattt 1140
ctttgcaaca aatgggaaat ataaaagtca tgttgaaatt cttgaactcc acaaagcatc 1200
acaattggct attgatcatg agaaagatga cattttggat aaaataaaca attggacaag 1260
agcttttatg gagcaaaaac tcttaaacaa tggcttcata gataggatgt caaagaaaga 1320
ggtggaactt gctttgagga agttttatac cacatctcat ctagcagaaa atagaagata 1380
tataaagtca tacgaagaga acaattttaa aatcttaaaa gcagcttata ggtcacccaa 1440
cattaacaat aaggacttgt tagcattttc aatacacgac tttgaattat gccaagctca 1500
acacagagaa gaacttcaac aactcaagag gtggtttgaa gattatagat tggaccaact 1560
cggacttgca gaaagatata tacatgctag ttacttattt ggtgttactg ttatccccga 1620
gcctgaatta tccgatgcta gactcatgta cgcgaaatac gtcatgctcc tgactattgt 1680
cgatgatcat ttcgagagtt ttgcatctaa agatgaatgt ttcaacatca ttgaattagt 1740
agaaaggtgg gatgactatg caagtgtagg ttataaatct gagaaggtta aagttttttt 1800
ttctgttttc tataaatcaa tagaggagct tgcaacaatt gctgaaatta aacaaggaag 1860
atccgtcaaa aatcacctta ttaatttgtg gcttgaattg atgaagttga tgttgatgga 1920
gagagtagag tggtgttctg gcaagacaat accaagcata gaagagtact tgtatgttac 1980
atctataaca ttttgtgcaa aattgattcc tctctcaaca caatattttc ttggaataaa 2040
aatatccaaa gatctactag aaagtgatga aatatgtggc ctatggaatt gtagcggtag 2100
agtgatgaga atccttaatg atttacaaga ttccaagaga gaacaaaagg aggtctcaat 2160
aaatttagtc acattactaa tgaaaagtat gtctgaggaa gaagctataa tgaagataaa 2220
ggaaatcttg gaaatgaata gaagagagtt attgaaaatg gttttagttc aaaaaaaggg 2280
aagccaattg cctcaattat gcaaagatat attttggagg acaagcaaat gggctcattt 2340
cacttattca caaactgatg gatatagaat tgcagaggaa atgaagaatc acattgatga 2400
agtcttttac aaaccactca atcattaata atagcataac cccttggggc ctctaaacgg 2460
gtcttgaggg gttttttgtc gactttttaa caaaatatat tgataaaaat aataatagtg 2520
ggtataatta agttgttaga gaaaacgtat aaattaggga taaactatgg aacttatgaa 2580
atagattgaa atggtttatc tgttaccccg taggatccag aatttaaaag gagggattaa 2640
aatgtctgct cgtggactca acaagatttc atgctcactc aacttacaaa ccgaaaagct 2700
ttgttatgag gataatgata atgatcttga tgaagaactt atgcctaaac acattgcttt 2760
gataatggat ggtaatagga gatgggcaaa ggataagggt ttagaagtat atgaaggtca 2820

caaacatatt attccaaaat taaaagagat ttgtgacatt tcttctaaat tgggaataca 2880

aattatcact gcttttgcat tctctactga aaattggaaa agatccaagg aggaggttga 2940

tttcttgttg caaatgttcg aagaaatcta tgatgagttt tcgaggtctg gagtaagagt 3000

gtctattata ggttgtaaat ccgacctccc aatgacatta caaaaatgca tagcattaac 3060

agaagagact acaaagggca acaaaggact tcaccttgtg attgcactaa actatggtgg 3120

atattatgac atattgcaag caacaaaaag cattgttaat aaagcaatga atggtttatt 3180

agatgtagaa gatatcaaca agaatttatt tgatcaagaa cttgaaagca agtgtccaaa 3240

tcctgattta cttataagga caggaggtga acaaagagtt agtaactttt tgttgtggca 3300

attggcttac actgaatttt acttcaccaa cacattgttt cctgattttg gagaggaaga 3360

tcttaaagag gcaataatga actttcaaca aaggcataga cgttttggtg gacacacata 3420

ttaataataa taattaattc gaacagaaaa aataagtatt tatataacgg ttaattgtaa 3480

ggagggtttt ttatgcaaac tgaacatgtt attttattga atgcacaggg agttcctact 3540

ggtactctgg aaaagtatgc cgcacataca gcagacaccc gcttacatct cgctttctcc 3600

agttggctgt ttaatgccaa aggacaatta ttagttacca gaagagcact gagcaaaaaa 3660

gcatggcctg gcgtgtggac taactctgtt tgtgggcatc cacaactggg agaaagcaac 3720

gaagacgcag tgatcagaag atgtcgttat gagcttggcg tggaaattac tcctcctgaa 3780

tctatctatc ctgactttag atacagagcc accgatccta gtggcattgt ggaaaatgaa 3840

gtgtgtcctg tatttgccgc aagaaccact agtgcattac agatcaatga tgatgaagtg 3900

atggattatc aatggtgtga tttagcagat gtattacatg gtattgatgc cactccttgg 3960

gctttcagtc cttggatggt gatgcaggca acaaatagag aagccagaaa aagattatct 4020

gcatttaccc agcttaaata attttaaaat ataagtgatt tagatattca taatatattt 4080

gggaggtaaa ttaatatgaa agaagttgta atagctagtg cagtaagaac agcgattgga 4140

tcttatggaa agtctcttaa ggatgtacca gcagtagatt taggagctac agctataaag 4200

gaagcagtta aaaaagcagg aataaaacca gaggatgtta atgaagtcat tttaggaaat 4260

gttcttcaag caggtttagg acagaatcca gcaagacagg catcttttaa agcaggatta 4320

ccagttgaaa ttccagctat gactattaat aaggtttgtg gttcaggact tagaacagtt 4380

agcttagcag cacaaattat aaaagcagga gatgctgacg taataatagc aggtggtatg 4440

gaaaatatgt ctagagctcc ttacttagcg aataacgcta gatggggata tagaatggga 4500

aacgctaaat ttgttgatga aatgatcact gacggattgt gggatgcatt taatgattac 4560

cacatgggaa taacagcaga aaacatagct gagagatgga acatttcaag agaagaacaa 4620

gatgagtttg ctcttgcatc acaaaaaaaa gctgaagaag ctataaaatc aggtcaattt 4680

aaagatgaaa tagttcctgt agtaattaaa ggcagaaagg gagaaactgt agttgataca 4740
gatgagcacc ctagatttgg atcaactata gaaggacttg caaaattaaa acctgccttc 4800
aaaaaagatg gaacagttac agctggtaat gcatcaggat taaatgactg tgcagcagta 4860
cttgtaatca tgagtgcaga aaaagctaaa gagcttggag taaaaccact tgctaagata 4920
gtttcttatg gttcagcagg agttgaccca gcaataatgg gatatggacc tttctatgca 4980
acaaaagcag ctattgaaaa agcaggttgg acagttgatg aattagattt aatagaatca 5040
aatgaagctt ttgcagctca aagtttagca gtagcaaaag atttaaaatt tgatatgaat 5100
aaagtaaatg taaatggagg agctattgcc cttggtcatc caattggagc atcaggtgca 5160
agaatactcg ttactcttgt acacgcaatg caaaaaagag atgcaaaaaa aggcttagca 5220
actttatgta taggtggcgg acaaggaaca gcaatattgc tagaaaagtg ctagtagaaa 5280
taagagttac cttaaatggt aactcttatt tttttaatgt cacatagaga atttcactct 5340
ttgcatttta tctaacatca aggggtttat ttgtcacaaa ttatgtaaaa ataaaacaaa 5400
gatgtaagaa agtcctatga tataaatttt gtaaacataa taaattagct ttcataagat 5460
tggaagaatg ataattacta cttagaactg ctaaaaatta ggaaagaggt gtcgttaatt 5520
aatgcagaaa agacaaaggg agctgagtgc gttgacacta cctacctctg ctgagggggt 5580
atcagaaagc catagggccc gttctgtcgg catcggtcgt gcccatgcca aggccatcct 5640
gctgggagag catgcggtag tatacggagc gccggcactc gctctgccta ttcctcagct 5700
cacggtcacg gccagcgttg gctggtcttc cgaggcctcc gacagtgcgg gtggcctgtc 5760
ctacacgatg accggtacgc cttctagggc actggtgacg caggcctccg acggcctgca 5820
taggctcacc gcggaattca tggcgaggat gggcgtgacg aacgcgcctc atctcgacgt 5880
gatcctggac ggcgcgatcc ctcacggcag gggtctcggc tccagcgcgg ccggctcacg 5940
tgcgatcgcc ttggccctcg ccgacctctt cggccacgaa ctggccgagc atacggcgta 6000
cgaactggtg cagacggccg agaacatggc gcatggcagg gccagcggcg tggacgcgat 6060
gacggtcggc gcgtccaggc ctctgctgtt ccagcagggc cgtaccgaga gactggccat 6120
cggctgcgac agcctgttca tcgtagccga cagcggcgta cctggcagca ccaaggaagc 6180
ggtagagatg ctgagggagg gattcacccg tagcgccgga acacaggagc ggttcgttgg 6240
cagggcgacg gaactgaccg aggccgccag gcaggccctc gccgacggca ggcccgagga 6300
gctgggctct cagctgacgt actaccatga gctgctccat gaggcccgtc tgagcaccga 6360
cggcatcgat gcgctggtag aggccgcgct gaaggcaggc agcctcggag ccaagatcac 6420
cggcggtggt ctgggcggct gcatgatcgc acaggccagg cccgaacagg ccagggaggt 6480
aaccaggcag ctccatgagg ccggtgccgt acagacctgg gtagtaccac tgaaagggct 6540
cgacaaccat gcgcagtaat aattttaaaa tataagtgat ttagatattc ataatatatt 6600

tgggaggtaa attaatatgc gtagtgaaca tcctaccacg accgtgctcc agtctaggga 6660

gcagggcagc gcggccggcg ccaccgcggt agcgcatcca aacatcgcgc tgatcaagta 6720

ctggggcaag cgtgacgaga ggctgatcct gccctgcacc accagcctgt ctatgacgct 6780

ggacgtattc cccacgacca ccgaggtcag gctcgacccc gccgccgagc atgacacggc 6840

cgccctcaac ggcgaggtgg ccacgggcga gacgctgcgt cgtatcagcg ccttcctctc 6900

cctggtgagg gaggtggcgg gcagcgacca gagggccgtg gtggacaccc gtaacaccgt 6960

gcccaccggg gcgggcctgg cgtcctccgc cagcgggttc gccgccctcg ccgtcgcggc 7020

cgcggccgcc tacgggctcg aactcgacga ccgtgggctg tccaggctgg ccagacgtgg 7080

atccggctcc gcctctcggt ctatcttcgg cggcttcgcc gtatggcatg ccggccccga 7140

cggcacggcc acggaagcgg acctcggctc ctacgccgag ccagtgcccg cggccgacct 7200

cgaccctgcg ctggttatcg ccgtggtaaa cgccggcccc aagcccgtat ccagccgtga 7260

ggccatgcgt cgcaccgtag acacctcacc actgtacagg ccatgggccg actccagtaa 7320

ggacgacctg gacgagatgc gttctgcgct gctgcgtggc gacctcgagg ccgtgggcga 7380

gatcgcggag cgtaacgcgc tcggcatgca tgccaccatg ctggccgccc gtcccgcggt 7440

gaggtacctg tcaccagcca cggtaaccgt gctcgacagc gtgctccagc tccgtaagga 7500

cggtgttctg gcctacgcga ccatggacgc cggtcccaac gtgaaggtgc tgtgcaggag 7560

ggcggacgcc gagagggtgg ccgacgttgt acgcgccgcc gcgtccggcg gtcaggtact 7620

cgtagccggg cctggagacg gtgcccgtct gctgagcgag ggcgcataat aattttaaaa 7680

tataagtgat ttagatattc ataatatatt tgggaggtaa attaatatga cgacaggtca 7740

gcgtacgatc gtcaggcatg cgcctggcaa gctgttcgta gcgggcgagt acgcggtagt 7800

ggatcctggc aaccctgcga tcctggtagc ggtagacagg catatcagcg taaccgtgtc 7860

cgacgccgac gcggacaccg gggccgccga cgtagtgatc tcctccgacc tcggtcctca 7920

ggcggtaggc tggcgttggc atgacggcag gctcgtagta cgtgaccctg acgacgggca 7980

gcaggcgcgt agcgccctgg cccatgtggt gtcggcgatc gagaccgtgg gcaggctgct 8040

gggcgaacgt ggacagaagg tacccgctct caccctctcc gttagcagcc gtctgcatga 8100

ggacggcagg aagttcggcc tgggctccag cggcgcggtg accgtggcga ccgtagccgc 8160

cgtagccgcg ttctgcggac tcgaactgtc caccgacgaa aggttcaggc tggccatgct 8220

cgccaccgcg gaactcgacc ccaagggctc cggcggggac ctcgccgcca gcacctgggg 8280

cggctggatc gcctaccagg cgcccgacag ggcctttgtg ctcgacctgg ccaggcgtgt 8340

gggagtagac aggacactga aggcgccctg gccggggcat tctgtgcgta gactgcctgc 8400

gcccaagggc ctcaccctgg aggtcggctg gaccggagag cccgcctcca ccgcgtccct 8460

ggtgtccgat ctgcatcgtc gtacctggag gggcagcgcc tcccatcaga ggttcgtaga 8520

gaccacgacc gactgtgtac gttccgcggt taccgccctg gagtccggcg acgacacgag 8580

cctgctgcat gagatccgca gggcccgtca ggagctggcc cgtctggacg acgaggtagg 8640

cctcggcatc ttcacaccca agctgacggc gctgtgcgac gccgccgaag ccgttggcgg 8700

cgcggccaag ccctccgggg caggcggcgg cgactgcggc atcgccctgc tggacgccga 8760

ggcgtctagg gacatcacac atgtaaggca acggtgggag acagccgggg tgctgcccct 8820

gcccctgact cctgccctgg aagggatcta ataattttaa aatataagtg atttagatat 8880

tcataatata tttgggaggt aaattaatat gagcctcgat tccagactgc ccgctttccg 8940

taacctgtcc cctgccgcga gactggacca catcggccag ttgctcggcc tgagccacga 9000

cgatgtcagc ctgctggcca acgccggtgc cctgccgatg gacatcgcca acggcatgat 9060

cgaaaacgtc atcggcacct tcgagctgcc ctatgccgtg gccagcaact tccagatcaa 9120

tggccgtgat gtgctggtgc cgctggtggt ggaagagccc tcgatcgtcg ccgctgcttc 9180

ttacatggcc aagctggccc gtgccaacgg cggcttcacc acctccagca gcgccccgct 9240

gatgcatgcc caggtacaga tcgtcggcat acaggacccg ctcaatgcac gtctgagcct 9300

gctgagaaga aaagacgaaa tcattgaact ggccaaccgt aaggaccagt tgctcaacag 9360

cctcggcggc ggctgcagag acatcgaagt gcacaccttc gccgataccc cgcgtggccc 9420

gatgctggtg gcgcacctga tcgtcgatgt aagagatgcc atgggcgcca acaccgtcaa 9480

taccatggcc gaggccgttg cgccgctgat ggaagccatc accggggccc aggtacgtct 9540

gagaattctg tccaacctgg ccgacctgcg cctggccagg gcccaggtga ggattactcc 9600

gcagcaactg gaaacggccg aattcagtgg cgaggcagtg atcgaaggca tcctcgacgc 9660

ctacgccttc gctgcggtcg acccttacag agcggccacc cacaacaagg gcatcatgaa 9720

tggcatcgac ccactgatcg tcgccactgg caacgactgg cgtgcagtgg aagccggcgc 9780

ccatgcgtat gcctgcagaa gtggtcacta cggctcgctg accacctggg aaaaggacaa 9840

caacggccat ttggtcggca ccctggaaat gccgatgccc gtaggcctgg tcggcggcgc 9900

caccaaaacc catccgctgg cgcaactgtc actgagaatc ctcggcgtga aaacagccca 9960

ggcgctcgct gagattgccg tggccgtagg cctggcgcaa aacctcgggg ccatgagagc 10020

cctggccacc gaaggcatcc agcgtggcca catggccctg catgcgagaa atattgccgt 10080

ggtggcgggc gcccgaggcg atgaggtgga ctgggttgcc cggcagttgg tggaatacca 10140

cgacgtgaga gccgacagag ccgtagcact gctgaaacaa aagagaggcc aatgatagtt 10200

ttaaaatata agtgatttag atattcataa tatatttggg aggtaaatta atatgtccat 10260

ctccataggc attcacgacc tgtctttcgc cacaaccgag ttcgtactgc ctcatacggc 10320

gctcgccgag tacaacggca ccgagatcgg caagtaccat gtaggcatcg gccagcagtc 10380

tatgagcgtg cctgccgccg acgaggacat cgtgaccatg gccgcgaccg cggcgaggcc 10440

catcatcgag cgtaacggca agagcaggat ccgtacggta gtgttcgcca cggagtcttc 10500

tatcgaccag gcgaaggcgg gcggcgtata cgtgcactcc ctgctggggc tggagtctgc 10560

ctgcagggta gtagagctga agcaggcctg ctacggggcc accgccgccc ttcagttcgc 10620

catcggcctg gtgaggcgtg accccgccca gcaggtactg gtaatcgcca gtgacgtatc 10680

caagtacgag ctggacagcc ccggcgaggc gacccagggc gcggccgcgg tggccatgct 10740

ggtaggcgcc gaccctgccc tgctgcgtat cgaggagcct tcgggcctgt tcaccgccga 10800

cgtaatggac ttctggcggc ccaactacct caccaccgct ctggtagacg gccaggagtc 10860

catcaacgcc tacctgcagg ccgtagaggg cgcctggaag gactacgcgg agcaggacgg 10920

caggtcactg gaggagttcg cggcgttcgt ataccaccag ccgttcacga agatggccta 10980

caaggcgcat cgccatctgc tgaacttcaa cggctacgac accgacaagg acgccatcga 11040

gggcgccctc ggccagacga cggcgtacaa caacgtaatc ggcaacagct acaccgcgtc 11100

tgtgtacctg ggcctggccg ccctgctcga ccaggcggac gacctgacgg gccgttccat 11160

cggcttcctg agctacggct ctggcagcgt agccgagttc ttctctggca ccgtagtagc 11220

cgggtaccgt gagcgtctgc gtaccgaggc gaaccaggag gcgatcgcca ggcgtaagag 11280

cgtagactac gccacctacc gtgagctgca cgagtacacg ctcccgtccg acggcggcga 11340

ccatgccacc cctgtgcaga ccaccggccc cttcaggctg gccgggatca acgaccataa 11400

gcgtatctac gaggcgcgtt aataatttaa aagcaaatat aaatgaaaaa ttgaacccta 11460

gcattatgta aatgcagggt ttaattttta tattaagcag cataatagaa agttttttaa 11520

atgcatgtat atatggggta tttaaaggga aatctataat ataatttagg actatacgcg 11580

tcgatcgccc ttcccaacag ttgcgcagcc tgaatggcga atggcgcctg atgcggtatt 11640

ttctccttac gcatctgtgc ggtatttcac accgcatatg gtgcactctc agtacaatct 11700

gctctgatgc cgcatagtta agccagcccc gacacccgcc aacacccgct gacgcgccct 11760

gacgggcttg tctgctcccg gcatccgctt acagacaagc tgtgaccgtc tccgggagct 11820

gcatgtgtca gaggttttca ccgtcatcac cgaaacgcgc gagacgaaag ggcctcgtga 11880

tacgcctatt tttataggtt aatgtcatga taataatggt ttcttagacg tcaggtggca 11940

cttttcgggg aaatgtgcgc ggaaccccta tttgtttatt tttctaaata cattcaaata 12000

tgtatccgct catgagacaa taaccctgat aaatgcttca ataatattga aaaaggaaga 12060

gtatgagtat tcaacatttc cgtgtcgccc ttattccctt ttttgcggca ttttgccttc 12120

ctgtttttgc tcacccagaa acgctggtga aagtaaaaga tgctgaagat cagttgggtg 12180

cacgagtggg ttacatcgaa ctggatctca acagcggtaa gatccttgag agttttcgcc 12240

ccgaagaacg ttttccaatg atgagcactt ttaaattaaa aatgaagttt taaaacttca 12300

tttttaattt aaattaaaaa tgaagtttta tcaaaaaaat ttccaataat cccactctaa 12360

gccacaaaca cgccctataa aatcccgctt taatcccact ttgagacaca tgtaatatta 12420

ctttacgccc tagtatagtg ataatttttt acattcaatg ccacgcaaaa aaataaaggg 12480

gcactataat aaaagttcct tcggaactaa ctaaagtaaa aaattatctt tacaacctcc 12540

ccaaaaaaaa gaacaggtac aaagtaccct ataatacaag cgtaaaaaaa tgagggtaaa 12600

aataaaaaaa taaaaaaata aaaaaataaa aaaataaaaa aaataaaaaa ataaaaaaat 12660

aaaaaaataa aaaaataaaa aaataaaaaa ataaaaaaat aaaaaaatat aaaaataaaa 12720

aaatataaaa ataaaaaaat ataaaaataa aaaaatataa aaataaaaaa ataaaaaaat 12780

ataaaaataa aaaaataaaa aaatataaaa atatttttta tttaaagttt gaaaaaaatt 12840

tttttatatt atataatctt tgaagaaaag aatataaaaa atgagccttt ataaaagccc 12900

attttttttc atatacgtaa tatgacgttc taatgttttt attggtactt ctaacattag 12960

agtaatttct ttatttttaa agcctttttc tttaagggct tttatttttt ttcttaatac 13020

atttaattcc tctttttttg ttgcttttcc tttagctttt aattgctctt gataattttt 13080

tttacctcta atattttctc ttctcttata ttccttttta gaaattatta ttgtcatata 13140

tttttgttct tcttctgtaa tttctaataa ctctataaga gtttcattct tatacttata 13200

ttgcttattt ttatctaaat aacatctttc agcacttcta gttgctctta taacttctct 13260

ttcacttaaa tgttgtctaa acatactatt aagttctaaa acatcattta atgccttctc 13320

aatgtcttct gtaaagctac aaagataata tctatataaa aataatataa gctctctgtg 13380

tccttttaaa tcatattctc ttagttcaca aagttttatt atgtcttgta ttcttccata 13440

atataaactt ctttctctat aaatataatt tattttgctt ggtctaccct tttttccttc 13500

atatggtttt aattcaggta aaaatccatt ttgtatttct cttaagtcat aaatatattc 13560

gtactcatct aatatattga ctactgtttt tgatttagag tttatacttc ctggaactct 13620

taatattctg gttgcatcta aggcttgtct atctgctcca aagtatttta attgattata 13680

taaatattct tgaaccgctt tccataatgg taatgcttta ctaggtactg catttattat 13740

ccatattaaa tacattcctc ttccactatc tattacatag tttggtatag gaatactttg 13800

attaaaataa ttcttttcta agtccattaa tacctggtct ttagttttgc cagttttata 13860

ataatccaag tctataaaca gtgtatttaa ctcttttata ttttctaatc gcctacacgg 13920

cttataaaag gtatttagag ttatatagat attttcatca ctcatatcta aatcttttaa 13980

ttcagcgtat ttatagtgcc attggctata tcctttttta tctataacgc tcctggttat 14040

ccacccttta cttctactat gaatattatc tatatagttc tttttattca gctttaatgc 14100

gtttctcact tattcacctc cccttctgta aaactaagaa aattatatca tattttcaat 14160

aattattaac tattcttaaa ctcttaataa aaaatagagt aagtccccaa ttgaaactta 14220

atctattttt tatgttttaa tttattattt ttattaaaat attttaaact aaattaaatg 14280

attcttttta attttttact atttcattcc ataatatatt actataatta tttacaaata 14340

atatttcttc atttgtaata tttagatgat ttactaattt tagtttttat atattaaata 14400

attaatgtat aatttatata aaaaatcaaa ggagcttata aattatgatt atttccaaag 14460

atactaaaga tttaattttt tcaattttaa caatactttt tgtaatatta tgtttaaatt 14520

taattgtatt tttttcatat aataaagccg ttgaagtaaa ccaatccatt ttccttatga 14580

tgttattatt aaatttaagt tttataataa tatctttatt atatttattg tttttaaaaa 14640

aactagtgaa atttccggct ttattaaact tatttttagg aattttattt tcattttcat 14700

ctttacagga tttgattata tctttaaata tgttttatca aatattatct ttttctaaat 14760

ttatatatat ttttattata tttattatta tatatatttt atttttaagt ttctttctaa 14820

cagctattaa aaagaaactt aaaaataaaa acacgtactc taaaccaata aataaaacta 14880

tttttattat tgctgccttg attggaatag tttttagtaa aattaatttc aatattccac 14940

aatattatat tataagctag ctttgcattg tacttttcaa tcgcttcacg aatgcggtta 15000

tctccgaaag ataaagtctt ttcatcttcc ttgatgaaga taagattttc tccgtctccg 15060

ccggcagaat tgaagcgggg tactacggta tcgtctgcgt catcttccgt tgtctgatag 15120

atgatagtca taggctcatt ttcttccgtt tcggtaaagg ggataggttc gccctttgag 15180

agcagggcgg cgatggaaag cattaacttg cttttcccat cgcccggatc tccctgcaat 15240

agcgtaactt tgccaaacgg aatatacgga taccacagcc actttacttc tttcggctcg 15300

atttcacttg ccttgatgat ttcaagaggt acgctgaaat tcatttcgtt ttcatttagt 15360

ttcatttttt cttgttctcc ttttctctga aaatataaaa accacagatt gatactaaaa 15420

ccttggttgt gttgcttttc ggggcttaaa tcaaggaaaa atccttgttt taagcctttc 15480

aaaaagaaac acaaggtctt tgtactaacc tgtggttatg tataaaattg tagattttag 15540

ggtaacaaaa aacaccgtat ttctacgatg tttttgctta aatacttgtt tttagttaca 15600

gacaaacctg aagttatcat agtcctaaat tatattatag atttcccttt aaatacccca 15660

tatatacatg catttaaaaa actttctatt atgctgctta atataaaaat taaaccctgc 15720

atttacataa tgctagggtt caatttttca tttatatttg cttttaaatt ataaaagcca 15780

gtcattaggc ctatctgaca attcctgaat agagttcata aacaatcctg catgataacc 15840

atcacaaaca gaatgatgta cctgtaaaga tagcggtaaa tatattgaat tacctttatt 15900

aatgaatttt cctgctgtaa taatgggtag aaggtaatta ctattattat tgatatttaa 15960

gttaaaccca gtaaatgaag tccatggaat aatagaaaga gaaaaagcat tttcaggtat 16020

aggtgttttg ggaaacaatt tccccgaacc attatatttc tctacatcag aaaggtataa 16080

atcataaaac tctttgaagt cattctttac aggagtccaa ataccagaga atgttttaga 16140

tacaccatca aaaattgtat aaagtggctc taacttatcc caataaccta actctccgtc 16200

gctattgtaa ccagttctaa aagctgtatt tgagtttatc acccttgtca ctaagaaaat 16260

aaatgcaggg taaaatttat atccttcttg ttttatgttt ctgtataaaa cactaatatc 16320

aatttctgtg gttatactaa aagtcgtttg ttggttcaaa taatgattaa atatctcttt 16380

tctcttccaa ttgtctaaat caattttatt aaagttcatg ttcatttcct ccctttaaat 16440

ttaacacaaa attacacaca cttatactat aatccttttt agttgtattt ttcaataaaa 16500

atcattcaaa aatataactt ttgataagaa atttcacaaa ttaaagtatc aaaaaatttt 16560

gctagtcaat actttactca atattatata atgtaaatca aataagcaaa aatttaatct 16620

gaagatgctt agtgggaatt tgtacccctt atcgatacaa attccccgta ggcgctaggg 16680

acactttttc actcgttaaa aagttttgag aatattttat atttttgttc atgtaatcac 16740

tccttcttaa ttacaaattt ttagcatcta atttaacttc aattcctatt atacaaaatt 16800

ttaagatact gcactatcaa cacactctta agtttgcttc taagtcttat ttccataact 16860

tcttttacgt ttccgggtac aattcgtaat catgtcatag ctgtttcctg tgtgaaattc 16920

ttatccgctc acaattccac acaacatacg agccggaagc ataaagtgta aagcctgggg 16980

tgcctaatga gtgagctaac tcacattaat tgcgttgcgc tcactgcccg ctttccagtc 17040

gggaaacctg tcgtgccaga aaacttcatt tttaatttaa aaggatctag gtgaagatcc 17100

tttttgataa tctcatgacc aaaatccctt aacgtgagtt ttcgttccac tgagcgtcag 17160

accccgtaga aaagatcaaa ggatcttctt gagatccttt ttttctgcgc gtaatctgct 17220

gcttgcaaac aaaaaaacca ccgctaccag cggtggtttg tttgccggat caagagctac 17280

caactctttt tccgaaggta actggcttca gcagagcgca gataccaaat actgtccttc 17340

tagtgtagcc gtagttaggc caccacttca agaactctgt agcaccgcct acatacctcg 17400

ctctgctaat cctgttacca gtggctgctg ccagtggcga taagtcgtgt cttaccgggt 17460

tggactcaag acgatagtta ccggataagg cgcagcggtc gggctgaacg gggggttcgt 17520

gcacacagcc cagcttggag cgaacgacct acaccgaact gagataccta cagcgtgagc 17580

tatgagaaag cgccacgctt cccgaaggga gaaaggcgga caggtatccg gtaagcggca 17640

gggtcggaac aggagagcgc acgagggagc ttccaggggg aaacgcctgg tatctttata 17700

gtcctgtcgg gtttcgccac ctctgacttg agcgtcgatt tttgtgatgc tcgtcagggg 17760

ggcggagcct atggaaaaac gccagcaacg cggccttttt acggttcctg gccttttgct 17820

ggccttttgc tcacatgttc tttcctgcgt tatcccctga ttctgtggat aaccgtatta 17880

ccgcctttga gtgagctgat accgctcgcc gcagccgaac gaccgagcgc agcgagtcag 17940

tgagcgagga agcggaagag cgcccaatac gcaaaccgcc tctccccgcg cgttggccga 18000

ttcattaatg cagctggcac gacaggtttc ccgactggaa agcgggcagt gagcgcaacg 18060

caattaatgt gagttagctc actcattagg caccccaggc tttacacttt atgcttccgg 18120

ctcgtatgtt gtgtggaatt gtgagcggat aacaatttca cacaggaaac agctatgacc 18180

atgattac 18188

<210> 14
<211> 16239
<212> DNA
<213> Artificial

<220>
<223> Gene cluster

<400> 14
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt ggagatcggt acttcgcgaa 420

tgcgtcgaga tggcgcgcct tggtaaagga tatatggtag tatttgcagg ggatacatag 480

ggagatatag aagttctacc aataggttca gaatttagcc atagttctga tctaaaagct 540

gtactagacg tatttgatac tgtaaaggta tttatatata acttaactcc tgaatcctct 600

gggtttatca agcctcccca agcatttaaa ttacgttcta gaattagaaa gggggtatga 660

cccataaaat attttccttt aaaagattca tatacacgat agggtaggtt tactacttta 720

gttggtttat catgatgact tacggtatcg gaatttaaaa tgttttgatt ttccataaat 780

atgacctcct agtatttagt attattttat gtaaatatat atgtagaagt gtaccatttg 840

tgcaagattt caataaaggg tatattttac ctattttttt agtataaaaa atgcaaaaaa 900

tatgaacaaa agtagagttc ctatgtatta aattgtaaaa tatccactaa aaaaataaaa 960

ttataataaa aaatacaaaa aaataattga caatatataa ataattatgc ataattatat 1020

catgataaca attagttaag cataattaca tatatatgaa cataatatga catcttagaa 1080

gcatatcttt cgttagtaat aatataattt cctttagaag aaaatgattt atttaaaata 1140

aatagtgtaa tgttttttat aatttcaaaa agttccccaa tttagcatac taggcatgat 1200

aaaaatagct tgaataagtg cccgggatta tttattgata catagagaat ttcactcttt 1260

gcattttatc taacatcaag gggtttattt gtcacaaatt atgtaaaaat aaaacaaaga 1320

tgtaagaaag tcctatgata taaattttgt aaacataata aattagcttt cataagattg 1380

gaagaatgat aattactact tagaactgct aaaaattagg aaagaggtgt cgttaattaa 1440

tggaaaccag aaggtctgcc aattatgaac caaatagctg ggattatgat tatttgctgt 1500

cttctgacac tgacgaatct attgaagtat acaaagacaa ggccaaaaag ctggaggctg 1560

aggtgagaag agagattaac aatgaaaagg cagagttttt gactctgcct gaactgatag 1620

ataatgttca aaggttagga ttaggttaca gattcgagag tgacataagg agagcccttg 1680

atagatttgt ttcttcagga ggatttgatg ctgttacaaa aactagcctt catgctactg 1740

ctcttagctt caggcttctc agacagcatg gctttgaggt atctcaagaa gctttcagcg 1800

gattcaagga tcaaaatggc aatttcttga aaaaccttaa ggaggacatc aaggcaatac 1860

taagcctata tgaagcttca tttcttgcct tagaaggaga aaatatcttg gatgaggcca 1920

aggtgtttgc aatatcacat ctaaaagagc ttagcgaaga aaagattgga aaagacctgg 1980

ccgaacaggt gaatcatgca ttggagcttc cattgcatag aaggacacaa agactagaag 2040

ctgtttggag cattgaagca tacagaaaaa aggaagatgc agatcaagta ctgctagaac 2100

ttgctatatt ggactacaac atgattcaat cagtatacca aagagatctt agagagacat 2160

caaggtggtg gaggagagtg ggtcttgcaa caaagttgca ttttgctaga gacaggttaa 2220

ttgaaagctt ttactgggca gttggagttg catttgaacc tcaatacagt gattgtagaa 2280

attccgtagc aaaaatgttt tcttttgtaa caatcattga tgatatctat gatgtttatg 2340

gtactctgga tgagttggag ctatttacag atgctgttga gagatgggat gttaatgcca 2400

tcgatgatct tcctgattat atgaagcttt gtttcctagc tctttataac actatcaatg 2460

agatagctta tgataatctg aaggacaagg gggaaaacat tcttccatac ctaacaaaag 2520

catgggcaga tttatgtaat gcattcctac aagaagcaaa atggttgtac aataagtcca 2580

caccaacatt tgatgaatat ttcggaaatg catggaaatc atcctcaggg cctcttcaac 2640

tagtttttgc ctactttgcc gttgttcaaa acatcaagaa agaggaaatt gataacttac 2700

aaaagtatca tgatatcatc agtaggcctt cccatatctt tagactttgt aacgacttgg 2760

cttcagcatc tgctgagata gcaagaggtg aaaccgcaaa ttctgtatca tgttacatga 2820

gaacaaaagg catttctgag gaacttgcta ctgaatccgt aatgaatttg atcgacgaaa 2880

cctggaaaaa gatgaacaaa gaaaagcttg gtggctctct gtttgcaaaa ccttttgttg 2940

aaacagctat taaccttgca agacaatccc attgtactta tcataacgga gatgcacata 3000

cttcaccaga tgagcttact aggaaaagag tactgtcagt aatcacagag cctattctac 3060

cttttgagag ataataattt taaaatataa gtgatttaga tattcataat atatttggga 3120

ggtaaattaa tatgaccagc gcccaacgta aggacgacca tgtaaggctc gccatcgagc 3180

agcataacgc ccatagcgga cgtaaccagt tcgacgacgt gtctttcgta catcatgccc 3240

tggccggcat cgacaggcca gacgtgtccc tggccacgtc cttcgccggg atctcctggc 3300

aggtgcctat ctacatcaac gcgatgaccg gcggcagcga gaagaccggc ctcatcaaca 3360

gggacctggc caccgccgcc cgtgagaccg gcgtacccat cgcgtccggg tccatgaacg 3420

cgtacatcaa ggacccctcc tgcgccgaca cgttccgtgt gctgcgtgac gagaacccca 3480

acgggttcgt aatcgcgaac atcaacgcca ccacgacggt tgacaacgcg cagcgtgcga 3540

tcgacctgat cgaggcgaac gccctgcaga tccatatcaa cacggcgcag gagacgccta 3600

tgcctgaggg cgacaggtct ttcgcgtcct gggtccctca gatcgagaag atcgcggcgg 3660

ccgtagacat ccccgtgatc gtaaaggagg taggcaacgg cctgagcagg cagaccatcc 3720

tgctgctcgc cgacctcggc gtgcaggcgg cggacgtaag cggccgtggc ggcacggact 3780

tcgcccgtat cgagaacggc cgtagggagc tcggcgacta cgcgttcctg catggctggg 3840

ggcagtccac cgccgcctgc ctgctggacg cccaggacat ctccctgccc gtactcgcct 3900

ccggcggtgt gcgtcatcct ctcgacgtgg tacgtgccct cgcgctcggc gcccgtgccg 3960

taggctcctc cgccggcttc ctgcgtaccc tgatggacga cggcgtagac gcgctgatca 4020

cgaagctcac gacctggctg gaccagctgg cggcgctgca gaccatgctc ggcgcgcgta 4080

cccctgccga cctcacccgt tgcgacgtgc tgctccatgg cgagctgcgt gacttctgcg 4140

ccgacagggg catcgacacg cgtcgtctcg cccagcgttc cagctccatc gaggccctcc 4200

agacgacggg aagcacaaga taataatttt aaaatataag tgatttagat attcataata 4260

tatttgggag gtaaattaat atgaaagaag ttgtaatagc tagtgcagta agaacagcga 4320

ttggatctta tggaaagtct cttaaggatg taccagcagt agatttagga gctacagcta 4380

taaaggaagc agttaaaaaa gcaggaataa aaccagagga tgttaatgaa gtcattttag 4440

gaaatgttct tcaagcaggt ttaggacaga atccagcaag acaggcatct tttaaagcag 4500

gattaccagt tgaaattcca gctatgacta ttaataaggt ttgtggttca ggacttagaa 4560

cagttagctt agcagcacaa attataaaag caggagatgc tgacgtaata atagcaggtg 4620

gtatggaaaa tatgtctaga gctccttact tagcgaataa cgctagatgg ggatatagaa 4680

tgggaaacgc taaatttgtt gatgaaatga tcactgacgg attgtgggat gcatttaatg 4740

attaccacat gggaataaca gcagaaaaca tagctgagag atggaacatt tcaagagaag 4800

aacaagatga gtttgctctt gcatcacaaa aaaaagctga agaagctata aaatcaggtc 4860

aatttaaaga tgaaatagtt cctgtagtaa ttaaaggcag aaagggagaa actgtagttg 4920

atacagatga gcaccctaga tttggatcaa ctatagaagg acttgcaaaa ttaaaacctg 4980

ccttcaaaaa agatggaaca gttacagctg gtaatgcatc aggattaaat gactgtgcag 5040

cagtacttgt aatcatgagt gcagaaaaag ctaaagagct tggagtaaaa ccacttgcta 5100

agatagtttc ttatggttca gcaggagttg acccagcaat aatgggatat ggacctttct 5160

atgcaacaaa agcagctatt gaaaaagcag gttggacagt tgatgaatta gatttaatag 5220

aatcaaatga agcttttgca gctcaaagtt tagcagtagc aaaagattta aaatttgata 5280

tgaataaagt aaatgtaaat ggaggagcta ttgcccttgg tcatccaatt ggagcatcag 5340

gtgcaagaat actcgttact cttgtacacg caatgcaaaa aagagatgca aaaaaaggct 5400

tagcaacttt atgtataggt ggcggacaag gaacagcaat attgctagaa aagtgctagt 5460

agtttaaaag caaatataaa tgaaaaattg aaccctagca ttatgtaaat gcagggttta 5520

atttttatat taagcagcat aatagaaagt tttttaaatg catgtatata tggggtattt 5580

aaagggaaat ctataatata atttaggact atacatagag aatttcactc tttgcatttt 5640

atctaacatc aaggggttta tttgtcacaa attatgtaaa aataaaacaa agatgtaaga 5700

aagtcctatg atataaattt tgtaaacata ataaattagc tttcataaga ttggaagaat 5760

gataattact acttagaact gctaaaaatt aggaaagagg tgtcgttaat taatgcagaa 5820

aagacaaagg gagctgagtg cgttgacact acctacctct gctgaggggg tatcagaaag 5880

ccatagggcc cgttctgtcg gcatcggtcg tgcccatgcc aaggccatcc tgctgggaga 5940

gcatgcggta gtatacggag cgccggcact cgctctgcct attcctcagc tcacggtcac 6000

ggccagcgtt ggctggtctt ccgaggcctc cgacagtgcg ggtggcctgt cctacacgat 6060

gaccggtacg ccttctaggg cactggtgac gcaggcctcc gacggcctgc ataggctcac 6120

cgcggaattc atggcgagga tgggcgtgac gaacgcgcct catctcgacg tgatcctgga 6180

cggcgcgatc cctcacggca ggggtctcgg ctccagcgcg gccggctcac gtgcgatcgc 6240

cttggccctc gccgacctct tcggccacga actggccgag catacggcgt acgaactggt 6300

gcagacggcc gagaacatgg cgcatggcag ggccagcggc gtggacgcga tgacggtcgg 6360

cgcgtccagg cctctgctgt tccagcaggg ccgtaccgag agactggcca tcggctgcga 6420

cagcctgttc atcgtagccg acagcggcgt acctggcagc accaaggaag cggtagagat 6480

gctgagggag ggattcaccc gtagcgccgg aacacaggag cggttcgttg gcagggcgac 6540

ggaactgacc gaggccgcca ggcaggccct cgccgacggc aggcccgagg agctgggctc 6600

tcagctgacg tactaccatg agctgctcca tgaggcccgt ctgagcaccg acggcatcga 6660

tgcgctggta gaggccgcgc tgaaggcagg cagcctcgga gccaagatca ccggcggtgg 6720

tctgggcggc tgcatgatcg cacaggccag gcccgaacag gccagggagg taaccaggca 6780

gctccatgag gccggtgccg tacagacctg ggtagtacca ctgaaagggc tcgacaacca 6840

tgcgcagtaa taattttaaa atataagtga tttagatatt cataatatat ttgggaggta 6900

aattaatatg cgtagtgaac atcctaccac gaccgtgctc cagtctaggg agcagggcag 6960

cgcggccggc gccaccgcgg tagcgcatcc aaacatcgcg ctgatcaagt actggggcaa 7020

gcgtgacgag aggctgatcc tgccctgcac caccagcctg tctatgacgc tggacgtatt 7080

ccccacgacc accgaggtca ggctcgaccc cgccgccgag catgacacgg ccgccctcaa 7140

cggcgaggtg gccacgggcg agacgctgcg tcgtatcagc gccttcctct ccctggtgag 7200

ggaggtggcg ggcagcgacc agagggccgt ggtggacacc cgtaacaccg tgcccaccgg 7260

ggcgggcctg gcgtcctccg ccagcgggtt cgccgccctc gccgtcgcgg ccgcggccgc 7320

ctacgggctc gaactcgacg accgtgggct gtccaggctg gccagacgtg gatccggctc 7380

cgcctctcgg tctatcttcg gcggcttcgc cgtatggcat gccggccccg acggcacggc 7440

cacggaagcg gacctcggct cctacgccga gccagtgccc gcggccgacc tcgaccctgc 7500

gctggttatc gccgtggtaa acgccggccc caagcccgta tccagccgtg aggccatgcg 7560

tcgcaccgta gacacctcac cactgtacag gccatgggcc gactccagta aggacgacct 7620

ggacgagatg cgttctgcgc tgctgcgtgg cgacctcgag gccgtgggcg agatcgcgga 7680

gcgtaacgcg ctcggcatgc atgccaccat gctggccgcc cgtcccgcgg tgaggtacct 7740

gtcaccagcc acggtaaccg tgctcgacag cgtgctccag ctccgtaagg acggtgttct 7800

ggcctacgcg accatggacg ccggtcccaa cgtgaaggtg ctgtgcagga gggcggacgc 7860

cgagagggtg gccgacgttg tacgcgccgc cgcgtccggc ggtcaggtac tcgtagccgg 7920

gcctggagac ggtgcccgtc tgctgagcga gggcgcataa taattttaaa atataagtga 7980

tttagatatt cataatatat ttgggaggta aattaatatg acgacaggtc agcgtacgat 8040

cgtcaggcat gcgcctggca agctgttcgt agcgggcgag tacgcggtag tggatcctgg 8100

caaccctgcg atcctggtag cggtagacag gcatatcagc gtaaccgtgt ccgacgccga 8160

cgcggacacc ggggccgccg acgtagtgat ctcctccgac ctcggtcctc aggcggtagg 8220

ctggcgttgg catgacggca ggctcgtagt acgtgaccct gacgacgggc agcaggcgcg 8280

tagcgccctg gcccatgtgg tgtcggcgat cgagaccgtg ggcaggctgc tgggcgaacg 8340

tggacagaag gtacccgctc tcaccctctc cgttagcagc cgtctgcatg aggacggcag 8400

gaagttcggc ctgggctcca gcggcgcggt gaccgtggcg accgtagccg ccgtagccgc 8460

gttctgcgga ctcgaactgt ccaccgacga aaggttcagg ctggccatgc tcgccaccgc 8520

ggaactcgac cccaagggct ccggcgggga cctcgccgcc agcacctggg gcggctggat 8580

cgcctaccag gcgcccgaca gggcctttgt gctcgacctg gccaggcgtg tgggagtaga 8640

caggacactg aaggcgccct ggccggggca ttctgtgcgt agactgcctg cgcccaaggg 8700

cctcaccctg gaggtcggct ggaccggaga gcccgcctcc accgcgtccc tggtgtccga 8760

tctgcatcgt cgtacctgga ggggcagcgc ctcccatcag aggttcgtag agaccacgac 8820

cgactgtgta cgttccgcgg ttaccgccct ggagtccggc gacgacacga gcctgctgca 8880

tgagatccgc agggcccgtc aggagctggc ccgtctggac gacgaggtag gcctcggcat 8940

cttcacaccc aagctgacgg cgctgtgcga cgccgccgaa gccgttggcg gcgcggccaa 9000

gccctccggg gcaggcggcg gcgactgcgg catcgccctg ctggacgccg aggcgtctag 9060

ggacatcaca catgtaaggc aacggtggga gacagccggg gtgctgcccc tgcccctgac 9120

tcctgccctg gaagggatct aataatttta aaatataagt gatttagata ttcataatat 9180

atttgggagg taaattaata tgagcctcga ttccagactg cccgctttcc gtaacctgtc 9240

ccctgccgcg agactggacc acatcggcca gttgctcggc ctgagccacg acgatgtcag 9300

cctgctggcc aacgccggtg ccctgccgat ggacatcgcc aacggcatga tcgaaaacgt 9360

catcggcacc ttcgagctgc cctatgccgt ggccagcaac ttccagatca atggccgtga 9420

tgtgctggtg ccgctggtgg tggaagagcc ctcgatcgtc gccgctgctt cttacatggc 9480

caagctggcc cgtgccaacg gcggcttcac cacctccagc agcgccccgc tgatgcatgc 9540

ccaggtacag atcgtcggca tacaggaccc gctcaatgca cgtctgagcc tgctgagaag 9600

aaaagacgaa atcattgaac tggccaaccg taaggaccag ttgctcaaca gcctcggcgg 9660

cggctgcaga gacatcgaag tgcacacctt cgccgatacc ccgcgtggcc cgatgctggt 9720

ggcgcacctg atcgtcgatg taagagatgc catgggcgcc aacaccgtca ataccatggc 9780

cgaggccgtt gcgccgctga tggaagccat caccgggggc caggtacgtc tgagaattct 9840

gtccaacctg gccgacctgc gcctggccag ggcccaggtg aggattactc cgcagcaact 9900

ggaaacggcc gaattcagtg gcgaggcagt gatcgaaggc atcctcgacg cctacgcctt 9960

cgctgcggtc gacccttaca gagcggccac ccacaacaag ggcatcatga atggcatcga 10020

cccactgatc gtcgccactg gcaacgactg gcgtgcagtg gaagccggcg cccatgcgta 10080

tgcctgcaga agtggtcact acggctcgct gaccacctgg gaaaaggaca acaacggcca 10140

tttggtcggc accctggaaa tgccgatgcc cgtaggcctg gtcggcggcg ccaccaaaac 10200

ccatccgctg gcgcaactgt cactgagaat cctcggcgtg aaaacagccc aggcgctcgc 10260

tgagattgcc gtggccgtag gcctggcgca aaacctcggg gccatgagag ccctggccac 10320

cgaaggcatc cagcgtggcc acatggccct gcatgcgaga aatattgccg tggtggcggg 10380

cgcccgaggc gatgaggtgg actgggttgc ccggcagttg gtggaatacc acgacgtgag 10440

agccgacaga gccgtagcac tgctgaaaca aaagagaggc caatgatagt tttaaaatat 10500

aagtgattta gatattcata atatatttgg gaggtaaatt aatatgtcca tctccatagg 10560

cattcacgac ctgtctttcg ccacaaccga gttcgtactg cctcatacgg cgctcgccga 10620

gtacaacggc accgagatcg gcaagtacca tgtaggcatc ggccagcagt ctatgagcgt 10680

gcctgccgcc gacgaggaca tcgtgaccat ggccgcgacc gcggcgaggc ccatcatcga 10740

gcgtaacggc aagagcagga tccgtacggt agtgttcgcc acggagtctt ctatcgacca 10800

ggcgaaggcg ggcggcgtat acgtgcactc cctgctgggg ctggagtctg cctgcagggt 10860

agtagagctg aagcaggcct gctacggggc caccgccgcc cttcagttcg ccatcggcct 10920

ggtgaggcgt gaccccgccc agcaggtact ggtaatcgcc agtgacgtat ccaagtacga 10980

gctggacagc cccggcgagg cgacccaggg cgcggccgcg gtggccatgc tggtaggcgc 11040

cgaccctgcc ctgctgcgta tcgaggagcc ttcgggcctg ttcaccgccg acgtaatgga 11100

cttctggcgg cccaactacc tcaccaccgc tctggtagac ggccaggagt ccatcaacgc 11160

ctacctgcag gccgtagagg gcgcctggaa ggactacgcg gagcaggacg gcaggtcact 11220

ggaggagttc gcggcgttcg tataccacca gccgttcacg aagatggcct acaaggcgca 11280

tcgccatctg ctgaacttca acggctacga caccgacaag gacgccatcg agggcgccct 11340

cggccagacg acggcgtaca acaacgtaat cggcaacagc tacaccgcgt ctgtgtacct 11400

gggcctggcc gccctgctcg accaggcgga cgacctgacg ggccgttcca tcggcttcct 11460

gagctacggc tctggcagcg tagccgagtt cttctctggc accgtagtag ccgggtaccg 11520

tgagcgtctg cgtaccgagg cgaaccagga ggcgatcgcc aggcgtaaga gcgtagacta 11580

cgccacctac cgtgagctgc acgagtacac gctcccgtcc gacggcggcg accatgccac 11640

ccctgtgcag accaccggcc ccttcaggct ggccgggatc aacgaccata agcgtatcta 11700

cgaggcgcgt taataattta aaagcaaata taaatgaaaa attgaaccct agcattatgt 11760

aaatgcaggg tttaattttt atattaagca gcataataga aagtttttta aatgcatgta 11820

tatatggggt atttaaaggg aaatctataa tataatttag gactattccg gataccgttc 11880

gtataatgta tgctatacga agttatttca gattaaattt ttgcttattt gatttacatt 11940

atataatatt gagtaaagta ttgactagca aaattttttg atactttaat ttgtgaaatt 12000

tcttatcaaa agttatattt ttgaatgatt tttattgaaa aatacaacta aaaaggatta 12060

tagtataagt gtgtgtaatt ttgtgttaaa tttaaaggga ggaaatgaac atgaacttta 12120

ataaaattga tttagacaat tggaagagaa aagagatatt taatcattat ttgaaccaac 12180

aaacgacttt tagtataacc acagaaattg atattagtgt tttatacaga aacataaaac 12240

aagaaggata taaattttac cctgcattta ttttcttagt gacaagggtg ataaactcaa 12300

atacagcttt tagaactggt tacaatagcg acggagagtt aggttattgg gataagttag 12360

agccacttta tacaattttt gatggtgtat ctaaaacatt ctctggtatt tggactcctg 12420

taaagaatga cttcaaagag ttttatgatt tatacctttc tgatgtagag aaatataatg 12480

gttcggggaa attgtttccc aaaacaccta tacctgaaaa tgctttttct ctttctatta 12540

ttccatggac ttcatttact gggtttaact taaatatcaa taataatagt aattaccttc 12600

tacccattat tacagcagga aaattcatta ataaaggtaa ttcaatatat ttaccgctat 12660
ctttacaggt acatcattct gtttgtgatg gttatcatgc aggattgttt atgaactcta 12720
ttcaggaatt gtcagatagg cctaatgact ggcttttata ataatttaaa agcaaatata 12780
aatgaaaaat tgaaccctag cattatgtaa atgcagggtt taatttttat attaagcagc 12840
ataatagaaa gttttttaaa tgcatgtata tatggggtat ttaaagggaa atctataata 12900
taatttagga ctatataact tcgtataatg tatgctatac gaacggtacc taggatatat 12960
aataaattga atatagtaaa caaaaaggga catatttata atatgttctt tttagtttaa 13020
tactcaattt ttgcacataa gaaattaact taatataaaa aaatttgcga agctttgctt 13080
cgcagtttaa tattgtttag gtggttaaat tatgaatctg gaagtgttaa aaacagagtt 13140
taagtattta agagataaaa taattgaaaa gcaatatgaa catcttgatc ctatgcaaag 13200
aaaagcagtt ttaaatggtg aaaataactg tattgttatt gcttgtcctg gagcaggaaa 13260
gacccagact attattaata gagtggacta cttatgtaga ttcggtccta tatacaatac 13320
agattatgta cctaattgtc taaagaccga tgatttacag ataatgaaga aatatttaaa 13380
tgataattct tttaaagatg tgactgcagt aaataaaatt gagcatttgt taaatagcaa 13440
taaaataaat ccacagaaca tagttgttat aacttttact agagcagctg ctctcaatat 13500
gaaaaacaga tacatatcta taggaaataa agaaaagtca cctttttttg gaacattcca 13560
ctccctattt tataatatat tgaaaaagca taataaagaa ataaatatta tagatcctta 13620
taaggcacat gagatagtta aaaatacact tatgtattat ctggacttta taggagaaga 13680
gagagtaaag gaagttctaa atgacatatc tcttttaaaa aatagtgaaa ctaacataga 13740
tttatttaaa agtaaaattg acaaaagtgt atttttaaaa tgttttaatg aatatgaaaa 13800
ttataaagct agaaataagc ttatggattt tgatgattta caattaaaag ttaaagatat 13860
gtttctaaat cagaaatcta ttctagatag ttatcagaat ttgttcaagt atattttagt 13920
tgatgagttt caggattcag ataacctcca aatattcgaa atcggatgcc gggaccgacg 13980
agtgcagagg cgtgcaagcg agcttggcgt aatcatggtc atagctgttt cctgtgtgaa 14040
attgttatcc gctcacaatt ccacacaaca tacgagccgg aagcataaag tgtaaagcct 14100
ggggtgccta atgagtgagc taactcacat taattgcgtt gcgctcactg cccgctttcc 14160
agtcgggaaa cctgtcgtgc cagctgcatt aatgaatcgg ccaacgcgcg gggagaggcg 14220
gtttgcgtat tgggcgctct tccgcttcct cgctcactga ctcgctgcgc tcggtcgttc 14280
ggctgcggcg agcggtatca gctcactcaa aggcggtaat acggttatcc acagaatcag 14340
gggataacgc aggaaagaac atgtgagcaa aaggccagca aaaggccagg aaccgtaaaa 14400
aggccgcgtt gctggcgttt ttccataggc tccgcccccc tgacgagcat cacaaaaatc 14460
gacgctcaag tcagaggtgg cgaaacccga caggactata aagataccag gcgtttcccc 14520

ctggaagctc cctcgtgcgc tctcctgttc cgaccctgcc gcttaccgga tacctgtccg 14580

cctttctccc ttcgggaagc gtggcgcttt ctcatagctc acgctgtagg tatctcagtt 14640

cggtgtaggt cgttcgctcc aagctgggct gtgtgcacga accccccgtt cagcccgacc 14700

gctgcgcctt atccggtaac tatcgtcttg agtccaaccc ggtaagacac gacttatcgc 14760

cactggcagc agccactggt aacaggatta gcagagcgag gtatgtaggc ggtgctacag 14820

agttcttgaa gtggtggcct aactacggct acactagaag aacagtattt ggtatctgcg 14880

ctctgctgaa gccagttacc ttcggaaaaa gagttggtag ctcttgatcc ggcaaacaaa 14940

ccaccgctgg tagcggtggt ttttttgttt gcaagcagca gattacgcgc agaaaaaaag 15000

gatctcaaga agatcctttg atcttttcta cggggtctga cgctcagtgg aacgaaaact 15060

cacgttaagg gattttggtc atgagattat caaaaaggat cttcacctag atccttttaa 15120

attaaaaatg aagttttaaa tcaatctaaa gtatatatga gtaaacttgg tctgacagtt 15180

accaatgctt aatcagtgag gcacctatct cagcgatctg tctatttcgt tcatccatag 15240

ttgcctgact ccccgtcgtg tagataacta cgatacggga gggcttacca tctggcccca 15300

gtgctgcaat gataccgcga gacccacgct caccggctcc agatttatca gcaataaacc 15360

agccagccgg aagggccgag cgcagaagtg gtcctgcaac tttatccgcc tccatccagt 15420

ctattaattg ttgccgggaa gctagagtaa gtagttcgcc agttaatagt ttgcgcaacg 15480

ttgttgccat tgctacaggc atcgtggtgt cacgctcgtc gtttggtatg gcttcattca 15540

gctccggttc ccaacgatca aggcgagtta catgatcccc catgttgtgc aaaaaagcgg 15600

ttagctcctt cggtcctccg atcgttgtca gaagtaagtt ggccgcagtg ttatcactca 15660

tggttatggc agcactgcat aattctctta ctgtcatgcc atccgtaaga tgcttttctg 15720

tgactggtga gtactcaacc aagtcattct gagaatagtg tatgcggcga ccgagttgct 15780

cttgcccggc gtcaatacgg gataataccg cgccacatag cagaacttta aaagtgctca 15840

tcattggaaa acgttcttcg gggcgaaaac tctcaaggat cttaccgctg ttgagatcca 15900

gttcgatgta acccactcgt gcacccaact gatcttcagc atcttttact ttcaccagcg 15960

tttctgggtg agcaaaaaca ggaaggcaaa atgccgcaaa aaagggaata agggcgacac 16020

ggaaatgttg aatactcata ctcttccttt ttcaatatta ttgaagcatt tatcagggtt 16080

attgtctcat gagcggatac atatttgaat gtatttagaa aaataaacaa ataggggttc 16140

cgcgcacatt tccccgaaaa gtgccacctg acgtctaaga aaccattatt atcatgacat 16200

taacctataa aaataggcgt atcacgaggc cctttcgtc 16239

<210> 15
<211> 17400
<212> DNA

<213>  Artificial

<220>
<223>  Gene cluster

<400>  15
   gaattcgagc tcggtacccg ggattattta ttgatacata gagaatttca ctctttgcat      60
   tttatctaac atcaaggggt ttatttgtca caaattatgt aaaaataaaa caaagatgta     120
   agaaagtcct atgatataaa ttttgtaaac ataataaatt agctttcata agattggaag     180
   aatgataatt actacttaga actgctaaaa attaggaaag aggtgtcgtt aattaatgga     240
   aaccagaagg tctgccaatt atgaaccaaa tagctgggat tatgattatt tgctgtcttc     300
   tgacactgac gaatctattg aagtatacaa agacaaggcc aaaaagctgg aggctgaggt     360
   gagaagagag attaacaatg aaaaggcaga gtttttgact ctgcctgaac tgatagataa     420
   tgttcaaagg ttaggattag gttacagatt cgagagtgac ataaggagag cccttgatag     480
   atttgtttct tcaggaggat ttgatgctgt tacaaaaact agccttcatg ctactgctct     540
   tagcttcagg cttctcagac agcatggctt tgaggtatct caagaagctt tcagcggatt     600
   caaggatcaa aatggcaatt tcttgaaaaa ccttaaggag gacatcaagg caatactaag     660
   cctatatgaa gcttcatttc ttgccttaga aggagaaaat atcttggatg aggccaaggt     720
   gtttgcaata tcacatctaa aagagcttag cgaagaaaag attggaaaag acctggccga     780
   acaggtgaat catgcattgg agcttccatt gcatagaagg acacaaagac tagaagctgt     840
   ttggagcatt gaagcataca gaaaaaagga agatgcagat caagtactgc tagaacttgc     900
   tatattggac tacaacatga ttcaatcagt ataccaaaga gatcttagag agacatcaag     960
   gtggtggagg agagtgggtc ttgcaacaaa gttgcatttt gctagagaca ggttaattga    1020
   aagcttttac tgggcagttg gagttgcatt tgaacctcaa tacagtgatt gtagaaattc    1080
   cgtagcaaaa atgttttctt ttgtaacaat cattgatgat atctatgatg tttatggtac    1140
   tctggatgag ttggagctat ttacagatgc tgttgagaga tgggatgtta atgccatcga    1200
   tgatcttcct gattatatga agctttgttt cctagctctt tataacacta tcaatgagat    1260
   agcttatgat aatctgaagg acaaggggga aaacattctt ccatacctaa caaaagcatg    1320
   ggcagattta tgtaatgcat tcctacaaga agcaaaatgg ttgtacaata agtccacacc    1380
   aacatttgat gaatatttcg gaaatgcatg gaaatcatcc tcagggcctc ttcaactagt    1440
   ttttgcctac tttgccgttg ttcaaaacat caagaaagag gaaattgata acttacaaaa    1500
   gtatcatgat atcatcagta ggccttccca tatctttaga ctttgtaacg acttggcttc    1560
   agcatctgct gagatagcaa gaggtgaaac cgcaaattct gtatcatgtt acatgagaac    1620
   aaaaggcatt tctgaggaac ttgctactga atccgtaatg aatttgatcg acgaaacctg    1680
   gaaaaagatg aacaaagaaa agcttggtgg ctctctgttt gcaaaacctt ttgttgaaac    1740
   agctattaac cttgcaagac aatcccattg tacttatcat aacggagatg cacatacttc 1800
   accagatgag cttactagga aaagagtact gtcagtaatc acagagccta ttctaccttt 1860
   tgagagataa taattttaaa atataagtga tttagatatt cataatatat ttgggaggta 1920
   aattaatatg accagcgccc aacgtaagga cgaccatgta aggctcgcca tcgagcagca 1980
   taacgcccat agcggacgta accagttcga cgacgtgtct ttcgtacatc atgccctggc 2040
   cggcatcgac aggccagacg tgtccctggc cacgtccttc gccgggatct cctggcaggt 2100
   gcctatctac atcaacgcga tgaccggcgg cagcgagaag accggcctca tcaacaggga 2160
   cctggccacc gccgcccgtg agaccggcgt acccatcgcg tccgggtcca tgaacgcgta 2220
   catcaaggac ccctcctgcg ccgacacgtt ccgtgtgctg cgtgacgaga accccaacgg 2280
   gttcgtaatc gcgaacatca acgccaccac gacggttgac aacgcgcagc gtgcgatcga 2340
   cctgatcgag gcgaacgccc tgcagatcca tatcaacacg gcgcaggaga cgcctatgcc 2400
   tgagggcgac aggtctttcg cgtcctgggt ccctcagatc gagaagatcg cggcggccgt 2460
   agacatcccc gtgatcgtaa aggaggtagg caacggcctg agcaggcaga ccatcctgct 2520
   gctcgccgac ctcggcgtgc aggcggcgga cgtaagcggc cgtggcggca cggacttcgc 2580
   ccgtatcgag aacggccgta gggagctcgg cgactacgcg ttcctgcatg gctgggggca 2640
   gtccaccgcc gcctgcctgc tggacgccca ggacatctcc ctgcccgtac tcgcctccgg 2700
   cggtgtgcgt catcctctcg acgtggtacg tgccctcgcg ctcggcgccc gtgccgtagg 2760
   ctcctccgcc ggcttcctgc gtaccctgat ggacgacggc gtagacgcgc tgatcacgaa 2820
   gctcacgacc tggctggacc agctggcggc gctgcagacc atgctcggcg cgcgtacccc 2880
   tgccgacctc acccgttgcg acgtgctgct ccatggcgag ctgcgtgact tctgcgccga 2940
   caggggcatc gacacgcgtc gtctcgccca gcgttccagc tccatcgagg ccctccagac 3000
   gacgggaagc acaagataat aattttaaaa tataagtgat ttagatattc ataatatatt 3060
   tgggaggtaa attaatatga aagaagttgt aatagctagt gcagtaagaa cagcgattgg 3120
   atcttatgga aagtctctta aggatgtacc agcagtagat ttaggagcta cagctataaa 3180
   ggaagcagtt aaaaaagcag gaataaaacc agaggatgtt aatgaagtca ttttaggaaa 3240
   tgttcttcaa gcaggtttag gacagaatcc agcaagacag gcatctttta aagcaggatt 3300
   accagttgaa attccagcta tgactattaa taaggtttgt ggttcaggac ttagaacagt 3360
   tagcttagca gcacaaatta taaaagcagg agatgctgac gtaataatag caggtggtat 3420
   ggaaaatatg tctagagctc cttacttagc gaataacgct agatggggat atagaatggg 3480
   aaacgctaaa tttgttgatg aaatgatcac tgacggattg tgggatgcat ttaatgatta 3540
   ccacatggga ataacagcag aaaacatagc tgagagatgg aacatttcaa gagaagaaca 3600
   agatgagttt gctcttgcat cacaaaaaaa agctgaagaa gctataaaat caggtcaatt 3660
   taaagatgaa atagttcctg tagtaattaa aggcagaaag ggagaaactg tagttgatac 3720
   agatgagcac cctagatttg gatcaactat agaaggactt gcaaaattaa aacctgcctt 3780
   caaaaaagat ggaacagtta cagctggtaa tgcatcagga ttaaatgact gtgcagcagt 3840
   acttgtaatc atgagtgcag aaaaagctaa agagcttgga gtaaaaccac ttgctaagat 3900
   agtttcttat ggttcagcag gagttgaccc agcaataatg ggatatggac ctttctatgc 3960
   aacaaaagca gctattgaaa aagcaggttg gacagttgat gaattagatt taatagaatc 4020
   aaatgaagct tttgcagctc aaagtttagc agtagcaaaa gatttaaaat ttgatatgaa 4080
   taaagtaaat gtaaatggag gagctattgc ccttggtcat ccaattggag catcaggtgc 4140
   aagaatactc gttactcttg tacacgcaat gcaaaaaaga gatgcaaaaa aaggcttagc 4200
   aactttatgt ataggtggcg gacaaggaac agcaatattg ctagaaaagt gctagtagtt 4260
   taaaagcaaa tataaatgaa aaattgaacc ctagcattat gtaaatgcag ggtttaattt 4320
   ttatattaag cagcataata gaaagttttt taaatgcatg tatatatggg gtatttaaag 4380
   ggaaatctat aatataattt aggactatac atagagaatt tcactctttg cattttatct 4440
   aacatcaagg ggtttatttg tcacaaatta tgtaaaaata aaacaaagat gtaagaaagt 4500
   cctatgatat aaattttgta aacataataa attagctttc ataagattgg aagaatgata 4560
   attactactt agaactgcta aaaattagga aagaggtgtc gttaattaat gcagaaaaga 4620
   caaagggagc tgagtgcgtt gacactacct acctctgctg agggggtatc agaaagccat 4680
   agggcccgtt ctgtcggcat cggtcgtgcc catgccaagg ccatcctgct gggagagcat 4740
   gcggtagtat acggagcgcc ggcactcgct ctgcctattc ctcagctcac ggtcacggcc 4800
   agcgttggct ggtcttccga ggcctccgac agtgcgggtg gcctgtccta cacgatgacc 4860
   ggtacgcctt ctagggcact ggtgacgcag gcctccgacg gcctgcatag gctcaccgcg 4920
   gaattcatgg cgaggatggg cgtgacgaac gcgcctcatc tcgacgtgat cctggacggc 4980
   gcgatccctc acggcagggg tctcggctcc agcgcggccg gctcacgtgc gatcgccttg 5040
   gccctcgccg acctcttcgg ccacgaactg gccgagcata cggcgtacga actggtgcag 5100
   acggccgaga acatggcgca tggcagggcc agcggcgtgg acgcgatgac ggtcggcgcg 5160
   tccaggcctc tgctgttcca gcagggccgt accgagagac tggccatcgg ctgcgacagc 5220
   ctgttcatcg tagccgacag cggcgtacct ggcagcacca aggaagcggt agagatgctg 5280
   agggagggat tcacccgtag cgccggaaca caggagcggt tcgttggcag ggcgacggaa 5340
   ctgaccgagg ccgccaggca ggccctcgcc gacggcaggc ccgaggagct gggctctcag 5400
   ctgacgtact accatgagct gctccatgag gcccgtctga gcaccgacgg catcgatgcg 5460
   ctggtagagg ccgcgctgaa ggcaggcagc ctcggagcca agatcaccgg cggtggtctg 5520
   ggcggctgca tgatcgcaca ggccaggccc gaacaggcca gggaggtaac caggcagctc 5580
   catgaggccg gtgccgtaca gacctgggta gtaccactga aagggctcga caaccatgcg 5640
   cagtaataat tttaaaatat aagtgattta gatattcata atatatttgg gaggtaaatt 5700
   aatatgcgta gtgaacatcc taccacgacc gtgctccagt ctagggagca gggcagcgcg 5760
   gccggcgcca ccgcggtagc gcatccaaac atcgcgctga tcaagtactg gggcaagcgt 5820
   gacgagaggc tgatcctgcc ctgcaccacc agcctgtcta tgacgctgga cgtattcccc 5880
   acgaccaccg aggtcaggct cgaccccgcc gccgagcatg acacggccgc cctcaacggc 5940
   gaggtggcca cgggcgagac gctgcgtcgt atcagcgcct tcctctccct ggtgagggag 6000
   gtggcgggca gcgaccagag ggccgtggtg gacacccgta acaccgtgcc caccggggcg 6060
   ggcctggcgt cctccgccag cgggttcgcc gccctcgccg tcgcggccgc ggccgcctac 6120
   gggctcgaac tcgacgaccg tgggctgtcc aggctggcca gacgtggatc cggctccgcc 6180
   tctcggtcta tcttcggcgg cttcgccgta tggcatgccg gccccgacgg cacggccacg 6240
   gaagcggacc tcggctccta cgccgagcca gtgcccgcgg ccgacctcga ccctgcgctg 6300
   gttatcgccg tggtaaacgc cggccccaag cccgtatcca gccgtgaggc catgcgtcgc 6360
   accgtagaca cctcaccact gtacaggcca tgggccgact ccagtaagga cgacctggac 6420
   gagatgcgtt ctgcgctgct gcgtggcgac ctcgaggccg tgggcgagat cgcggagcgt 6480
   aacgcgctcg gcatgcatgc caccatgctg gccgcccgtc ccgcggtgag gtacctgtca 6540
   ccagccacgg taaccgtgct cgacagcgtg ctccagctcc gtaaggacgg tgttctggcc 6600
   tacgcgacca tggacgccgg tcccaacgtg aaggtgctgt gcaggagggc ggacgccgag 6660
   agggtggccg acgttgtacg cgccgccgcg tccggcggtc aggtactcgt agccgggcct 6720
   ggagacggtg cccgtctgct gagcgagggc gcataataat tttaaaatat aagtgattta 6780
   gatattcata atatatttgg gaggtaaatt aatatgacga caggtcagcg tacgatcgtc 6840
   aggcatgcgc ctggcaagct gttcgtagcg ggcgagtacg cggtagtgga tcctggcaac 6900
   cctgcgatcc tggtagcggt agacaggcat atcagcgtaa ccgtgtccga cgccgacgcg 6960
   gacaccgggg ccgccgacgt agtgatctcc tccgacctcg gtcctcaggc ggtaggctgg 7020
   cgttggcatg acggcaggct cgtagtacgt gaccctgacg acgggcagca ggcgcgtagc 7080
   gccctggccc atgtggtgtc ggcgatcgag accgtgggca ggctgctggg cgaacgtgga 7140
   cagaaggtac ccgctctcac cctctccgtt agcagccgtc tgcatgagga cggcaggaag 7200
   ttcggcctgg gctccagcgg cgcggtgacc gtggcgaccg tagccgccgt agccgcgttc 7260
   tgcggactcg aactgtccac cgacgaaagg ttcaggctgg ccatgctcgc caccgcggaa 7320
   ctcgacccca agggctccgg cggggacctc gccgccagca cctggggcgg ctggatcgcc 7380
   taccaggcgc ccgacagggc ctttgtgctc gacctggcca ggcgtgtggg agtagacagg 7440
   acactgaagg cgccctggcc ggggcattct gtgcgtagac tgcctgcgcc caagggcctc 7500
   accctggagg tcggctggac cggagagccc gcctccaccg cgtccctggt gtccgatctg 7560
   catcgtcgta cctggagggg cagcgcctcc catcagaggt tcgtagagac cacgaccgac 7620
   tgtgtacgtt ccgcggttac cgccctggag tccggcgacg acacgagcct gctgcatgag 7680
   atccgcaggg cccgtcagga gctggcccgt ctggacgacg aggtaggcct cggcatcttc 7740
   acacccaagc tgacggcgct gtgcgacgcc gccgaagccg ttggcggcgc ggccaagccc 7800
   tccggggcag gcggcggcga ctgcggcatc gccctgctgg acgccgaggc gtctagggac 7860
   atcacacatg taaggcaacg gtgggagaca gccggggtgc tgcccctgcc cctgactcct 7920
   gccctggaag ggatctaata attttaaaat ataagtgatt tagatattca taatatattt 7980
   gggaggtaaa ttaatatgag cctcgattcc agactgcccg ctttccgtaa cctgtcccct 8040
   gccgcgagac tggaccacat cggccagttg ctcggcctga gccacgacga tgtcagcctg 8100
   ctggccaacg ccggtgccct gccgatggac atcgccaacg gcatgatcga aaacgtcatc 8160
   ggcaccttcg agctgcccta tgccgtggcc agcaacttcc agatcaatgg ccgtgatgtg 8220
   ctggtgccgc tggtggtgga agagccctcg atcgtcgccg ctgcttctta catggccaag 8280
   ctggcccgtg ccaacggcgg cttcaccacc tccagcagcg ccccgctgat gcatgcccag 8340
   gtacagatcg tcggcataca ggacccgctc aatgcacgtc tgagcctgct gagaagaaaa 8400
   gacgaaatca ttgaactggc caaccgtaag gaccagttgc tcaacagcct cggcggcggc 8460
   tgcagagaca tcgaagtgca caccttcgcc gataccccgc gtggcccgat gctggtggcg 8520
   cacctgatcg tcgatgtaag agatgccatg ggcgccaaca ccgtcaatac catggccgag 8580
   gccgttgcgc cgctgatgga agccatcacc gggggccagg tacgtctgag aattctgtcc 8640
   aacctggccg acctgcgcct ggccagggcc caggtgagga ttactccgca gcaactggaa 8700
   acggccgaat tcagtggcga ggcagtgatc gaaggcatcc tcgacgccta cgccttcgct 8760
   gcggtcgacc cttacagagc ggccacccac aacaagggca tcatgaatgg catcgaccca 8820
   ctgatcgtcg ccactggcaa cgactggcgt gcagtggaag ccggcgccca tgcgtatgcc 8880
   tgcagaagtg gtcactacgg ctcgctgacc acctgggaaa aggacaacaa cggccatttg 8940
   gtcggcaccc tggaaatgcc gatgcccgta ggcctggtcg gcggcgccac caaaacccat 9000
   ccgctggcgc aactgtcact gagaatcctc ggcgtgaaaa cagcccaggc gctcgctgag 9060
   attgccgtgg ccgtaggcct ggcgcaaaac ctcggggcca tgagagccct ggccaccgaa 9120
   ggcatccagc gtggccacat ggccctgcat gcgagaaata ttgccgtggt ggcgggcgcc 9180
   cgaggcgatg aggtggactg ggttgcccgg cagttggtgg aataccacga cgtgagagcc 9240
   gacagagccg tagcactgct gaaacaaaag agaggccaat gatagtttta aaatataagt 9300
   gatttagata ttcataatat atttgggagg taaattaata tgtccatctc cataggcatt 9360
   cacgacctgt ctttcgccac aaccgagttc gtactgcctc atacggcgct cgccgagtac 9420
   aacggcaccg agatcggcaa gtaccatgta ggcatcggcc agcagtctat gagcgtgcct 9480
   gccgccgacg aggacatcgt gaccatggcc gcgaccgcgg cgaggcccat catcgagcgt 9540
   aacggcaaga gcaggatccg tacggtagtg ttcgccacgg agtcttctat cgaccaggcg 9600
   aaggcgggcg gcgtatacgt gcactccctg ctggggctgg agtctgcctg cagggtagta 9660
   gagctgaagc aggcctgcta cggggccacc gccgcccttc agttcgccat cggcctggtg 9720
   aggcgtgacc ccgcccagca ggtactggta atcgccagtg acgtatccaa gtacgagctg 9780
   gacagccccg gcgaggcgac ccagggcgcg gccgcggtgg ccatgctggt aggcgccgac 9840
   cctgccctgc tgcgtatcga ggagccttcg ggcctgttca ccgccgacgt aatggacttc 9900
   tggcggccca actacctcac caccgctctg gtagacggcc aggagtccat caacgcctac 9960
   ctgcaggccg tagagggcgc ctggaaggac tacgcggagc aggacggcag gtcactggag 10020
   gagttcgcgg cgttcgtata ccaccagccg ttcacgaaga tggcctacaa ggcgcatcgc 10080
   catctgctga acttcaacgg ctacgacacc gacaaggacg ccatcgaggg cgccctcggc 10140
   cagacgacgg cgtacaacaa cgtaatcggc aacagctaca ccgcgtctgt gtacctgggc 10200
   ctggccgccc tgctcgacca ggcggacgac ctgacgggcc gttccatcgg cttcctgagc 10260
   tacggctctg gcagcgtagc cgagttcttc tctggcaccg tagtagccgg gtaccgtgag 10320
   cgtctgcgta ccgaggcgaa ccaggaggcg atcgccaggc gtaagagcgt agactacgcc 10380
   acctaccgtg agctgcacga gtacacgctc ccgtccgacg gcggcgacca tgccacccct 10440
   gtgcagacca ccggcccctt caggctggcc gggatcaacg accataagcg tatctacgag 10500
   gcgcgttaat aatttaaaag caaatataaa tgaaaaattg aaccctagca ttatgtaaat 10560
   gcagggttta atttttatat taagcagcat aatagaaagt tttttaaatg catgtatata 10620
   tggggtattt aaagggaaat ctataatata atttaggact attccggagc atgcttggca 10680
   ctggccgtcg ttttacaacg tcgtgactgg gaaaaccctg gcgttaccca acttaatcgc 10740
   cttgcagcac atcccccttt cgccagctgg cgtaatagcg aagaggcccg caccgatcgc 10800
   ccttcccaac agttgcgcag cctgaatggc gaatggcgcc tgatgcggta ttttctcctt 10860
   acgcatctgt gcggtatttc acaccgcata tggtgcactc tcagtacaat ctgctctgat 10920
   gccgcatagt taagccagcc ccgacacccg ccaacacccg ctgacgcgcc ctgacgggct 10980
   tgtctgctcc cggcatccgc ttacagacaa gctgtgaccg tctccgggag ctgcatgtgt 11040
   cagaggtttt caccgtcatc accgaaacgc gcgagacgaa agggcctcgt gatacgccta 11100
   tttttatagg ttaatgtcat gataataatg gtttcttaga cgtcaggtgg cacttttcgg 11160
   ggaaatgtgc gcggaacccc tatttgttta tttttctaaa tacattcaaa tatgtatccg 11220
   ctcatgagac aataaccctg ataaatgctt caataatatt gaaaaaggaa gagtatgagt 11280
   attcaacatt tccgtgtcgc ccttattccc ttttttgcgg cattttgcct tcctgttttt 11340
   gctcacccag aaacgctggt gaaagtaaaa gatgctgaag atcagttggg tgcacgagtg 11400
   ggttacatcg aactggatct caacagcggt aagatccttg agagttttcg ccccgaagaa 11460
   cgttttccaa tgatgagcac ttttaaatta aaaatgaagt tttaaacttc catttttaat 11520
   ttaaattaaa aatgaagttt tatcaaaaaa atttccaata atcccactct aagccacaaa 11580
   cacgccctat aaaatcccgc tttaatccca ctttgagaca catgtaatat tactttacgc 11640
   cctagtatag tgataatttt ttacattcaa tgccacgcaa aaaaataaag gggcactata 11700
   ataaaagttc cttcggaact aactaaagta aaaaattatc tttacaacct ccccaaaaaa 11760
   aagaacaggt acaaagtacc ctataataca agcgtaaaaa aatgagggta aaaataaaaa 11820
   aataaaaaaa taaaaaaata aaaaaataaa aaaaataaaa aaataaaaaa ataaaaaaat 11880
   aaaaaaataa aaaaataaaa aaataaaaaa ataaaaaaat ataaaaataa aaaaatataa 11940
   aaataaaaaa atataaaaat aaaaaaatat aaaaataaaa aaataaaaaa atataaaaat 12000
   aaaaaaataa aaaaatataa aaatattttt tatttaaagt ttgaaaaaaa tttttttata 12060
   ttatataatc tttgaagaaa agaatataaa aaatgagcct ttataaaagc ccattttttt 12120
   tcatatacgt aatatgacgt tctaatgttt ttattggtac ttctaacatt agagtaattt 12180
   ctttattttt aaagcctttt tctttaaggg cttttatttt ttttcttaat acatttaatt 12240
   cctctttttt tgttgctttt cctttagctt ttaattgctc ttgataattt tttttacctc 12300
   taatattttc tcttctctta tattcctttt tagaaattat tattgtcata tatttttgtt 12360
   cttcttctgt aatttctaat aactctataa gagtttcatt cttatactta tattgcttat 12420
   ttttatctaa ataacatctt tcagcacttc tagttgctct tataacttct ctttcactta 12480
   aatgttgtct aaacatacta ttaagttcta aaacatcatt taatgccttc tcaatgtctt 12540
   ctgtaaagct acaaagataa tatctatata aaaataatat aagctctctg tgtcctttta 12600
   aatcatattc tcttagttca caaagtttta ttatgtcttg tattcttcca taatataaac 12660
   ttctttctct ataaatataa tttattttgc ttggtctacc ctttttcctt tcatatggtt 12720
   ttaattcagg taaaaatcca ttttgtattt ctcttaagtc ataaatatat tcgtactcat 12780
   ctaatatatt gactactgtt tttgatttag agtttatact tcctggaact cttaatattc 12840
   tggttgcatc taaggcttgt ctatctgctc caaagtattt taattgatta tataaatatt 12900
   cttgaaccgc tttccataat ggtaatgctt tactaggtac tgcatttatt atccatatta 12960
   aatacattcc tcttccacta tctattacat agtttggtat aggaatactt tgattaaaat 13020
   aattcttttc taagtccatt aatacctggt ctttagtttt gccagtttta taataatcca 13080
   agtctataaa cagtgtattt aactctttta tattttctaa tcgcctacac ggcttataaa 13140
   aggtatttag agttatatag atattttcat cactcatatc taaatctttt aattcagcgt 13200
   atttatagtg ccattggcta tatccttttt tatctataac gctcctggtt atccaccctt 13260
   tacttctact atgaatatta tctatatagt tctttttatt cagctttaat gcgtttctca 13320
   cttattcacc tccccttctg taaaactaag aaaattatat catattttca ataattatta 13380
   actattctta aactcttaat aaaaaataga gtaagtcccc aattgaaact taatctattt 13440
   tttatgtttt aatttattat ttttattaaa atattttaaa ctaaattaaa tgattctttt 13500
   taatttttta ctatttcatt ccataatata ttactataat tatttacaaa taatatttct 13560
   tcatttgtaa tatttagatg atttactaat tttagttttt atatattaaa taattaatgt 13620
   ataatttata taaaaaatca aaggagctta taaattatga ttatttccaa agatactaaa 13680
   gatttaattt tttcaatttt aacaatactt tttgtaatat tatgtttaaa tttaattgta 13740
   tttttttcat ataataaagc cgttgaagta aaccaatcca ttttccttat gatgttatta 13800
   ttaaatttaa gttttataat aatatcttta ttatatttat tgtttttaaa aaaactagtg 13860
   aaatttccgg ctttattaaa cttattttta ggaattttat tttcattttc atctttacag 13920
   gatttgatta tatctttaaa tatgttttat caaatattat ctttttctaa atttatatat 13980
   atttttatta tatttattat tatatatatt ttatttttaa gtttctttct aacagctatt 14040
   aaaaagaaac ttaaaaataa aaacacgtac tctaaaccaa taaataaaac tatttttatt 14100
   attgctgcct tgattggaat agtttttagt aaaattaatt tcaatattcc acaatattat 14160
   attataagct agctttgcat tgtacttttc aatcgcttca cgaatgcggt tatctccgaa 14220
   agataaagtc ttttcatctt ccttgatgaa gataagattt tctccgtctc cgccggcaga 14280
   attgaagcgg ggtactacgg tatcgtctgc gtcatcttcc gttgtctgat agatgatagt 14340
   cataggctca ttttcttccg tttcggtaaa ggggataggt tcgccctttg agagcagggc 14400
   ggcgatggaa agcattaact tgcttttccc atcgcccgga tctccctgca atagcgtaac 14460
   tttgccaaac ggaatatacg gataccacag ccactttact tctttcggct cgatttcact 14520
   tgccttgatg atttcaagag gtacgctgaa attcatttcg ttttcattta gtttcatttt 14580
   ttcttgttct ccttttctct gaaaatataa aaaccacaga ttgatactaa aaccttggtt 14640
   gtgttgcttt tcggggctta aatcaaggaa aaatccttgt tttaagcctt tcaaaaagaa 14700
   acacaaggtc tttgtactaa cctgtggtta tgtataaaat tgtagatttt agggtaacaa 14760
   aaaacaccgt atttctacga tgtttttgct taaatacttg tttttagtta cagacaaacc 14820
   tgaagttatc atagtcctaa attatattat agatttccct ttaaataccc catatataca 14880
   tgcatttaaa aaactttcta ttatgctgct taatataaaa attaaaccct gcatttacat 14940
   aatgctaggg ttcaattttt catttatatt tgcttttaaa ttataaaagc cagtcattag 15000
   gcctatctga caattcctga atagagttca taaacaatcc tgcatgataa ccatcacaaa 15060
   cagaatgatg tacctgtaaa gatagcggta aatatattga attaccttta ttaatgaatt 15120
   ttcctgctgt aataatgggt agaaggtaat tactattatt attgatattt aagttaaacc 15180
   cagtaaatga agtccatgga ataatagaaa gagaaaaagc attttcaggt ataggtgttt 15240
   tgggaaacaa tttccccgaa ccattatatt tctctacatc agaaaggtat aaatcataaa 15300
   actctttgaa gtcattcttt acaggagtcc aaataccaga gaatgtttta gatacaccat 15360
   caaaaattgt ataaagtggc tctaacttat cccaataacc taactctccg tcgctattgt 15420
   aaccagttct aaaagctgta tttgagttta tcacccttgt cactaagaaa ataaatgcag 15480
   ggtaaaattt atatccttct tgttttatgt ttctgtataa aacactaata tcaatttctg 15540
   tggttatact aaaagtcgtt tgttggttca aataatgatt aaatatctct tttctcttcc 15600
   aattgtctaa atcaatttta ttaaagttca tgttcatttc ctccctttaa atttaacaca 15660
   aaattacaca cacttatact ataatccttt ttagttgtat ttttcaataa aaatcattca 15720
   aaaatataac ttttgataag aaatttcaca aattaaagta tcaaaaaatt ttgctagtca 15780
   atactttact caatattata taatgtaaat caaataagca aaaatttaat ctgaagatgc 15840
   ttagtgggaa tttgtacccc ttatcgatac aaattccccg taggcgctag ggacactttt 15900
   tcactcgtta aaaagttttg agaatatttt atatttttgt tcatgtaatc actccttctt 15960
   aattacaaat ttttagcatc taatttaact tcaattccta ttatacaaaa ttttaagata 16020
   ctgcactatc aacacactct taagtttgct tctaagtctt atttccataa cttcttttac 16080
   gtttccgggt acaattcgta atcatgtcat agctgtttcc tgtgtgaaat tcttatccgc 16140
   tcacaattcc acacaacata cgagccggaa gcataaagtg taaagcctgg ggtgcctaat 16200
   gagtgagcta actcacatta attgcgttgc gctcactgcc cgctttccag tcgggaaacc 16260
   tgtcgtgcca gaaaacttca tttttaattt aaaaggatct aggtgaagat cctttttgat 16320
   aatctcatga ccaaaatccc ttaacgtgag ttttcgttcc actgagcgtc agaccccgta 16380
   gaaaagatca aaggatcttc ttgagatcct ttttttctgc gcgtaatctg ctgcttgcaa 16440
   acaaaaaaac caccgctacc agcggtggtt tgtttgccgg atcaagagct accaactctt 16500
   tttccgaagg taactggctt cagcagagcg cagataccaa atactgtcct tctagtgtag 16560
   ccgtagttag gccaccactt caagaactct gtagcaccgc ctacatacct cgctctgcta 16620
   atcctgttac cagtggctgc tgccagtggc gataagtcgt gtcttaccgg gttggactca 16680
   agacgatagt taccggataa ggcgcagcgg tcgggctgaa cggggggttc gtgcacacag 16740
   cccagcttgg agcgaacgac ctacaccgaa ctgagatacc tacagcgtga gctatgagaa 16800
   agcgccacgc ttcccgaagg gagaaaggcg gacaggtatc cggtaagcgg cagggtcgga 16860
   acaggagagc gcacgaggga gcttccaggg ggaaacgcct ggtatcttta tagtcctgtc 16920
   gggtttcgcc acctctgact tgagcgtcga tttttgtgat gctcgtcagg ggggcggagc 16980
   ctatggaaaa acgccagcaa cgcggccttt ttacggttcc tggccttttg ctggcctttt 17040
   gctcacatgt tctttcctgc gttatcccct gattctgtgg ataaccgtat taccgccttt 17100
   gagtgagctg ataccgctcg ccgcagccga acgaccgagc gcagcgagtc agtgagcgag 17160
   gaagcggaag agcgcccaat acgcaaaccg cctctccccg cgcgttggcc gattcattaa 17220
   tgcagctggc acgacaggtt tcccgactgg aaagcgggca gtgagcgcaa cgcaattaat 17280
   gtgagttagc tcactcatta ggcaccccag gctttacact ttatgcttcc ggctcgtatg 17340
   ttgtgtggaa ttgtgagcgg ataacaattt cacacaggaa acagctatga ccatgattac 17400

## Description

### TECHNICAL FIELD

The present invention relates to a recombinant cell capable of producing an organic compound having 4 or more carbon atoms from specific C1 compounds such as carbon monoxide, a method for manufacturing the same, and a method for producing the organic compound having 4 or more carbon atoms using the recombinant cell.

### BACKGROUND ART

Syngas (synthesis gas) is a mixed gas mainly containing carbon monoxide, carbon dioxide, and hydrogen, which is efficiently obtained from waste, natural gas and coal by action of a metal catalyst under high temperature and high pressure. In the field of C1 chemistry by metal catalysts starting from syngas, a process for mass production of liquid chemicals such as methanol, formic acid and formaldehyde at a low cost has been developed.

Carbon monoxide and carbon dioxide are contained in syngas derived from waste, industrial exhaust gas, or syngas derived from natural gas, and are available almost permanently. However, at present, there are very few examples of producing chemicals by microorganisms from these carbon sources. Only production of ethanol, 2,3-butanediol or the like, is currently under development. In particular, there are few reports about utilization of syngas assimilating substance by a recombinant. Patent Document 1 discloses a production technique of isopropanol by a recombinant of *Escherichia coli.* In this technique, a plurality of CO metabolic enzyme genes are introduced into *E. coli* to impart syngas assimilating ability, and isopropanol is produced from syngas.

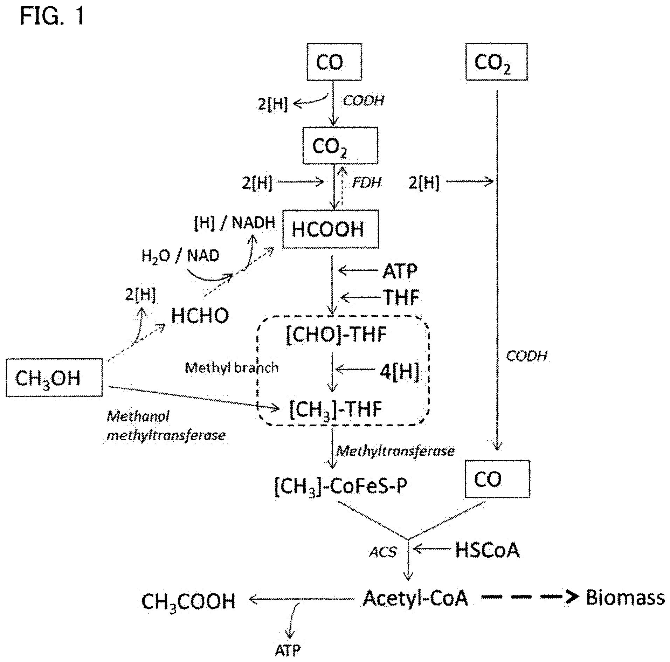

Well-known examples of a *Clostridium* bacterium and a *Moorella* bacterium include syngas assimilating bacterium capable of assimilating carbon monoxide or carbon dioxide included in the syngas. The syngas assimilating bacterium is known to have carbon monoxide dehydrogenase (CO dehydrogenase, CODH) (for example, EC 1.2.99.2/1.2.7.4) having action of generating carbon dioxide and proton from carbon monoxide and water, and its reverse reaction, that is, action of generating carbon monoxide and water from carbon dioxide and proton. The carbon monoxide dehydrogenase is one of enzymes acting in an acetyl-CoA pathway (see Fig. 1).

Acetyl-CoA synthesized in the acetyl-CoA pathway is metabolized into acetic acid, acetaldehyde, ethanol, and the like. In particular, most of excessive acetyl-CoA in the proliferation stationary phase is converted into ethanol. At this time, reduced nicotinamide adenine dinucleotide (NADH) is consumed. In the process in which acetaldehyde is produced from acetyl-CoA and ethanol is produced from acetaldehyde, NADH is consumed in any cases, and oxidized nicotinamide adenine dinucleotide (NAD) is generated. Furthermore, similarly, nicotinamide adenine dinucleotide phosphate in which a phosphate group is attached at position 2' of nucleotide of adenosine of nicotinamide adenine dinucleotide includes a reduced type (NADPH) and an oxidized type (NADP), and is known to work as a coenzyme of dehydrogenase. The balance of NAD(P)H/NAD(P) in a cell affects the growth. Non-Patent Document 1 mentions that *Clostridium acetbutylicum* cannot grow in glycerol metabolism because of excessive NADPH, and that introduction of a gene of 1,3-propanediol NADP-dependent dehydrogenase (YqhD) synthase that consumes NADPH permits growth and, at the same time, 1,3-propanediol is synthesized.

Some attempts for producing an organic compound having C4 or more from specific C1 compounds such as carbon monoxide by recombinant have been reported. Patent Documents 2 and 3 disclose an example in which an isoprene synthesis has been attempted using a *Clostridium* bacterium into which a gene of a mevalonate pathway that is an NAD(P)H consumption pathway (also referred to as an MVA pathway) in addition to an isoprene synthesis gene. However, in any cases, production amount of isoprene is very small.

Patent Document 4 discloses a recombinant cell capable of producing isoprene from a specific C1 compound such as carbon monoxide. It discloses an example in which isoprene is produced from syngas using a recombinant of a *Clostridium* bacterium.

The mevalonate pathway is a pathway to synthesize isopentenyl diphosphate (IPP) and dimethylallyl diphosphate (DMAPP), and is a starting point of synthesis of industrially applicable compounds such as terpene and steroid.

Terpene is a group of hydrocarbons having isoprene as a constituent unit. Terpene includes isoprene (the number of carbon atoms: 5), monoterpene (the number of carbon atoms: 10), sesquiterpene (the number of carbon atoms: 15), diterpene (the number of carbon atoms: 20), and triterpene (the number of carbon atoms: 30). Isoprene is a monomer raw material for synthetic polyisoprene, and is an important material, in particular, in a tire industry. As the cyclic monoterpene, β-Pinene, α-Pinene, limonene, α-Phellandrene, and the like, are considered to be a monomer raw material of adhesives or transparent resin (Non-Patent Document 3). Much attention has been paid on farnesene as one type of sesquiterpene as a raw material of tire (Patent Document 5). As triterpene, glycyrrhizic acid known as a licorice extract has been known.

### PRIOR ART DOCUMENTS

### PATENT DOCUMENTS

Patent Document 1: WO2009/094485
Patent Document 2: WO2013/181647
Patent Document 3: WO2013/180584
Patent Document 4: WO2014/065271
Patent Document 5: WO2013/047348

### NON-PATENT DOCUMENTS

Non-Patent Document 1: Tang X, Tan Y, Zhu H, Zhao K, Shen W., Appl Environ Microbiol. 2009 Mar; 75(6): 1628-34. doi: 10.1128/AEM.02376-08. Epub 2009 Jan 9.
Non-Patent Document 2: Kiriukhin M, Tyurin M., Bioprocess Biosyst Eng. 2014 Feb; 37(2): 245-60. doi: 10.1007/s00449-013-0991-6. Epub 2013 Jun 18
Non-Patent Document 3: Schilmiller, A. L., et al., Proc Natl Acad Sci U S A., 2009.

### DISCLOSURE OF INVENTION

### TECHNICAL PROBLEM

As mentioned above, technology development for producing an organic compound such as isoprene from syngas using a recombinant has been advanced, but techniques for improving productivity are demanded. Then, an object of the present invention is to provide a series of techniques for obtaining organic compounds of C4 or more from C1 carbon source such as syngas by using a recombinant at high efficiency.

### SOLUTION TO PROBLEM

One aspect of the present invention for solving the aforementioned problem is a recombinant cell having a function of synthesizing acetyl-CoA from methyltetrahydrofolate, carbon monoxide, and CoA. The recombinant cell includes a gene that expresses an exogenous NAD(P)H consumption pathway, and the gene is expressed in the recombinant cell. Expression in at least one of endogenous NAD(P)H consumption pathways of the recombinant cell is down-regulated, and the endogenous NAD(P)H consumption pathway is different from the exogenous NAD(P)H consumption pathway. The recombinant cell produces an organic compound having 4 or more carbon atoms from at least one selected from the group consisting of carbon monoxide and carbon dioxide via the exogenous NAD(P)H consumption pathway.

This aspect relates to a recombinant cell capable of producing an organic compound having 4 or more carbon atoms. The recombinant cell of this aspect has a "function of synthesizing acetyl-CoA from methyltetrahydrofolate, carbon monoxide, and CoA". Furthermore, the recombinant cell of this aspect includes a "gene that expresses an exogenous NAD(P)H consumption pathway," and the gene is expressed in the recombinant cell. In other words, in the recombinant cell of this aspect, an NAD(P)H consumption pathway is newly added or enhanced.

Furthermore, in the recombinant cell of this aspect, expression of at least one of the endogenous NAD(P)H consumption pathways is down-regulated, and the endogenous NAD(P)H consumption pathway is different from that of the exogenous NAD(P)H consumption pathway.

The recombinant cell of this aspect produces an organic compound having 4 or more carbon atoms from at least one selected from the group consisting of carbon monoxide and carbon dioxide via the exogenous NAD(P)H consumption pathway.

In the recombinant cell of this aspect, since the expression of an endogenous NAD(P)H consumption pathway that is not involved in production of the organic compound, among the NAD(P)H consumption pathways, is down-regulated, the NAD(P)H is preferentially supplied to the exogenous NAD(P)H consumption pathway involved in production of the organic compound. Therefore, production of the organic compound is efficiently carried out. For example, by culturing the recombinant cell of this aspect, the organic compound having 4 or more carbon atoms can be mass-produced.

Examples of the cell having the "function of synthesizing acetyl-CoA from methyltetrahydrofolate, carbon monoxide, and CoA" in this aspect include anaerobic microorganisms having an acetyl-CoA pathway (Wood-Ljungdahl pathway) and a methanol pathway shown in Fig. 1.

The NAD(P)H represents NADH or NADPH. Similarly, the NAD(P) represents NAD or NADP.

The NAD(P)H consumption pathway means a pathway involving conversion of NAD(P)H to NAD(P), and in which NAD(P)H is consumed. The exogenous NAD(P)H consumption pathway means an NAD(P)H consumption pathway that is introduced into the host cell from the outside. The endogenous NAD(P)H consumption pathway represents an NAD(P)H consumption pathway which the host cell originally has.

The down-regulation of gene expression means regulation for reducing an expression amount of a gene, and regulation for impairing a function of a gene. Examples of the down-regulation of gene expression include a gene knockout and a gene knockdown. Furthermore, the gene knockout includes deletion of a gene itself.

Preferably, the recombinant cell is a *Clostridium* bacterium or a *Moorella* bacterium.

Preferably, the recombinant cell is a *Clostridium ljungdahlii, Clostridium autoethanogenum, Clostridium carboxidivorans, Clostridium ragsdalei, Clostridium kluyveri,* or *Moorella thermoacetica.*

Preferably, the exogenous NAD(P)H consumption pathway is a mevalonate pathway.

With such a configuration, a synthesis ability of isopentenyl diphosphate (IPP) and dimethylallyl diphosphate (DMAPP) is improved.

Preferably, the mevalonate pathway is a mevalonate pathway of yeast, prokaryote, or actinomycete.

Preferably, the mevalonate pathway is a mevalonate pathway of actinomycete.

Preferably, HMG-CoA reductase of the mevalonate pathway includes NADH-dependent HMG-CoA reductase.

Preferably, the HMG-CoA reductase is mvaA (P13702) derived from *Pseudomonas mevalonii,* hmgA-1Mtc_0274 (H8I942) derived from *Methanocella conradii,* mvaA LLKF_1694 (D2BKK7) derived from *Lactococcus lactis subsp. lactis* (strain KF147), or mvaA SSA_0337 (A3CKT9) derived from *Streptococcus sanguinis* (strain SK36).

Preferably, in the endogenous NAD(P)H consumption pathway, expression of at least one selected from the group consisting of ethanol dehydrogenase, acetaldehyde dehydrogenase, lactate dehydrogenase, and 2,3-butanediol dehydrogenase is down-regulated.

Examples of the endogenous NAD(P)H consumption pathway in *Clostridium* bacterium and the like include pathways for carrying out conversion from acetaldehyde to ethanol, conversion from acetyl-CoA to acetaldehyde, conversion from pyruvate to lactate, and conversion from pyruvate to 2,3-butanediol, respectively. Then, in this aspect, expression of enzymes that work in these endogenous NAD(P)H consumption pathways are down-regulated.

Preferably, in the endogenous NAD(P)H consumption pathway, expression of at least ethanol dehydrogenase is down-regulated.

Preferably, in the endogenous NAD(P)H consumption pathway, expression of acetaldehyde dehydrogenase is further down-regulated.

Preferably, in the endogenous NAD(P)H consumption pathway, expression of lactate dehydrogenase or 2,3-butanediol dehydrogenase is further down-regulated.

Preferably, at least one of the down-regulations is a lack of expression.

Preferably, the expression of ethanol dehydrogenase and/or acetaldehyde dehydrogenase lacks.

Preferably, a gene that expresses the mevalonate pathway is incorporated in a genome instead of a gene encoding ethanol dehydrogenase and/or acetaldehyde dehydrogenase, by homologous recombination.

Preferably, the expression of phosphotransacetylase and/or acetate kinase is down-regulated.

Phosphotransacetylase and acetate kinase are enzymes acting on pathway for converting acetyl-CoA to acetic acid. According to this aspect, wasteful consumption of acetyl-CoA is suppressed.

Preferably, expression of at least phosphotransacetylase is down-regulated.

Preferably, at least one of the down-regulations is carried out by gene deletion or modification of a gene expression-regulating region.

Preferably, the gene that expresses an exogenous NAD(P)H consumption pathway is a gene cluster that includes a gene that expresses a mevalonate pathway and a gene encoding an enzyme for producing isoprene from isopentenyl diphosphate, and the organic compound is isoprene.

With such a configuration, a recombinant cell capable of mass-producing isoprene is provided.

Preferably, an enzyme for producing isoprene from the isopentenyl diphosphate is isoprene synthase.

Preferably, the gene that expresses an exogenous NAD(P)H consumption pathway is a gene cluster that includes a gene that expresses a mevalonate pathway and a gene encoding an enzyme for producing cyclic terpene from isopentenyl diphosphate, and the organic compound is cyclic terpene.

With such a configuration, a recombinant cell capable of mass-producing cyclic terpene is provided.

Preferably, the enzyme for producing cyclic terpene from isopentenyl diphosphate is geranyl diphosphate synthase and/or neryl diphosphate synthase, and cyclic monoterpene synthase, and the organic compound is cyclic monoterpene.

Preferably, the cyclic monoterpene synthase is β-phellandrene synthase, and the cyclic monoterpene is β-phellandrene, 4-carene, or limonene.

According to this aspect, by the action of β-phellandrene synthase expressed in the cell, β-phellandrene, 4-carene, or limonene is synthesized from geranyl diphosphate (GPP) and/or neryl diphosphate (NPP). As a result, by culturing the recombinant cell of this aspect, β-phellandrene, 4-carene, or limonene can be mass-produced.

Preferably, the gene cluster is incorporated in a genome of the recombinant cell.

Preferably, a part or whole of an adhE1 gene and an adhE2 gene of a host cell that is a basis of the recombinant cell is deleted, and the gene cluster is incorporated in a genome, instead of the adhE1 gene and the adhE2 gene, by homologous recombination.

The adhE1 gene and the adhE2 gene are included in a *Clostridium* bacterium and the like, and are considered to include both an acetaldehyde dehydrogenase gene and an ethanol dehydrogenase gene.

Another aspect of the present invention relates to a method for manufacturing a recombinant cell. The method includes a first step of providing a host cell having a function of synthesizing acetyl-CoA from methyltetrahydrofolate, carbon monoxide, and CoA; and a second step of introducing a gene that expresses an exogenous NAD(P)H consumption pathway into the host cell. The gene that has been introduced in the second step is expressed in the host cell. Expression in at least one of endogenous NAD(P)H consumption pathways of the host cell is down-regulated, and the endogenous NAD(P)H consumption pathway is different from the exogenous NAD(P)H consumption pathway. The recombinant cell produces the organic compound having 4 or more carbon atoms from at least one selected from the group consisting of carbon monoxide and carbon dioxide, via the exogenous NAD(P)H consumption pathway.

This aspect relates to a method for manufacturing a recombinant cell capable of producing an organic compound having 4 or more carbon atoms. The method of this aspect includes a first step of providing a host cell having a function of synthesizing acetyl-CoA from methyltetrahydrofolate, carbon monoxide, and CoA; and a second step of introducing gene that expresses an exogenous NAD(P)H consumption pathway into the host cell. Then, the gene that has been introduced in the second step is expressed in the host cell. In other words, in the recombinant cell manufactured by the method of this aspect, an NAD(P)H consumption pathway is newly added or enhanced in the host cell.

Furthermore, in the recombinant cell manufactured by the method of this aspect, expression in at least one of the endogenous NAD(P)H consumption pathways of the host cell is down-regulated, and the endogenous NAD(P)H consumption pathway is different from the exogenous NAD(P)H consumption pathway.

The recombinant cell manufactured by the method of this aspect produces the organic compound having 4 or more carbon atoms from at least one selected from the group consisting of carbon monoxide and carbon dioxide, via the exogenous NAD(P)H consumption pathway.

In the recombinant cell manufactured by the method of this aspect, since the expression of an endogenous NAD(P)H consumption pathway that is not involved in production of the organic compound, among the NAD(P)H consumption pathways, is down-regulated, NAD(P)H is preferentially supplied to the exogenous NAD(P)H pathway that is involved in the organic compound. Therefore, production of the organic compound is carried out efficiently. For example, by culturing the recombinant cell of this aspect, the organic compound having 4 or more carbon atoms can be mass-produced.

Preferably, the host cell is a *Clostridium* bacterium or a *Moorella* bacterium.

Preferably, the host cell is *Clostridium ljungdahlii, Clostridium autoethanogenum, Clostridium carboxidivorans, Clostridium ragsdalei, Clostridium kluyveri,* or *Moorella thermoacetica.*

Preferably, the exogenous NAD(P)H consumption pathway is a mevalonate pathway.

Preferably, the mevalonate pathway is a mevalonate pathway of yeast, prokaryote, or actinomycete.

Preferably, the mevalonate pathway is a mevalonate pathway of actinomycete.

Preferably, HMG-CoA reductase of the mevalonate pathway includes NADH-dependent HMG-CoA reductase.

Preferably, the HMG-CoA reductase is mvaA (P13702) derived from *Pseudomonas mevalonii,* hmgA-1Mtc_0274 (H8I942) derived from *Methanocella conradii,* mvaA LLKF_1694 (D2BKK7) derived from *Lactococcus lactis subsp. lactis* (strain KF147), or mvaA SSA_0337 (A3CKT9) derived from *Streptococcus sanguinis* (strain SK36).

Preferably, in the endogenous NAD(P)H consumption pathway, expression of at least one selected from the group consisting of ethanol dehydrogenase, acetaldehyde dehydrogenase, lactate dehydrogenase, and 2,3-butanediol dehydrogenase is down-regulated.

Preferably, in the endogenous NAD(P)H consumption pathway, expression of at least ethanol dehydrogenase is down-regulated.

Preferably, in the endogenous NAD(P)H consumption pathway, expression of acetaldehyde dehydrogenase is further down-regulated.

Preferably, in the endogenous NAD(P)H consumption pathway, expression of lactate dehydrogenase or 2,3-butanediol dehydrogenase is further down-regulated.

Preferably, at least one of the down-regulations is a lack of expression.

Preferably, expression of ethanol dehydrogenase and/or acetaldehyde dehydrogenase lacks.

Preferably, a gene that expresses the mevalonate pathway is incorporated in a genome of the recombinant cell instead of a gene encoding ethanol dehydrogenase and/or acetaldehyde dehydrogenase, by homologous recombination.

Preferably, the expression of phosphotransacetylase and/or acetate kinase is further down-regulated.

Preferably, expression of at least phosphotransacetylase is down-regulated.

Preferably, at least one of the down-regulations is carried out by gene deletion or modification of a gene expression-regulating region.

Preferably, the gene that has been introduced in the second step is a gene cluster that includes a gene that expresses a mevalonate pathway and a gene encoding an enzyme for producing isoprene from isopentenyl diphosphate, and the organic compound is isoprene.

Preferably, an enzyme for producing isoprene from the isopentenyl diphosphate is isoprene synthase.

Preferably, the gene that has been introduced in the second step is a gene cluster that includes a gene that expresses a mevalonate pathway and a gene encoding an enzyme for producing cyclic terpene from isopentenyl diphosphate, and the organic compound is cyclic terpene.

Preferably, the enzyme for producing cyclic terpene from the isopentenyl diphosphate is geranyl diphosphate synthase and/or neryl diphosphate synthase, and cyclic monoterpene synthase, and the organic compound is cyclic monoterpene.

Preferably, the cyclic monoterpene synthase is β-phellandrene synthase, and the cyclic monoterpene is β-phellandrene, 4-carene, or limonene.

Preferably, the gene cluster is incorporated in a genome of a host cell.

Preferably, a part or whole of an adhE1 gene and an adhE2 gene of a host cell is deleted, and the gene cluster is incorporated in a genome, instead of the adhE1 gene and the adhE2 gene, by homologous recombination.

Another aspect of the present invention is a method for producing an organic compound. The method includes bringing at least one C1 compound selected from the group consisting of carbon monoxide and carbon dioxide into contact with the above-mentioned recombinant cell or a recombinant cell manufactured by the above-mentioned method to produce an organic compound having 4 or more carbon atoms from the C1 compound in the recombinant cell.

This aspect relates to a method for producing an organic compound having 4 or more carbon atoms. In this aspect, at least one C1 compound selected from the group consisting of carbon monoxide and carbon dioxide is brought into contact with the above-mentioned recombinant cell or the recombinant cell manufactured by the above-mentioned to produce the organic compound from the C1 compound. According to the method of this aspect, for example, it is possible to produce the organic compound from syngas including carbon monoxide or carbon dioxide.

Preferably, the recombinant cell is cultured using at least one C1 compound selected from the group consisting of carbon monoxide and carbon dioxide as a carbon source to produce an organic compound having 4 or more carbon atoms in the recombinant cell.

Preferably, the recombinant cell is provided with a gas mainly containing carbon monoxide, gas mainly containing carbon monoxide and hydrogen, gas mainly containing carbon dioxide and hydrogen, or gas mainly containing carbon monoxide, carbon dioxide and hydrogen.

The wording "provide a recombinant cell with a gas" means bringing the gas into contact with the recombinant cell. Examples thereof include providing the recombinant cell with gas as carbon source and the like when the recombinant cell is cultured.

Preferably, formic acid or methanol is further provided to the recombinant cell.

The wording "provide a recombinant cell with a gas and methanol" means bringing a gas and methanol into contact with a recombinant cell. Examples thereof include providing a recombinant cell with a gas and methanol as carbon source and the like in culturing the recombinant cell.

Preferably, the recombinant cell is a *Clostridium* bacterium or a *Moorella* bacterium.

Preferably, the organic compound released to outside the recombinant cell is collected.

Preferably, the organic compound is collected from a gas phase of a culture system of the recombinant cell.

### EFFECT OF INVENTION

The present invention permits production of an organic compound having 4 or more carbon atoms by a recombinant via an exogenous NAD(P)H consumption pathway with high efficiency. In particular, with a configuration in which a mevalonate pathway is introduced as the exogenous NAD(P)H consumption pathway, isoprene or cyclic monoterpene can be produced by a recombinant with high efficiency.

### BRIEF DESCRIPTION OF DRAWINGS

Fig. 1 is an explanatory diagram showing an acetyl-CoA pathway and a methanol pathway.
Fig. 2 is an explanatory diagram showing a part of central metabolic pathway in a *Clostridium* bacterium or a *Moorella* bacterium in which a mevalonate pathway has been introduced.

### BEST MODE FOR CARRYING OUT THE INVENTION

Hereinafter, the exemplary embodiment of the present invention will be described. Note here that in the present invention, all the terms "gene" can be replaced with terms "nucleic acid" or "DNA".

A recombinant cell of the present invention has a function of synthesizing acetyl-CoA from methyltetrahydrofolate, carbon monoxide, and CoA. The recombinant cell includes a gene that expresses an exogenous NAD(P)H consumption pathway. The gene is expressed in the recombinant cell. Expression in at least one of endogenous NAD(P)H consumption pathways of the recombinant cell is down-regulated, and the endogenous NAD(P)H consumption pathway is different from the exogenous NAD(P)H consumption pathway. The recombinant cell produces an organic compound having 4 or more carbon atoms from at least one selected from the group consisting of carbon monoxide and carbon dioxide via the exogenous NAD(P)H consumption pathway.

For example, the recombinant cell of the present invention is prepared by introducing a gene that expresses an exogenous NAD(P)H consumption pathway into a host cell having a function of synthesizing acetyl-CoA from methyltetrahydrofolate, carbon monoxide, and CoA, and the gene is expressed in the host cell. In at least one endogenous NAD(P)H consumption pathway of the host cell, the expression is down-regulated. The endogenous NAD(P)H consumption pathway is different from the exogenous NAD(P)H consumption pathway. The recombinant cell produces an organic compound having 4 or more carbon atoms from at least one selected from the group consisting of carbon monoxide and carbon dioxide via the exogenous NAD(P)H consumption pathway.

A method for manufacturing a recombinant cell of the present invention includes a first step of providing a host cell having a function of synthesizing acetyl-CoA from methyltetrahydrofolate, carbon monoxide, and CoA, and a second step of introducing a gene that expresses an exogenous NAD(P)H consumption pathway into the host cell. The gene that has been introduced in the second step is expressed in the host cell. In at least one endogenous NAD(P)H consumption pathway of the host cell, the expression is down-regulated, and the endogenous NAD(P)H consumption pathway is different from the exogenous NAD(P)H consumption pathway. The recombinant cell is capable of producing an organic compound having 4 or more carbon atoms from at least one selected from the group consisting of carbon monoxide and carbon dioxide via the exogenous NAD(P)H consumption pathway.

The recombinant cell of the present invention has a function of synthesizing acetyl-CoA from methyltetrahydrofolate, carbon monoxide, and CoA. For example, a host cell that is a basis of the recombinant cell of the present invention has a function of synthesizing acetyl-CoA from methyltetrahydrofolate, carbon monoxide, and CoA. A pathway for synthesizing acetyl-CoA from methyltetrahydrofolate ([CH3]-THF), carbon monoxide (CO), and CoA is included in, for example, an acetyl-CoA pathway (Wood-Ljungdahl pathway) and a methanol pathway (Methanol pathway) shown in Fig. 1.

As shown in Fig. 1, in the acetyl-CoA pathway, carbon dioxide (CO₂) is reduced into carbon monoxide (CO) and a methyl cation source in two pathways, separately. Then, a thiol group of CoA (described as HSCoA in Fig. 1) is acetylated using these two carbon sources as a substrate, and one molecule of acetyl-CoA is synthesized. Enzymes such as an acetyl-CoA synthase (ACS), a methyltransferase, a carbon monoxide dehydrogenase (CODH), a formate dehydrogenase (FDH) act in the acetyl-CoA pathway. Note here that the pathway from formyltetrahydrofolate ([CHO]-THF) to [CH₃]-THF is referred to as methyl branch.

On the other hand, the methanol pathway includes a pathway for converting methanol into formaldehyde (HCHO), and further into formic acid (HCOOH), and a pathway for inducing from methanol to [CH₃]-THF.

That is to say, a pathway in which acetyl-CoA is synthesized from methyltetrahydrofolate ([CH3]-THF), carbon monoxide (CO), and CoA is in common in the acetyl-CoA pathway and the methanol pathway.

Examples of the host cell include anaerobic microorganism such as a *Clostridium* bacterium and a *Moorella* bacterium. Specific examples include a *Clostridium* bacterium or a *Moorella* bacterium, such as *Clostridium ljungdahlii, Clostridium autoethanogenum, Clostridium carboxidivorans, Clostridium ragsdalei* (Kopke M. et al., Appl. Environ. Microbiol. 2011, 77(15), 5467-5475), *Clostridium kluyveri, Moorella thermoacetica* (that is the same as *Clostridium* thermoaceticum) (Pierce EG. et al., Environ. Microbiol. 2008, 10, 2550-2573). In particular, *Clostridium* bacteria are preferable as the host cell because their host-vector systems and culture methods have been established.

Other examples include *Acetobacterium* bacteria such as *Acetobacterium woodii* (Dilling S. et al., Appl. Environ. Microbiol. 2007, 73(11), 3630-3636). Furthermore, bacteria such as *Carboxydocella sporoducens* sp. Nov. (Slepova TV. et al., Inter. J. Sys. Evol. Microbiol. 2006, 56, 797-800), *Rhodopseudomonas gelatinosa* (Uffen RL, J. Bacteriol. 1983, 155(3), 956-965), *Eubacterium limosum* (Roh H. et al., J. Bacteriol. 2011, 193(1), 307-308), *Butyribacterium methylotrophicum* (Lynd, LH. et al., J. Bacteriol. 1983, 153(3), 1415-1423) are also examples of the host cell.

The recombinant cell of the present invention may have carbon monoxide dehydrogenase (CODH). In detail, a cell that grows mainly by carbon monoxide metabolism, that is, by a function of generating carbon dioxide and proton from carbon monoxide and water by the action of carbon monoxide dehydrogenase is preferred. The anaerobic microorganism having the acetyl-CoA pathway and methanol pathway mentioned above includes carbon monoxide dehydrogenase (CODH).

All of proliferation and CODH activity of the bacteria mentioned above are oxygen sensitive. However, oxygen insensitive CODH is also known. For example, oxygen insensitive CODH exists in other bacterial species represented by *Oligotropha carboxidovorans* (Schubel, U. et al., J. Bacteriol., 1995, 2197-2203), and *Bradyrhizobium japonicum* (Lorite MJ. et al., Appl. Environ. Microbiol., 2000, 66(5), 1871-1876) (King GM et al., Appl. Environ. Microbiol. 2003, 69 (12), 7257-7265). Also in *Ralsotonia* bacteria which are aerobic hydrogen oxidizing bacteria, oxygen insensitive CODH exists (NCBI Gene ID: 4249199, 8019399).

As described above, bacteria having CODH widely exist. The host cell for use in the present invention can be appropriately selected from such bacteria. For example, using a selective medium containing CO, CO/H₂ (gas mainly containing CO and H₂), or CO/CO₂/H₂ (gas mainly containing CO, CO₂ and H₂) as the only carbon source and energy source, a bacterium having CODH that is usable as the host cell can be isolated in anaerobic, microaerobic or aerobic conditions.

As mentioned above, the NAD(P)H consumption pathway means a pathway involving conversion from NAD(P)H to NAD(P), and in which NAD(P)H is consumed. Examples of the NAD(P)H consumption pathways include a pathway in which NAD(P)H works as a coenzyme of dehydrogenase.

The recombinant cell of the present invention includes a gene that expresses an exogenous NAD(P)H consumption pathway, and the gene is expressed. For example, a gene that expresses an exogenous NAD(P)H consumption pathway is introduced into a host cell, and the gene is expressed. In a preferable embodiment, the host cell is a *Clostridium* bacterium or a *Moorella* bacterium, the exogenous NAD(P)H consumption pathway is a mevalonate pathway.

Synthesis pathways of isopentenyl diphosphate (IPP) are generally classified into a mevalonate pathway (MVA pathway) and a non-mevalonate pathway (MEP pathway). Among them, the mevalonate pathway is inherent in eukaryotes, and uses acetyl-CoA as a starting substance. Enzymes acting in the mevalonate pathway include acetyl-CoA acetyl transferase, HMG-CoA synthase, HMG-CoA reductase, mevalonate kinase, 5-phosphomevalonate kinase, diphosphomevalonate decarboxylase, and isopentenyl diphosphate isomerase, in the order from the upstream.

When IPP is synthesized from acetyl-CoA via the mevalonate pathway, NAD(P)H is consumed.

A gene that expresses the mevalonate pathway is a gene encoding these enzymes and synthesizing IPP from acetyl-CoA by the expression of the gene.

Note here that the mevalonate pathway is inherent in all eukaryotes, but is found also in prokaryotes. Examples of prokaryotes having a mevalonate pathway include, with respect to actinomycetes, *Streptomyces sp.* Strain CL190 (Takagi M. et al., J. Bacteriol. 2000, 182 (15), 4153-7), and *Streptomyces griseolosporeus* MF730-N6 (Hamano Y. et al., Biosci. Biotechnol. Biochem. 2001, 65(7), 1627-35).

Examples thereof include, with respect to bacteria, *Lactobacillus helvecticus* (Smeds A et al., DNA seq. 2001, 12(3), 187-190), *Corynebacterium amycolatum, Mycobacterium marinum, Bacillus coagulans, Enterococcus faecalis, Streptococuss agalactiae, Myxococcus xanthus* and the like (Lombard J. et al., Mol. Biol. Evol. 2010, 28 (1), 87-99).

Examples thereof include, with respect to archaea, genus *Aeropyrum,* genus *Sulfolobus,* genus *Desulfurococcus,* genus *Thermoproteus,* genus *Halobacterium,* genus *Methanococcus,* genus *Thermococcus,* genus *Pyrococcus,* genus *Methanopyrus,* genus *Thermoplasma* and the like (Lombard J. et al., Mol. Biol. Evol. 2010, 28 (1), 87-99).

In the present invention, the origin of mevalonate pathway as the exogenous NAD(P)H consumption pathway is not particularly limited, but a mevalonate pathway of yeast, prokaryote, or actinomycete is preferable, and a mevalonate pathway of actinomycete is particularly preferable.

Note here that the HMG-CoA reductase includes two types of reductase, i.e., NADPH-dependent HMG-CoA reductase (EC1.1.1.34) and NADH-dependent HMG-CoA reductase (EC1.1.1.88). The HMG-CoA reductase of actinomycete is NADPH-dependent. However, in the present invention, according to the purposes, NADH-dependent HMG-CoA reductase may be used. Examples of the NADH-dependent HMG-CoA reductase include mvaA (P13702) derived from *Pseudomonas mevalonii,* hmgA-1Mtc_0274 (H8I942) derived from *Methanocella conradii,* mvaA LLKF_1694 (D2BKK7) derived from *Lactococcus lactis subsp. lactis* (strain KF147), mvaA SSA_0337 (A3CKT9) derived from *Streptococcus sanguinis* (strain SK36), or the like.

In the recombinant cell of the present invention, the expression of at least one endogenous NAD(P)H consumption pathway that is different from the exogenous NAD(P)H consumption pathway is down-regulated. Hereinafter, an embodiment of the recombinant cell that is a *Clostridium* bacterium or a *Moorella* bacterium, in which the exogenous NAD(P)H consumption pathway is a mevalonate pathway, is specifically described.

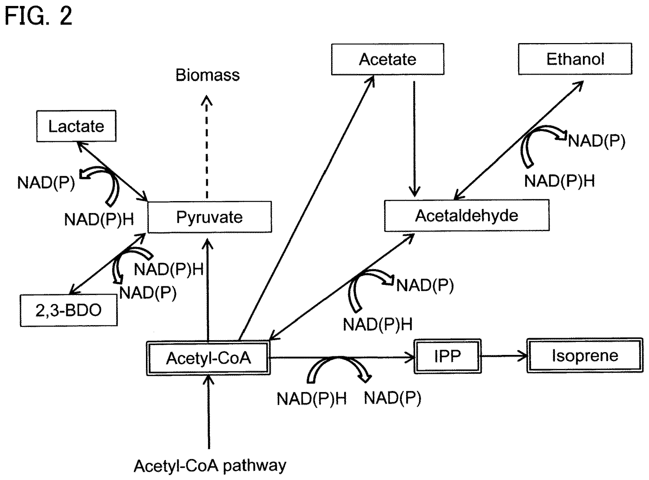

Fig. 2 shows a part of a central metabolic pathway in the *Clostridium* bacterium or *Moorella* bacterium in which the mevalonate pathway has been introduced. In the example shown in Fig. 2, IPP is synthesized from acetyl-CoA via the mevalonate pathway (exogenous NAD(P)H consumption pathway). Note here that the acetyl-CoA is supplied from the acetyl-CoA pathway (Wood-Ljungdahl pathway) (Fig. 1).

In an example shown in Fig. 2, the endogenous NAD(P)H consumption pathway includes pathways for carrying out conversion from acetaldehyde to ethanol, conversion from acetyl-CoA to acetaldehyde, conversion from pyruvate to lactate, and conversion from pyruvic acid to 2,3-butanediol, respectively. Herein, the conversion from acetaldehyde to ethanol proceeds by ethanol dehydrogenase, the conversion from acetyl-CoA to acetaldehyde proceeds by acetaldehyde dehydrogenase, the conversion from pyruvic acid to lactate proceeds by lactate dehydrogenase, and the conversion from pyruvate to 2,3-butanediol proceeds by 2,3-butanediol dehydrogenase, respectively.

In this embodiment, expression of at least one of the enzymes involved in the endogenous NAD(P)H consumption pathway is down-regulated. Therefore, an consumption amount of the NAD(P)H in the endogenous NAD(P)H consumption pathway is suppressed, and NAD(P)H is preferentially supplied to the mevalonate pathway that is the exogenous NAD(P)H consumption pathway. As a result, IPP synthesis via the mevalonate pathway is carried out efficiently.

The number of the above-mentioned down-regulated enzyme may be one or more than one.

As mentioned above, the down-regulation of gene expression means regulation for reducing an expression amount of a gene, and regulation for impairing a function of a gene. Examples of the down-regulation of gene expression include a gene knockout and a gene knockdown. Furthermore, the gene knockout includes deletion of the gene itself. Furthermore, an embodiment in which expression amount of a gene is reduced by modifying a gene expression-regulating region of a promoter and the like is also included in the down-regulation of gene expression. When a plurality of down-regulated genes exist, embodiments of the down-regulations of gene may be the same as each other or different from each other.

Examples of a gene knockout method of a *Clostridium* bacterium or a *Moorella* bacterium include a technique using a homologous recombination (Leang C. et al., Appl Environ Microbiol. 2013 79(4), 1102-9), a technique using a group II intron (John T. Heap et al., Journal of Microbiological Methods 70 (2007) 452-464), a technique using Cre-Lox66/lox71 (Vel Berzin et al., Appl Bioichem Biotechnol., 2012 168: 1384-1393), and the like.

Note here that an example shown in Fig. 2 includes a pathway in which a conversion from acetyl-CoA to acetic acid is carried out exists other than the NAD(P)H consumption pathway. This pathway proceeds by phosphotransacetylase. Then, in addition to the above-mentioned endogenous NAD(P)H consumption pathway, by down-regulating the expression of phosphotransacetylase, wasteful consumption of acetyl-CoA is suppressed. As a result, the IPP synthesis via the mevalonate pathway is carried out more efficiently.

Furthermore, more preferably, by down-regulating the expression of acetate kinase, generation of excessive hydrocarbon can be suppressed in a pathway in which a conversion from acetyl-CoA to acetic acid is carried out.

An organic compound of not less than C4, which is produced by the recombinant cell of the present invention, is not particularly limited, and examples thereof include terpene. As one embodiment, as a gene that expresses an exogenous NAD(P)H consumption pathway a gene cluster that includes a gene that expresses mevalonic acid and a gene encoding an enzyme for generating isoprene from IPP is employed. Thus, it is possible to provide a recombinant cell capable of producing isoprene.

Examples of the enzyme for producing isoprene from IPP include isoprene synthase. The isoprene synthase is not particularly limited as long as it can exert its enzyme activity in the recombinant cell. Similarly, the gene encoding isoprene synthase is not particularly limited as long as it is normally transcribed and translated in the recombinant cell. The gene encoding isoprene synthase may be codon-modified for ease of transcription in the recombinant cell. For example, when a host cell is a *Clostridium* bacterium, the codon of the nucleic acid to be introduced may be modified based on the information of codon usage frequency of *Clostridium* bacteria.

Isoprene synthase is found in many plants. Specific examples of the isoprene synthase include one derived from poplar (*Populus nigra*) (GenBank Accession No.: AM410988.1). Other examples include one derived from *Bacillus subtilis* (Sivy TL. et al., Biochem. Biophys. Res. Commu. 2002, 294(1), 71-5).

SEQ ID NO: 1 shows a nucleotide sequence of a gene (DNA) encoding the isoprene synthase derived from poplar and a corresponding amino acid sequence. SEQ ID NO: 2 shows only the amino acid sequence. DNA having the nucleotide sequence of SEQ ID NO: 1 is one example of the nucleic acid encoding isoprene synthase.

Further, the gene encoding isoprene synthase includes at least a nucleic acid encoding the following protein (a), (b) or (c):
(a) a protein consisting of an amino acid sequence of SEQ ID NO: 2,
(b) a protein consisting of an amino acid sequence in which 1 to 20 amino acids are deleted, substituted or added in the amino acid sequence of SEQ ID NO: 2, and having isoprene synthase activity, and
(c) a protein consisting of an amino acid sequence having homology of 60% or more with the amino acid sequence of SEQ ID NO: 2, and having isoprene synthase activity.

The homology of the amino acid sequence in (c) is more preferably 80% or more, further preferably 90% or more, and particularly preferably 95% or more.

As another embodiment, as a gene that expresses an exogenous NAD(P)H consumption pathway, a gene cluster that includes a gene that expresses the mevalonate pathway and a gene encoding an enzyme for producing cyclic terpene from isopentenyl diphosphate is employed. Thus, a recombinant cell capable of producing cyclic terpene can be provided.

Examples of the enzyme for producing cyclic terpene from isopentenyl diphosphate include geranyl diphosphate synthase (GPP synthase) and/or neryl diphosphate synthase (NPP synthase), and cyclic monoterpene synthase.

Any one of the GPP synthase and the NPP synthase may be employed, or both may be employed.

The GPP synthase is not particularly limited as long as it can exert its enzyme activity in the recombinant cell. Similarly, the gene encoding GPP synthase is not particularly limited as long as it is normally transcribed and translated in the recombinant cell.

The same is true for NPP synthase, cyclic monoterpene synthase, and genes encoding synthase.

Specific examples of the GPP synthase include one derived from *Arabidopsis thaliana* (GenBank Accession No.: Y17376/At2g34630; Bouvier, F., et al., Plant J,. 2000, 24, 241-52.), one derived from *Mycobacterium tuberculosis* (GenBank Accession No.: NP_215504; Mann, F. M., et al., FEBS Lett., 2011, 585, 549-54.), and the like.

SEQ ID NO: 3 shows a nucleotide sequence of the above-mentioned gene (DNA) encoding GPP synthase derived from *Arabidopsis thalania* and the corresponding amino acid sequence. SEQ ID NO: 4 shows only an amino acid sequence. DNA having the nucleotide sequence of SEQ ID NO: 3 is an example of a gene encoding the GPP synthase.

Furthermore, a gene encoding the GPP synthase includes the nucleic acid encoding protein of the following (a), (b) or (c):
(a) a protein consisting of an amino acid sequence of SEQ ID NO: 4,
(b) a protein consisting of an amino acid sequence in which 1 to 20 amino acids are deleted, substituted or added in the amino acid sequence of SEQ ID NO: 4, and having geranyl diphosphate synthase activity, and
(c) a protein consisting of an amino acid sequence having homology of 60% or more with the amino acid sequence of SEQ ID NO: 4, and having geranyl diphosphate synthase activity.

Note here that the homology of an amino acid sequence in (c) is more preferably 80% or more, further preferably 90% or more, and particularly preferably 95% or more.

Specific examples of the NPP synthase includes one derived from *Solanum lycopersicum* (GenBank Accession No.: FJ797956), and the like.

SEQ ID NO: 5 shows a nucleotide sequence of the above-mentioned gene (DNA) encoding the NPP synthase derived from *Solanum lycopersicum* and the corresponding amino acid sequence. SEQ ID NO: 6 shows only an amino acid sequence thereof. DNA having the nucleotide sequence of SEQ ID NO: 5 is an example of a gene encoding the NPP synthase.

Furthermore, a gene encoding the NPP synthase includes at least a nucleic acid encoding protein of the following (d), (e) or (f):
(d) a protein consisting of the amino acid sequence of SEQ ID NO: 6,
(e) a protein consisting of the amino acid sequence in which 1 to 20 amino acids are deleted, substituted or added in the amino acid sequence of SEQ ID NO: 6, and having neryl diphosphate synthase activity, and
(f) a protein consisting of an amino acid sequence having homology of 60% or more with the amino acid sequence of SEQ ID NO: 6, and having neryl diphosphate synthase activity.

Note here that the homology of the amino acid sequence in (f) is more preferably 80% or more, further preferably 90% or more, and particularly preferably 95% or more.

Examples of the cyclic monoterpene synthase include β-phellandrene synthase. By the action of the β-phellandrene synthase, β-phellandrene is synthesized from GPP and/or NPP. Furthermore, as byproduct, 4-carene and limonene can be also synthesized.

Specific examples of the β-phellandrene synthase and genes encoding the same include one derived from *Solanum lycopersicum* (GenBank Accession No.: FJ797957; Schilmiller, A. L., et al., Proc Natl Acad Sci U S A., 2009, 106, 10865-70.), one derived from *Lavandula angustifolia* (GenBank Accession No.: HQ404305; Demissie, Z. A., et al., Planta, 2011,. 233, 685-96), and the like.

SEQ ID NO: 7 shows a nucleotide sequence of the above-mentioned gene (DNA) encoding the β-phellandrene synthase derived from *Solanum lycopersicum* and the corresponding amino acid sequence. SEQ ID NO: 8 shows only an amino acid sequence.

SEQ ID NO: 9 shows a nucleotide sequence of the above-mentioned gene (DNA) encoding the β-phellandrene synthase derived from *Lavandula angustifolia* and the corresponding amino acid sequence. SEQ ID NO: 10 shows only an amino acid sequence.

DNA having the nucleotide sequence of SEQ ID NO: 7 or SEQ ID NO: 9 is an example of a gene encoding the β-phellandrene synthase.

Furthermore, a gene encoding the β-phellandrene synthase includes at least a nucleic acid encoding protein of the following (g), (h) or (i):
(g) a protein consisting of the amino acid sequence of SEQ ID NO: 8 or 10;
(h) a protein consisting of the amino acid sequence in which 1 to 20 amino acids are deleted, substituted or added in the amino acid sequence of SEQ ID NO: 8 or 10, and having β-phellandrene synthase activity, and
(i) a protein consisting of an amino acid sequence having homology of 60% or more with the amino acid sequence of SEQ ID NO: 8 or 10, and having β-phellandrene synthase activity.

Note here that the homology of the amino acid sequence in (i) is more preferably 80% or more, further preferably 90% or more, and particularly preferably 95% or more.

Specific embodiments of introducing a gene that expresses an exogenous NAD(P)H consumption pathway and down-regulating the expression of the endogenous NAD(P)H consumption pathway includes substituting a part or whole of an adhE1 gene and an adhE2 gene existing on a genome of a *Clostridium* bacterium or a *Moorella* bacterium with the above-mentioned gene cluster by homologous recombination. Both of the adhE1 gene and the adhE2 gene include both of an acetaldehyde dehydrogenase gene and an ethanol dehydrogenase gene, and both are located adjacent to each other on the genome.

The recombinant cell of the present invention includes a gene that expresses an exogenous NAD(P)H consumption pathway. For example, a gene that expresses an exogenous NAD(P)H consumption pathway is introduced into a host cell. The introduced gene may be incorporated into the genome of the host cell, or may exist out of the genome in a state in which it is incorporated in a plasmid.

The method of introducing a gene into the host cell is not particularly limited, and may be selected appropriately depending on the kind of the host cell and the like. For example, a vector that can be introduced into the host cell and can allow expression of the gene incorporated therein may be used.

For example, when the host cell is a prokaryote such as a bacterium, a vector that can self duplicate or can be incorporated in chromosome in the host cell, and contains a promoter at the position allowing transcription of the inserted gene (DNA) can be used. For example, it is preferred to construct in the host cell a series of structures including a promoter, a ribosome binding sequence, the above gene (DNA) and a transcription termination sequence by using the vector.

In the case where the host cell is a *Clostridium* bacterium (including related species such as *Moorella* bacteria), a shuttle vector pIMP1 between *Clostridium* bacterium and *Escherichia coli* (Mermelstein LD et al., Bio/technology 1992, 10, 190-195) may be used. The shuttle vector is a fusion vector of pUC9 (ATCC 37252) and pIM13 isolated from *Bacillus subtilis* (Projan SJ et al., J. Bacteriol. 1987, 169 (11), 5131-5139) and is retained stably in the *Clostridium* bacterium. Other examples of a shuttle vector between *Clostridium* bacterium and *E. coli* include pSOS95 (GenBank: AY187686.1).

For gene introduction into the *Clostridium* bacterium, an electroporation method is generally used. However, the introduced exogenous plasmid immediately after gene introduction is liable to be decomposed by a restriction enzyme Cac824I and the like, and is therefore very instable. For this reason, it is preferred to once amplify the vector from pIMP1 in *E. coli,* for example, strain ER2275 having pAN1 (Mermelstein LD et al., Apply. Environ. Microbiol. 1993, 59(4), 1077-1081) carrying a methyl transferase gene from *Bacillus subtilis* phage Φ3T1, followed by a methylation treatment, and to recover the resultant vector from *E*. *coli* for use in transformation by electroporation. Recently, Cac824I gene-deficient *Clostridium acetobuthylicum* has been developed, and make it possible to stably carry a vector which is not subjected to a methylation treatment (Dong H. et al., PLoS ONE 2010, 5 (2), e9038). Furthermore, it should be noted that an unmethylated vector is efficiently introduced by amplifying the vector with NEB express which is an improved version of *E. coli* BL21 strain (Leang C. et al., Appl Environ Microbiol. 2013 79(4), 1102-9). Also, it should be noted that a gene is introduced into a genome of a *Clostridium* bacterium by using pBluescipt II KS(-) or pUC19 vector into which a sequence homologous to that of the host cell is integrated (Leang C. et al., Appl Environ Microbiol. 2013 79(4), 1102-9, Berzin V. et al., Appl Biochem Biotechnol (2012) 167:338-347).

Examples of the promoter for heterologous gene expression in *Clostridium* bacteria include thl (thiolase) promoter (Perret S et al., J. Bacteriol. 2004, 186(1), 253-257), Dha (glycerol dehydratase) promoter (Raynaud C. et al., PNAS 2003, 100(9), 5010-5015), ptb (phosphotransbutyrylase) promoter (Desai RP et al., Appl. Environ. Microbiol. 1999, 65(3), 936-945), and adc (acetoacetate decarboxylase) promoter (Lee J et al., Appl. Environ. Microbiol. 2012, 78 (5), 1416-1423). However, in the present invention, sequences of promoter regions used in operons of various metabolic systems found in the host cell or the like may be used without limited to the above examples.

Furthermore, promoters that regulate pta (phosphate acetyltransferase), adhE (aldehyde/alcohol dehydrogenase), CODH (carbon monoxide dehydrogenase), acsA (acetyl-coA synthase α subunit), ferredoxin, Rnf complex, hydrogenase, GroE, ATP synthase, or the like can be used. In the case of conducting a syngas fermentation, promoters that can be activated by carbon monoxide, carbon dioxide, or hydrogen are also suitable.

For introducing plural kinds of genes into the host cell by using a vector, the genes may be incorporated in one vector, or incorporated in individual vectors. When plural kinds of genes are incorporated in one vector, these genes may be expressed under a common promoter for these genes, or expressed under individual promoters. An exemplary form of introducing plural kinds of genes include a mode of introducing isoprene synthase gene, GPP synthase gene, NPP synthase gene, cyclic monoterpene synthase gene, or the like in addition to the gene acting in the mevalonate pathway.

As described above, while the known vectors that can be used in the present invention have been shown, the region involved in transcription control and replication regions such as promoter and terminator can be modified depending on the purpose. The modification includes change to other natural gene sequence in each host cell or its related species, and change to an artificial gene sequence.

One aspect of the method for producing an organic compound of the present invention includes bringing at least one C1 compound selected from the group consisting of carbon monoxide and carbon dioxide into contact with the above-mentioned recombinant cell to produce an organic compound having 4 or more carbon atoms from the C1 compound in the recombinant cell.

In a preferable embodiment, the recombinant cell is cultured by using at least one C1 compound selected from the group consisting of carbon monoxide and carbon dioxide as a carbon source to produce an organic compound having 4 or more carbon atoms in the recombinant cell. The C1 compound used as a carbon source may be used singly or in combination of two or more. Furthermore, the C1 compound is preferably used as a main carbon source, and more preferably as the only carbon source.

Furthermore, it is preferable to concurrently provide hydrogen (H₂) as an energy source.

The method for culturing the recombinant cell of the present invention is not particularly limited, and can be appropriately carried out depending on the type of the recombinant cell, and the like. When the recombinant cell is a *Clostridium* bacterium (obligatory anaerobic and strictly anaerobic), it is cultured, for example, in a nutrient condition including inorganic salts required for growth, and syngas. Preferably, it is cultured under a pressurized condition at about 0.2 to 0.3 MPa (absolute pressure). Furthermore, for improving initial proliferation and attained cell density, small amounts of organic substances such as vitamins, yeast extract, corn steep liquor, and Bacto Tryptone, may be added.

Note here that the recombinant cell is aerobic or facultative anaerobic, for example, it may be cultured in a liquid medium under aeration and stirring.

Methods other than culture can be employed. That is, the organic compound can be produced by bringing the above-mentioned C1 compound into contact with the recombinant cell regardless of whether or not cell division (cell proliferation) occurs. For example, the above-mentioned C1 compound is continuously fed to the immobilized recombinant cell, so that the organic compound can be continuously produced. Also in this case, the C1 compound used may be used singly or in combination of two or more. Furthermore, it is preferable to bring hydrogen (H₂) into contact concurrently as an energy source.

In a preferable embodiment, the recombinant cell is provided with a gas mainly containing carbon monoxide, a gas mainly containing carbon monoxide and hydrogen, a gas mainly containing carbon dioxide and hydrogen, or a gas mainly containing carbon monoxide, carbon dioxide and hydrogen. In other words, the organic compound is produced from carbon monoxide or carbon dioxide in such a gas by culturing the recombinant cell by using the above-mentioned gas as a carbon source, or by bringing the above-mentioned gas into contact with the recombinant cell. Also in this case, hydrogen is used as an energy source.

In addition to these C1 compounds, the recombinant cell may be provided with formic acid or methanol. In other words, the recombinant cell is cultured by using formic acid or methanol and the above-mentioned gas is used as a carbon source, or formic acid or methanol and the above-mentioned gas are brought into contact with the recombinant cell. Addition of formic acid or methanol allows for the culture efficiency and the production efficiency of the organic compound to be improved. Examples of the embodiment of providing include concurrently providing a recombinant cell with formic acid or methanol and the above-mentioned gas.

According to the embodiment in which a gene cluster that includes a gene that expresses a mevalonate pathway and a gene encoding an enzyme for producing isoprene from isopentenyl diphosphate as the gene expressing an exogenous NADP consumption pathway, isoprene can be produced as the organic compound.

Furthermore, according to the embodiment in which a gene cluster including a gene that expresses a mevalonate pathway and a gene encoding an enzyme for producing cyclic terpene from isopentenyl diphosphate, as the gene expressing an exogenous NADP consumption pathway, cyclic terpene can be produced as the organic compound. According to an embodiment in which the enzyme for producing cyclic terpene from isopentenyl diphosphate is geranyl diphosphate synthase and/or neryl diphosphate synthase, as well as cyclic monoterpene synthase, cyclic monoterpene can be produced. Furthermore, according to an embodiment in which the cyclic monoterpene synthase is β-phellandrene synthase, as the cyclic monoterpene, β-phellandrene, 4-carene, or limonene can be produced.

The produced organic compound is accumulated in the cell or released to the outside of the cell. For example, in an embodiment in which cyclic terpene is produced, by using the recombinant cell prepared from a host cell of a *Clostridium* bacterium or a *Moorella* bacterium, and collecting cyclic monoterpene that has been released to the outside of the cell, followed by performing isolation and purification by, for example, distillation, purified cyclic terpene can be obtained.

Furthermore, methods for isolation and purification of the organic compound from the cultured product of the recombinant cell are not particularly limited. When cyclic terpene is produced, for example, a culture solution (a culture supernatant) can be extracted with an appropriate solvent such as pentane, and purified to a high purity by chromatography such as reverse-phase chromatography and gas chromatography. Since most of cyclic terpene released to the outside of the cell is evaporated into a vapor phase, it may be liquefied and collected by a cold trap and the like.

Bicarbonate can be sometimes used in place of carbon dioxide. In other words, Clostridium bacteria and related species are known to have carbonic anhydrase (CA) (EC 4.2.1.1: H₂O + CO₂ ⇔ HCO₃⁻ + H⁺) (Braus-Stromeyer SA et al., J. Bacteriol. 1997, 179(22), 7197-7200). Bicarbonate such as NaHCO₃ which is a source of HCO₃⁻ can be used as a CO₂ source.

Herein, combinations of carbon monoxide, carbon dioxide, formic acid, and methanol that can be provided to the recombinant cell in the case where the host cell has the acetyl-CoA pathway and the methanol pathway (Fig. 1) are described.

In acetyl-CoA synthesis by the acetyl-CoA pathway, a synthesis process of acetyl-CoA from CoA, methyltetrahydrofolate ([CH₃]-THF), and CO by the actions of methyltransferase, Corrinoid iron-sulfur protein (CoFeS-P), acetyl-CoA synthase (ACS), and CODH is essential (Ragsdale SW et al., B.B.R.C. 2008, 1784(12), 1873-1898).

On the other hand, it is known that adding formic acid and/or methanol besides CO and/or CO₂ as a carbon source in culturing of *Butyribacterium methylotrophicum* increases the content of tetrahydrofolate (THF) in CO metabolism, namely, methyl branch in the acetyl-CoA pathway, and activities of CODH, formate dehydrogenase (FDH) and hydrogenase required in CO metabolism (Kerby R. et al., J. Bacteriol. 1987, 169(12), 5605-5609). Also in *Eubacterium limosum* or the like, it is demonstrated that high proliferation is achieved by using CO₂ and methanol as a carbon source in an anaerobic condition (Genthner BRS. et al., Appl. Environ. Microbiol., 1987, 53(3), 471-476).

The influence of methanol on syngas utilizing microorganisms, and the results of genome analysis of *Moorella thermoacetica* (*Clostridium thermoaceticum*), *Clostridium ljungdahlii* and the like (Pierce E. et al., Environ. Microbiol. 2008, 10(10), 2550-2573; Durre P. et al., PNAS 2010, 107(29), 13087-13092) can give an explanation for involvement of the methanol pathway as shown in Fig. 1 as a donor of a methyl group in the acetyl-CoA pathway (Wood-Ljungdahl pathway) in these microorganism species.

Actually in some *Clostridium* bacteria, the forward activity of formate dehydrogenase (FDH) (EC 1.2.1.2/1.2.1.43: Formate + NAD(P)⁺ ⇔ CO₂ + NAD(P)H) (formation of CO₂ from formate) is confirmed (Liu CL et al., J. Bacteriol. 1984, 159(1), 375-380; Keamy JJ et al., J. Bacteriol. 1972, 109(1), 152-161). Therefore, in these strains, a reaction in the direction of generating CO₂ from methanol (CH₃OH) and/or formic acid (HCOOH) can partly proceed when CO₂ and/or CO is deficient (Fig. 1). This can also be explained by the phenomenon that formate dehydrogenase activity and CODH activity increase by addition of CH₃OH (Kerby R et al., J. Bateriol. 1987, 169(12), 5605-5609) as described above. In other words, these can be proliferated with formic acid (HCOOH) or methanol (CH₃OH) as the sole carbon source.

Even if the host cell strain inherently lacks the forward activity of formate dehydrogenase, it may be provided with the forward activity by gene modification such as introduction of mutation, introduction of foreign gene, or genome shuffling.

For these reasons, it is possible to produce the aforementioned organic compound using the following gas or liquid when the host cell has the acetyl-CoA pathway and the methanol pathway.
CO
CO₂
CO/H₂
CO₂/H₂
CO/CO₂/H₂
CO/HCOOH
CO₂/HCOOH
CO/CH₃OH
CO₂/CH₃OH
CO/H₂/HCOOH
CO₂/H₂/HCOOH
CO/H₂/CH₃OH
CO₂/H₂/CH₃OH
CO/CO₂/H₂/HCOOH
CO/CO₂/H₂/CH₃OH
CH₃OH/H₂
HCOOH/H₂
CH₃OH
HCOOH

When the recombinant cell of the present invention is cultured exclusively for cell proliferation, rather than for production of the organic compound, it is not necessary to use carbon monoxide and/or carbon dioxide as a carbon source. For example, the recombinant cell may be cultured using other carbon sources such as saccharides or glycerin which is easily assimilated by the recombinant cell of the present invention

In the following, the present invention will be described more specifically by way of Examples. However, the present invention is not limited to these examples.

### EXAMPLE 1

In this Example, in the recombinant cell of *Clostridium ljungdahlii* as one type of syngas assimilating bacteria, high efficiency production of β-phellandrene was carried out.

### (1) Construction of various vectors

With reference to Appl Biochem Biotechnol (2012) 168:1384-1393 and Bioprocess Biosyst Eng (2014) 37:245_260, pUC-ΔadhE-Cat (SEQ ID NO: 11) including an adhE1 (CLJU_c16510) upstream sequence of *C. ljungdahlii,* a lox66 sequence, a chloramphenicol resistant gene (FM201786), a lox71 sequence, and an adhE2 (CLJU_c16520) downstream sequence of *C*. *ljungdahlii* was prepared.

Furthermore, pJIR750ai (Sigma-Aldrich) that is a *Clostridium*/*E.coli* binary vector was modified to construct pSK1-PHS-NPPS (SEQ ID NO: 12) including a nucleotide sequence in which a β-phellandrene synthase gene (GenBank Accession No.: FJ797957; Schilmiller, A. L., et al., Proc Natl Acad Sci U S A., 2009, 106, 10865-70.), a neryl diphosphate (NPP) synthase gene (GenBank Accession No.: FJ797956), and a chloramphenicol resistant gene (FM201786) had been codon-modified.

Furthermore, in addition to the sequence of SEQ ID NO: 12, pSK1-PHS-NPPS-MVA (SEQ ID NO: 13) further including a mevalonate pathway gene cluster derived from actinomycete (including mvaA derived from *Pseudomonas mevalonii*) was constructed. Each sequence is codon-modified with consideration of the codon usage frequency of a *Clostridium* bacterium.

### (2) Preparation of adhE gene knock out Clostridium strain

pUC-ΔadhE-Cat was introduced into *C*. *ljungdahlii* (DSM13528/ATCC55383) using a technique recommended in Leang C. et al., Appl Environ Microbiol. 2013 79(4), 1102-9, and selected in an ATCC1754 agar medium (1.5% agar) including 10 µg/mL chloramphenicol to produce adhE1/adhE2 knock out strains (ΔadhE strains). A ΔadhE strain competent cell was prepared using a technique recommended in Leang C. et al., Appl Environ Microbiol. 2013 79(4), 1102-9. A pUCΔadhE strain competent cell was subjected to Cre-recombinase treatment to remove a chloramphenicol resistant gene by a technique described in Bioprocess Biosyst Eng (2014) 37: 245-260 so as to obtain a chloramphenicol sensitive strain (ΔadhEΔCm strain).

### (3) Gene introduction into DSM13528/ATCC55383 strain and ΔadhEΔCm strain

The pSK1-PHS-NPPS and the pSK1-PHS-NPPS-MVA were introduced into the DSM13528/ATCC55383 strain, and the pSK1-PHS-NPPS-MVA was introduced into the ΔadhEΔCm strain, respectively, by electroporation using a technique described in Leang C. et al., Appl Environ Microbiol. 2013 79(4), 1102-9, and selected in an ATCC1754 agar medium containing 10 µg/mL chloramphenicol (1.5% agar containing fructose).

### (4) β-phellandrene quantification

The pSK1-PHS-NPPS-introduced DSM13528/ATCC55383 strain, the pSK1-PHS-NPPS-MVA-introduced DSM13528/ATCC55383 strain, and the pSK1-PHS-NPPS-MVA-introduced ΔadhEΔCm strain selected in the above item (3) were respectively cultured at 37°C in an aerobic condition. As a culture medium for inoculation, 5 mL of an ATCC1754 medium containing 10 µg/mL chloramphenicol (pH = 5.0, fructose was not contained) was used. A 27 mL-volume hermetically-sealable headspace vial vessel was charged with a mixed gas of CO/CO₂/H₂ = 33/33/34% (volume ratio), and filled with the mixed gas at a gas pressure of 0.25 MPa (absolute pressure), and hermetically sealed with an aluminum cap, and then shaking culture was conducted. In the cultured product in which proliferation was found, at the time when OD600 reached 1.0, culture was terminated, and the vapor phase was analyzed using a gas chromatograph mass spectrometer (Shimadzu GCMS-QP2010 Ultra).

As a result, in the pSK1-PHS-NPPS-introduced DSM13528/ATCC55383 strain, β-phellandrene was detected in a production amount of 0.15 mg on average per dry cell (g). In the pSK1-PHS-NPPS-MVA-introduced DSM13528/ATCC55383 strain, β-phellandrene was detected in a production amount of 10 mg on average per dry cell (g). In the pSK1-PHS-NPPS-MVA-introduced ΔadhEΔCm strain, β-phellandrene was detected in an amount of 55 mg on average per dry cell (g).

As mentioned above, it was shown that when a foreign mevalonate pathway for producing a β-phellandrene precursor was introduced into a host cell, the production amount of the β-phellandrene that is one type of cyclic monoterpene was found to be increased. In addition, it was shown that when adhE1 and adhE2 genes for the NAD(P)H consumption pathway were knocked out, β-phellandrene can be produced more efficiently.

### EXAMPLE 2

In this Example, a sequence in which a gene cluster that includes a genome sequence of *C*. *ljungdahlii,* as well as an exogenous mevalonate pathway and isoprene synthase had been introduced into pUC57 (GenBank Accession No.Y14837) was constructed, and introduced into *C*. *ljungdahlii* by homologous recombination to prepare a recombinant cell in which adhE1 and adhE2 of the host cell had been knocked out, and the gene cluster had been introduced into a genome. Furthermore, isoprene was produced in this recombinant cell at high efficiency.

### (1) Construction of various vectors

A gene cluster was introduced into pUC57 (GenBank Accession No. Y14837) to construct pUC-ΔadhE-IspS-MVA (SEQ ID NO: 14), the gene cluster including an adhE1 (CLJU_c16510) upstream sequence and an adhE2 (CLJU_c 16520) downstream sequence both of *C*. *ljungdahlii* (DSM13528/ATCC55383), a mevalonate pathway gene derived from actinomycete (including mvaA derived from *Pseudomonas mevalonii*), an isoprene synthase gene derived from *Populus nigra* (GenBank Accession No.: AM410988.1), and a chloramphenicol resistant gene (FM201786) (Appl Biochem Biotechnol. 2012 May;167(2):338-47.). Furthermore, three genes were introduced into the sequence-modified pJIR750ai (Sigma-Aldrich) to construct pSK1-IspS-MVA (SEQ ID NO: 15), the three genes including an isoprene synthase gene derived from *Populus nigra* (GenBank Accession No.: AM410988.1), a chloramphenicol resistant gene (FM201786), and a mevalonate pathway gene derived from actinomycete (including mvaA derived from *Pseudomonas mevalonii*)*.* Each sequence was codon-modified with consideration of the codon usage frequency of a *Clostridium* bacterium.

### (2) Gene introduction

The pUC-ΔadhE-IspS-MVA was introduced into *C*. *ljungdahlii* (DSM13528/ATCC55383) by the electroporation technique of Leang C. *et al.* and selected in an ATCC1754 agar medium containing 10 ug/mL chloramphenicol (1.5% agar containing fructose). A reconbinant cell in which deletion of adhE1 and adhE2 and introduction of an isoprene synthase gene into a genome by gene introduction of pUC-ΔadhE-IspS-MVA had been recognized was selcted and referred to as a ΔadhE-IspS-MVA strain. Furthermore, pSK1-IspS-MVA was introduced into *C*. *ljungdahlii* (DSM13528/ATCC55383) and ΔadhEΔCm strain prepared in Example 1, respectively, by the electroporation technique of Leang C. *et al,* followed by selecting in an ATCC1754 agar medium containing 10 µg/mL chloramphenicol (1.5% agar containing fructose).

### (3) Isoprene quantification

The ΔadhE-IspS-MVA strain, pSK1-IspS-MVA-introduced DSM13528/ATCC55383, pSK1-IspS-MVA-introduced ΔadhEΔCm strain that had been obtained in the above item (2) were respectively cultured at 37°C in an anaerobic condition. A 27 mL-volume hermetically-sealable headspace vial vessel was charged with 5 mL of ATCC medium 1754 PETC medium containing 10 µg/mL chloramphenicol (pH = 5.0, fructose was not contained), and filled with a mixed gas of CO/CO₂/H₂ = 33/33/34% (volume ratio) at a gas pressure of 0.25 MPa (absolute pressure), and sealed with an aluminum cap, and then shaking culture was conducted. The culture was terminated at the time when OD600 reached 1.0, and the vapor phase was analyzed using a gas chromatograph mass spectrometer (Shimadzu GCMS-QP2010 Ultra). As a result, in the ΔadhE-IspS-MVA strain, 185 mg on average of isoprene per dry cell (g) was detected. In the pSK1-IspS-MVA-introduced ΔadhEΔCm strain, 74 mg on average of isoprene per dry cell (g) was detected. In pSK1-IspS-MVA-introdued DSM13528/ATCC55383, 15 mg of isoprene on average per dry cell (g) was detected.

As mentioned above, it was shown that when a foreign mevalonate pathway for producing an isoprene precursor was introduced into a host cell, and adhE1 and adhE2 genes for the host NAD(P)H consumption pathway were knocked out, isoprene can be produced more efficiently. Furthermore, it was shown that by incorporating the nucleotide sequence into a genome by homologous recombination, isoprene could be produced more efficiently.

## Claims

1. A recombinant cell having a function of synthesizing acetyl-CoA from methyltetrahydrofolate, carbon monoxide, and CoA, comprising:
a gene that expresses an exogenous NAD(P)H consumption pathway, the gene being expressed in the recombinant cell,
wherein expression in at least one of endogenous NAD(P)H consumption pathways of the recombinant cell is down-regulated, and the endogenous NAD(P)H consumption pathway is different from the exogenous NAD(P)H consumption pathway, and
the recombinant cell produces an organic compound having 4 or more carbon atoms from at least one selected from the group consisting of carbon monoxide and carbon dioxide via the exogenous NAD(P)H consumption pathway.

2. The recombinant cell according to claim 1, being a *Clostridium* bacterium or a *Moorella* bacterium.

3. The recombinant cell according to claim 2, being *Clostridium ljungdahlii, Clostridium autoethanogenum, Clostridium carboxidivorans, Clostridium ragsdalei, Clostridium kluyveri,* or *Moorella thermoacetica.*

4. The recombinant cell according to claim 2 or 3, wherein the exogenous NAD(P)H consumption pathway is a mevalonate pathway.

5. The recombinant cell according to claim 4, wherein the mevalonate pathway is a mevalonate pathway of yeast, prokaryote, or actinomycete.

6. The recombinant cell according to claim 4, wherein the mevalonate pathway is a mevalonate pathway of actinomycete.

7. The recombinant cell according to claim 4, wherein HMG-CoA reductase of the mevalonate pathway includes NADH-dependent HMG-CoA reductase.

8. The recombinant cell according to claim 7, wherein the HMG-CoA reductase is mvaA (P13702) derived from *Pseudomonas mevalonii,* hmgA-1Mtc_0274 (H8I942) derived from *Methanocella conradii,* mvaA LLKF 1694 (D2BKK7) derived from *Lactococcus lactis subsp. lactis* (strain KF147), or mvaA SSA_0337 (A3CKT9) derived from *Streptococcus sanguinis* (strain SK36).

9. The recombinant cell according to any one of claims 4 to 8, wherein in the endogenous NAD(P)H consumption pathway, expression of at least one selected from the group consisting of ethanol dehydrogenase, acetaldehyde dehydrogenase, lactate dehydrogenase, and 2,3-butanediol dehydrogenase is down-regulated.

10. The recombinant cell according to claim 9, wherein in the endogenous NAD(P)H consumption pathway, expression of at least ethanol dehydrogenase is down-regulated.

11. The recombinant cell according to claim 10, wherein in the endogenous NAD(P)H consumption pathway, expression of acetaldehyde dehydrogenase is further down-regulated.

12. The recombinant cell according to claim 10 or 11, wherein in the endogenous NAD(P)H consumption pathway, expression of lactate dehydrogenase or 2,3-butanediol dehydrogenase is further down-regulated.

13. The recombinant cell according to any one of claims 9 to 12, wherein at least one of the down-regulation is a lack of expression.

14. The recombinant cell according to claim 13, wherein expression of ethanol dehydrogenase and/or acetaldehyde dehydrogenase lacks.

15. The recombinant cell according to claim 14, wherein instead of a gene encoding ethanol dehydrogenase and/or acetaldehyde dehydrogenase, a gene that expresses the mevalonate pathway is incorporated in a genome by homologous recombination.

16. The recombinant cell according to any one of claims 9 to 15, wherein expression of phosphotransacetylase and/or acetate kinase is further down-regulated.

17. The recombinant cell according to claim 16, wherein expression of at least phosphotransacetylase is down-regulated.

18. The recombinant cell according to any one of claims 1 to 17, wherein at least one of the down-regulation is carried out by gene deletion or modification of a gene expression-regulating region.

19. The recombinant cell according to any one of claims 9 to 18, wherein the gene that expresses the exogenous NAD(P)H consumption pathway is a gene cluster that includes a gene that expresses a mevalonate pathway and a gene encoding an enzyme for producing isoprene from isopentenyl diphosphate, and the organic compound is isoprene.

20. The recombinant cell according to claim 19, wherein the enzyme for producing isoprene from isopentenyl diphosphate is isoprene synthase.

21. The recombinant cell according to any one of claims 9 to 18, wherein the gene for expressing an exogenous NAD(P)H consumption pathway is a gene cluster that includes a gene that expresses a mevalonate pathway and a gene encoding an enzyme for producing cyclic terpene from isopentenyl diphosphate, and the organic compound is cyclic terpene.

22. The recombinant cell according to claim 21, wherein the enzyme for producing cyclic terpene from isopentenyl diphosphate is geranyl diphosphate synthase and/or neryl diphosphate synthase, and cyclic monoterpene synthase, and the organic compound is cyclic monoterpene.

23. The recombinant cell according to claim 22, wherein the cyclic monoterpene synthase is β-phellandrene synthase, and the cyclic monoterpene is β-phellandrene, 4-carene, or limonene.

24. The recombinant cell according to any one of claims 18 to 23, wherein the gene cluster is incorporated in a genome of the recombinant cell.

25. The recombinant cell according to claim 19 or 20, wherein a part or whole of an adhE1 gene and an adhE2 gene of a host cell that is a basis of the recombinant cell is deleted, and the gene cluster, instead of the adhE1 gene and the adhE2 gene, is incorporated in a genome by homologous recombination.

26. The recombinant cell according to any one of claims 21 to 23, wherein a part or whole of an adhE1 gene and an adhE2 gene of a host cell that is a basis of the recombinant cell is deleted, and the gene cluster, instead of the adhE1 gene and the adhE2 gene, is incorporated in a genome by homologous recombination.

27. A method for manufacturing a recombinant cell, the method comprising:
a first step of providing a host cell having a function of synthesizing acetyl-CoA from methyltetrahydrofolate, carbon monoxide, and CoA; and
a second step of introducing a gene that expresses an exogenous NAD(P)H consumption pathway into the host cell,
wherein
the gene that has been introduced in the second step is expressed in the host cell;
the expression in at least one of endogenous NAD(P)H consumption pathways of the host cell is down-regulated, and the endogenous NAD(P)H consumption pathway is different from the exogenous NAD(P)H consumption pathway; and
the recombinant cell produces the organic compound having 4 or more carbon atoms from at least one selected from the group consisting of carbon monoxide and carbon dioxide, via the exogenous NAD(P)H consumption pathway.

28. The method according to claim 27, wherein the host cell is a *Clostridium* bacterium or a *Moorella* bacterium.

29. The method according to claim 28, wherein the host cell is *Clostridium ljungdahlii, Clostridium autoethanogenum, Clostridium carboxidivorans, Clostridium ragsdalei, Clostridium kluyveri,* or *Moorella thermoacetica.*

30. The method according to claim 28 or 29, wherein the exogenous NAD(P)H consumption pathway is a mevalonate pathway.

31. The method according to claim 30, wherein the mevalonate pathway is a mevalonate pathway of yeast, prokaryote, or actinomycete.

32. The method according to claim 30, wherein the mevalonate pathway is a mevalonate pathway of actinomycete.

33. The method according to claim 30, wherein HMG-CoA reductase of the mevalonate pathway includes NADH-dependent HMG-CoA reductase.

34. The method according to claim 33, wherein the HMG-CoA reductase is mvaA (P13702) derived from *Pseudomonas mevalonii,* hmgA-1Mtc_0274 (H8I942) derived from *Methanocella conradii,* mvaA LLKF_1694 (D2BKK7) derived from *Lactococcus lactis subsp. lactis* (strain KF147), or mvaA SSA_0337 (A3CKT9) derived from *Streptococcus sanguinis* (strain SK36).

35. The method according to any one of claims 30 to 34, wherein in the endogenous NAD(P)H consumption pathway, expression of at least one selected from the group consisting of ethanol dehydrogenase, acetaldehyde dehydrogenase, lactate dehydrogenase, and 2,3-butanediol dehydrogenase is down-regulated.

36. The method according to claim 35, wherein in the endogenous NAD(P)H consumption pathway, expression of at least ethanol dehydrogenase is down-regulated.

37. The method according to claim 36, wherein in the endogenous NAD(P)H consumption pathway, expression of acetaldehyde dehydrogenase is further down-regulated.

38. The method according to claim 36 or 37, wherein in the endogenous NAD(P)H consumption pathway, expression of lactate dehydrogenase or 2,3-butanediol dehydrogenase is further down-regulated.

39. The method according to any one of claims 35 to 37, wherein at least one of the down-regulation is a lack of expression.

40. The method according to claim 39, wherein the expression of ethanol dehydrogenase and/or acetaldehyde dehydrogenase lacks.

41. The method according to claim 40, wherein instead of a gene encoding ethanol dehydrogenase and/or acetaldehyde dehydrogenase, a gene that expresses the mevalonate pathway is incorporated in a genome of the recombinant cell by homologous recombination.

42. The method according to any one of claims 35 to 41, wherein expression of phosphotransacetylase and/or acetate kinase is further down-regulated.

43. The method according to claim 42, wherein expression of at least phosphotransacetylase is down-regulated.

44. The method according to any one of claims 27 to 43, wherein at least one of the down-regulation is carried out by gene deletion or modification of a gene expression-regulating region.

45. The method according to any one of claims 35 to 44, wherein the gene that has been introduced in the second step is a gene cluster that includes a gene that expresses a mevalonate pathway and a gene encoding an enzyme for producing isoprene from isopentenyl diphosphate, and the organic compound is isoprene.

46. The method according to claim 45, wherein the enzyme for producing isoprene from isopentenyl diphosphate is isoprene synthase.

47. The method according to any one of claims 35 to 44, wherein the gene that has been introduced in the second step is a gene cluster that includes a gene that expresses a mevalonate pathway and a gene encoding an enzyme for producing cyclic terpene from isopentenyl diphosphate, and the organic compound is cyclic terpene.

48. The method according to claim 47, wherein the enzyme for producing cyclic terpene from isopentenyl diphosphate is geranyl diphosphate synthase and/or neryl diphosphate synthase, and cyclic monoterpene synthase, and the organic compound is cyclic monoterpene.

49. The method according to claim 48, wherein the cyclic monoterpene synthase is β-phellandrene synthase, and the cyclic monoterpene is β-phellandrene, 4-carene, or limonene.

50. The method according to any one of claims 44 to 49, wherein the gene cluster is incorporated in a genome of a host cell.

51. The method according to claim 45 or 46, wherein a part or whole of an adhE1 gene and an adhE2 gene of a host cell is deleted, and the gene cluster, instead of the adhE1 gene and the adhE2 gene, is incorporated in a genome by homologous recombination.

52. The method according to claim 47 to 49, wherein a part or whole of an adhE1 gene and an adhE2 gene of a host cell is deleted, and the gene cluster, instead of the adhE1 gene and the adhE2 gene, is incorporated in a genome by homologous recombination.

53. A method for producing an organic compound, the method comprising: bringing at least one C1 compound selected from the group consisting of carbon monoxide and carbon dioxide into contact with the recombinant cell according to any one of claims 1 to 26 or the recombinant cell manufactured by the method according to any one of claims 27 to 52.

54. The method according to claim 53, wherein the recombinant cell is cultured using at least one C1 compound selected from the group consisting of carbon monoxide and carbon dioxide as a carbon source to produce an organic compound having 4 or more carbon atoms in the recombinant cell.

55. The method according to claim 53 or 54, wherein the recombinant cell is provided with gas mainly containing carbon monoxide, gas mainly containing carbon monoxide and hydrogen, gas mainly containing carbon dioxide and hydrogen, or gas mainly containing carbon monoxide, carbon dioxide and hydrogen.

56. The method according to claim 55, wherein the recombinant cell is further provided with formic acid or methanol.

57. The method according to any one of claims 53 to 56, wherein the recombinant cell is a *Clostridium* bacterium or a *Moorella* bacterium.

58. The method according to any one of claims 53 to 57, wherein the organic compound released to outside the recombinant cell is collected.

59. The method according to any one of claims 53 to 58, wherein the organic compound is collected from a gas phase of a culture system of the recombinant cell.
